# EUROPEAN PATENT APPLICATION

(11) **EP 3 950 940 A1**
(43) Date of publication of application: **09.02.2022**
(21) Application number: 19921710.0
(22) Date of filing: 11.11.2019
(51) Int. Cl.: C12N 9/22, C12N 15/09, C12P 21/02, C07K 1/18, C07K 1/32, C07K 1/36

(54) **METHOD FOR PRODUCING A PREPARATION OF HIGHLY-PURIFIED RECOMBINANT CAS NUCLEASE**

(30) Priority: 28.03.2019 RU 2019109018
(71) Applicant: Federal Budget Institut of Science "Central Research Institute for Epidemiology", Moscow 111123 (RU)
(72) Inventor: AKIMKIN, Vasiliy Gennadevich, Moscow, 111250 (RU); TIUMENTSEV, Aleksandr Igorevich, Moscow, 111123 (RU); TIUMENTSEVA, Marina Alekseevna, Moscow, 111123 (RU); SHAGIN, Dmitriy Alekseevich, Moscow, 117574 (RU)
(74) Representative: AAA Patendibüroo OÜ
(86) International application number: PCT/RU2019/000800
(87) International publication number: WO 2020/197436

(57) **Abstract**

The invention relates to the field of bioengineering, specifically to a method for producing a Cas-family recombinant nuclease preparation for a CRISPR/Cas system in cells of the Escherichia coli strain Rosetta-gami B (DE3), to a Cas-family recombinant nuclease preparation for a CRISPR/Cas system, and to a kit containing the given preparation to be used in a CRISPR/Cas system. The claimed method, which includes two-step chromatographic purification and a step of additional purification of preparations using Triton X-114 detergent, makes it possible to decrease bacterial endotoxin content to a level of 0.3-1.5 EU/ml in resulting preparations and to increase target protein yield to no less that 20 milligrams from 1 liter of a culture of a producing strain.

## Description

### TECHNICAL FIELD

The present invention relates to the field of biotechnology, in particular to the method of producing the recombinant Cas nuclease preparation by means of recombinant production in cells of the Escherichia coli, purification and additional purification of the Cas nuclease from bacterial endotoxins. The present invention also relates to the products of recombinant Cas nuclease obtained by the method offered and kits to be used in CRISPR/Cas system containing the product of recombinant Cas nuclease, substantially free from the bacterial endotoxins.

The present invention allows producing recombinant Cas nuclease in significant quantities and in an accelerated time the preparation is characterized by minimal toxicity, which allows using it in the CRISPR / Cas system in various fields of technology related to the genome editing. The applicability varies from creating cell models of hereditary diseases of humans and animals, functional screening of genomes, epigenomes study and visualization of cellular processes to applications in the food industry to produce fortified foods, and in agriculture to create new breeds of animals and varieties of plants, as well as in medicine.

The CRISPR/Cas system with the recombinant Cas nuclease obtained according to the method offered in the present application meets the highest requirements for the efficiency safety and availability and can be used for diagnostics of diseases by means of genetic sequences identification, for example, viruses and oncogenes, prevention of infectious diseases by means of modifying the genes not of a human but carriers of diseases such as malaria and treatment of socially significant diseases, both hereditary and acquired, including cancer, autoimmune, orphan diseases, infectious diseases, including HIV and many others.

### BACKGROUND ART

Genome editing based on the using programmable nucleases has taken a leading position among genome modification technologies in a short time. Today exist three main systems for the directed genome editing: «zinc finger» nucleases, TALE-nucleases and CRISPR/CAS nucleases (Nemudryi AA, Valetdinova KR, Medvedev SP and Zakian SM., TALEN and CRISPR/Cas Genome Editing Systems: Tools of Discovery, Acta Naturae, 2014, v.6, n.3, p:19-40; German DM, Mitalipov S, Mishra A and Kaul S., Therapeutic Genome Editing in Cardiovascular Diseases, JACC Basic Transl Sci., 2019, v.4, n.1, p:122-131; Kwarteng A, Ahuno ST and Kwakye-Nuako G., The therapeutic landscape of HIV-1 via genome editing, AIDS Res Ther., 2017, v.14, n.1, p:32; A programmable dual RNA-guided DNA endonuclease in adaptive bacterial immunity // Martin Jinek, Krzysztof Chylinski, Ines Fonfara, Michael Hauer, Jennifer A. Doudna and Emmanuelle Charpentier, Science, 2012, v.337, n.6096, p:816-821).

The genome editing with the use of the CRISPR/CAS nucleases has a number of advantages such as high efficiency, possibility of multiple editing, low price and also speed of development (Loureiro A and da Silva GJ., CRISPR-Cas: Converting A Bacterial Defence Mechanism into A State-of-the-Art Genetic Manipulation Tool, Antibiotics (Basel), 2019, v.8, n.1, pii:E18; Karimian A, Azizian K, Parsian H, Rafieian S, Shafiei-Irannejad V, Kheyrollah M, Yousefi M, Majidinia M and Yousefi B., CRISPR/Cas9 technology as a potent molecular tool for gene therapy, J. Cell Physiol., 2019, doi: 10.1002/jcp.27972).

The CRISPR/CAS system of the genome editing includes CAS-nuclease (protein) and guide RNA specific to the target sequence. Delivery of the CRISPR/CAS system to the living cell is executed by means of three methods: in the form of a complex of plasmid DNA that codes CAS-protein and guide RNA; in the form of mRNA mixture that codes the CAS-protein and guide RNA; and the third way to deliver the CRISPR/CAS system implies delivery into the cell of the assembled ribonucleoprotein complex (mixture of CAS-protein and guide RNA). Delivery of the CRISPR/CAS system in the form of the assembled ribonucleoprotein complex has a number of advantages among which are: the high efficiency of editing; low non-specific activity; editing immediately after delivery to the cell; possibility of the quick screening of guide RNA efficiency in the test tube (in vitro); the immunogenicity reduced due to the short residence time of the CRISPR/CAS system elements in the target cell. The most promising direction from the point of view of creating therapeutic agents lies the in genome editing using CRISPR/CAS systems working with the assembled ribonucleoprotein complexes.

Currently several heterologous expression systems are widely used to obtain recombinant proteins, including bacterial cells (Escherichia coli, etc.); yeast cells (Saccharomyces cerevisiae, Pichia pastoris, etc.); insect cells; plant cells and mammalian cells.

Each of the mentioned expression systems has advantages and disadvantages and the final choice of expression system usually depends on the properties of the target product (Xu J, Ge X and Dolan MC, Towards high-yield production of pharmaceutical proteins with plant cell suspension cultures, Biotechnol Adv., 2011, v.29, n.3, p:278-299).

The main advantages of the expression of recombinant proteins in Escherichia coli cells include the simplicity and speed of the obtaining of a recombinant strain; the relatively low cost of the development process; the scalability of the expression and purification processes and the high yield of the target protein.

When recombinant proteins of the CRISPR/CAS family are obtained in E. coli cells, there is a problem of additional purification from bacterial endotoxins, the content of which is regulated by the General Pharmacopoeia Article of the OFS.1.2.4.0006.15 bacterial endotoxins. The threshold pyrogenic dose is considered a dose equal to 5 EU / kg per 1 hour for the test preparation, if it is administered to the patient by any parenteral route other than intrathecal. Proteins of the CRISPR/CAS family have sufficiently high values of the isoelectric point (pl > 9) so in solutions with physiological pH = 6.5-8.0 they have a high positive total charge. The high positive total charge of proteins of the CRISPR/CAS family leads to co-isolation of the negatively charged bacterial endotoxins of destroyed bacterial cell walls with proteins of the CRISPR/CAS family.

It is worth noting that production of recombinant CRISPR/CAS family in other heterological expression systems presumably will not result in complete absence of bacterial endotoxins due to the difficultly to obtain preparations free from bacterial endotoxins (with account of their physical and chemical properties, pI > 9). This happens because residual bacterial endotoxins can be contained in the culture medium and additives used for cultivation, as well as in the water used for the preparation of buffer solutions.

Production of the recombinant nucleases of the CRISPR/CAS family in E. coli cells with different approaches to purification of the target proteins was described in the several articles, for example: Nandhakishore Rajagopalan, Sateesh Kagale, Pankaj Bhowmik and Halim Song, A Two-Step Method for Obtaining Highly Pure Cas9 Nuclease for Genome Editing, Biophysical, and Structural Studies, 2018, v.1, n.17, p.1-8; Wenqiang Li, Shungtang Li, Jie Qiao, Fei Wang, Yang Liu, Ruyi He, Yi Liu and Lixing Ma, Direct preparation of Cas9 ribonucleoprotein from E. coli for PCR-free seamless DNA assembly, doi: https://doi.org/10.1101/328468; Sojung Kim, Daesik Kim, Seung Woo Cho, Jungeun Kim and Jin-Soo Kim, Highly efficient RNA-guided genome editing in human cells via delivery of purified Cas9 ribonucleoproteins, Genome Res., 2014, v.24, n.6, p:1012-1019; Zuris JA, Thompson DB, Shu Y2, Guilinger JP, Bessen JL, Hu JH, Maeder ML, Joung JK, Chen ZY and Liu DR, Cationic lipid-mediated delivery of proteins enables efficient protein-based genome editing in vitro and in vivo, Nat. Biotechnol., 2015, v.33, n.1, p.73-80; Koziel D, Michaelis U, Kruse T., Broad application and optimization of a single wash-step for integrated endotoxin depletion during protein purification, J. Chromatogr. B Analyt. Technol. Biomed. Life Sci., 2018, v.1091, p.101-107; Lin S, Staahl BT, Alla RK and Doudna JA, Enhanced homology-directed human genome engineering by controlled timing of CRISPR/Cas9 delivery, Elife, 2014, n.3, p:e04766; Liang Z, Chen K, Li T, Zhang Y, Wang Y, Zhao Q, Liu J, Zhang H, Liu C, Ran Y and Gao C, Efficient DNA-free genome editing of bread wheat using CRISPR/Cas9 ribonucleoprotein complexes, Nat. Commun., 2017, n.8, p:14261, and also in patent documents, for example, EA 201790968 A1, published on January 31, 2018; WO 2016205703 A1, published on December 22, 2016; EA 24121 B9, published on January 30, 2017 and WO 2016161516 A1, published on October 13, 2016.

The closest analogue of the invention is a method for obtaining a recombinant protein of the Cas family in E. coli cells by using metal-affinity and cation exchange chromatography for purification of the target protein, disclosed in the following article: Nandhakishore Rajagopalan, Sateesh Kagale, Pankaj Bhowmik and Halim Song, A Two-Step Method for Obtaining Highly Pure Cas9 Nuclease for Genome Editing, Biophysical, and Structural Studies, 2018, v.1, n.17, p.1-8. This method describes obtaining Cas9 Streptococcus pyogenes (NLS-Cas9-6xHis) in the E. coli BL21 star (DE3)pLysS cells using the Luria-Bertani broth (LB), containing ampicillin (100 µg/mL), where 5 mL of the starting culture were inoculated and were grown overnight at 37° C until reaching the optic density of OD₆₀₀≈0,6. Then cells were induced with IPTG (0.5 mM) and incubated at 18_{°}C for 16 hours. Upon completion of the cultivation, the centrifuging was carried out by 5000×g for 15 minutes at 4° C. Then cell pellet was resuspended in the ice cold buffer for lysis LW (20 mM HEPES pH 7.5, 300 mM NaCl, 25 mM imidazole, 0.5 mM TCEP). Cell destruction was performed using a french press followed by centrifugation of the lysate at 39706×g at 4° C for 20 minutes. Purification of the target protein was performed by two-stage chromatography. At the first stage, metal-affinity chromatography was performed using the Ni-NTA agarose chromatography resin (Qiagen, USA) with an ice cold buffer LW. Elution was executed with the ice cold buffer EB (pH 7.5, 20 mM HEPES, 300 mM NaCl, 250 mM imidazole, 0.5 mM TCEP). At the second stage, cation exchange chromatography was performed using a chromatography resin Resource S (GE Healthcare, Great Britain) and buffer A (pH 7.5, 20 mM HEPES, 200 mM KCl, 10 mM MgCl₂, 0.5 mM TCEP). Elution was executed with buffer B (pH 7.5, 20 mM HEPES, 1 M KCl, 10 mM MgCl₂, 0.5 mM TCEP). Then centrifugation was performed, buffer A and glycerol were added for freezing. The described method makes it possible, after two-stage chromatographic purification, to obtain about 1 milligram of highly purified Cas9 Streptococcus pyogenes protein from 1 liter of the producing strain culture.

The disadvantages of the described in the nearest analogue method include relatively low yield of the target protein, as well as the lacking of any information about the content of residual bacterial endotoxins in the resulting protein preparation, which calls into question the safety of using nucleases obtained by described method in the living organisms (in vivo).

Based on this, a technical problem consisting in the need to increase the yield of Cas nucleases in recombinant methods of obtaining E. coli and minimize the content of toxic impurities in the preparations of recombinant proteins of the CRISPR/CAS family arises.

### SUMMARY OF INVENTION

The invention relates to a method of producing recombinant Cas nucleases by recombinant production in Escherichia coli cells, purification and additional purification of Cas nucleases from bacterial endotoxins, as well as recombinant Cas nucleases obtained by the proposed method, and kits for use in CRISPR/Cas system containing the recombinant Cas nucleases, substantially free from bacterial endotoxins.

The technical objective of the proposed invention is to increase the degree of purification of recombinant proteins of the CRISPR/Cas family with a decrease in the content of bacterial endotoxins in solutions of recombinant proteins of the CRISPR/Cas family to allow their use in recombinant Cas nucleases of the CRISPR/Cas system applied in vivo.

When the present invention will be implemented in accordance with the combination of the essential features given in the claims the following technical result is achieved: increasing of the yield of the target protein during expression in E. coli, as well as improving of the efficiency of the recombinant nucleases of the Cas family purification, preserving or increasing their specific activity and a decrease in the content of bacterial endotoxins to the level of 0,3-1,5 EU/ml in preparations from the obtained recombinant Cas nucleases, suitable for using in the living organisms (in vivo). It was possible to achieve a yield of at least 20 milligrams of highly purified, substantially endotoxin-free recombinant protein of the CRISPR/Cas family from 1 liter of the producing strain culture.

The solution of the technical problem is achieved at the expense of:
- Using the parental Rosetta-gami B (DE3) strain of E. coli for the production of recombinant Cas nucleases and compliance with the protocol of cell culture of this strain using Luria-Bertani broth (LB) with 100 mkg/ml of ampicillin supplemented with MgSO4 and glucose for the starting culture, followed by the cultivation until induction in the same culture medium, but with a reduced glucose content of at least 2 times. Deviations from the protocol lead to a lack of protein expression;
- Induction of expression after culture achieves optical density OD₆₀₀≈1,5-1,9 and therefore significantly increases the yield of the target protein;
- Implementation of protein expression at 22°C with deviation from the protocol is leading to protein aggregation during subsequent purification;
- Compliance with the protocol developed by the authors for the chromatographic purification of the recombinant Cas nucleases with a certain buffer composition and a strict requirement for the conductivity of the Cas protein solution subjected to cation exchange chromatography. Deviation from the protocol leads to the losses in the amount of the purified protein;
- Compliance with the protocol developed by the authors for the removal of the bacterial endotoxins from solutions of the recombinant CAS nucleases expressed in E. coli cells using Triton X-114 detergent and at least 6-fold repetition of purification cycles to achieve a yield of at least 20 milligrams per 1 liter of the producing strain culture of a highly purified recombinant protein of the CRISPR/Cas family with a minimum content of endotoxins.

### DESCRIPTION OF THE INVENTION

Before describing the methods, preparations and kits according to the present invention, it should be noted that the present invention is not limited to the specific described methods means and approaches, products or combinations used since such methods means and approaches, products or combinations can undoubtedly vary. Also worth noting, that the terminology used in this application is not restrictive, since the scope of the present invention will be limited only to the offered claims.

Unless otherwise defined all terms and concepts used in the disclosure of the present invention including technical and scientific terms have a meaning that is commonly understood by the average specialist of the technical field to which the present invention belongs. As a rule, the classification and methods used in cell biology, molecular biology, immunology, microbiology, genetics, analytical chemistry, organic synthesis chemistry, medical and pharmaceutical chemistry, as well as hybridization and chemistry of proteins and nucleic acids described in this application are well known to specialists and are widely used in this field. Methods for recombinant production and cultivation of producing cells isolation of the target protein and purification and verification of the activity of the target protein are carried out in the accordance with the manufacturer's instructions, as it is usually done in this field or as described in detail in this application.

The following definitions are the terms and concepts used in this application for a better understanding the essence of the present invention.

The singular form used in this application include objects in both the singular and the plural, unless the context clearly indicates otherwise.

The terms "containing", "contains" and "contained" used in this application are synonymous to "including", "includes" or "covering", "covers" and are inclusive or non-exclusive and do not exclude additional non-mentioned parts components of products or stages of methods. The terms "containing" "contains" and "contained" used in this application include the terms "consisting of" "consists" and "consists of" as well as the terms "consisting essentially of" "consists essentially" and "consists essentially of".

The description of a range of numeric values using extreme points implies all numbers and fractional numbers belonging to the corresponding ranges as well as the specified extreme points.

The terms "approximately" or "about" used in this application when referring to a measured value, such as a parameter, quantity, time interval, etc., cover changes of +/-20% or less, preferably +/-10% or less, more preferably +/-5% or less, and even more preferably +/-1% or less, of the specified value, to the extent that such changes are suitable for implementation in the disclosed invention. The meaning to which the term "approximately" or "about" refers is also disclosed specifically and preferable by itself.

Although the concepts of "one or more" or "at least one" such as, for example one or more or at least one component (s) belonging to the group of components are clear and follow examples given in this application, this concept covers among other things any of the specified components or any two or more of the specified components such as, for example any of the 3, 4, 5, 6, 7, 8, 9, 10, etc. of the specified components and up to all of the specified components.

The term "substantially" does not mean "completely" but also does not exclude such a possibility, for example a preparation that is "substantially purified" may contain no impurities at all or may contain impurities in small amounts. This application proposes a preparation of the recombinant purified Cas protein, wherein "purified" means that the target protein is present in the composition of the preparation, which substantially does not contain other expressed proteins or producing endotoxins, while "substantially does not contain" means that the composition of the preparation contains minimal amounts of impurities.

All documents quoted or cited as references in this application and all documents quoted or cited as references in documents cited in this application together with any manufacturer's instructions, descriptions and modes of use of the method or products mentioned in this application or in any document included in this application by reference are included in the offered application by reference and can be used in the implementation of the proposed invention in practice.

In the following parts the various aspects of the present invention are defined. Each aspect defined in this way can be combined with any other aspect or aspects, unless explicitly stated otherwise. In particular, any attribute designated as being preferred or preferential may be combined with any other attribute or attributes designated as being preferred or preferential.

The term "if necessary" should be understood as denoting an additional optional condition, stage, technique or component of a method or product or as denoting an alternative embodiment of the invention or an option of its implementation in particular its conditions, stages, techniques or components of the composition.

CRISPR (from Clustered Regularly Interspaced Short Palindromic Repeats; short palindromic repeats, regularly arranged in groups) are special loci of bacteria and archaea, consisting of repeating sequences that are separated by unique sequences (spacers).

The CRISPR-Cas genome editing system ("CRISPR-Cas system", "CRISPR-Cas9 system" or "CRISPR system") consists of a CAS nuclease (protein) and a guide RNA specific to the target sequence. In general, the CRISPR-Cas system is characterized by elements which contribute to the formation of the CRISPR complex in the target sequence site (protospacer).

In the context of the CRISPR complex "target sequence" refers to the sequence against which the guide sequence is constructed in such a way as to be complementary, with hybridization between the target sequence and the guide sequence contributing to the formation of the CRISPR complex. The target sequence can contain any polynucleotide such as DNA or RNA polynucleotides. In some embodiments the target sequence locates in the nucleus or cytoplasm of the cell and it may include nucleic acids in or from mitochondria, organelles, vesicles, liposomes or particles present within the cell.

For specific targeting of the CRISPR/Cas system protein various types of guide RNAs can be used to direct Cas into the target locus. For example, the guide RNA can be a complex of CRISPR RNA crRNA responsible for specific recognition of the target, as well as transactivating CRISPR RNA tracrRNA responsible for binding the enzyme of the CRISPR/Cas system. In addition, the guide RNA can be a single combined RNA (sgRNA) with the properties both from CRISPR RNA crRNA and CRISPR RNA tracrRNA in a single molecule.

The guide RNA may contain a guide sequence capable of hybridizing with the target genome locus in the cell, a tracr sequence and a paired tracr sequence. All of them can be in a single RNA, i.e. sgRNA (located in the 5' - 3 ' orientation) or tracrRNA can be a RNA other than the RNA containing the guide and tracr sequence. The tracr sequence can hybridize with the paired tracr sequence and direct the CRISPR/Cas complex to the target sequence.

The term "crRNA" or "guide RNA" or "single guide RNA" or "sgRNA" includes any polynucleotide sequence characterized by sufficient complementarity with the target nucleic acid sequence to hybridize with the target nucleic acid sequence and control sequence-specific binding of the nucleic acid targeting complex to the target nucleic acid sequence.

The ability of the guide sequence to control sequence-specific binding of the nucleic acid targeting complex to the target nucleic acid sequence within the guide RNA for nucleic acid targeting can be evaluated by means of any suitable analysis. For example, components of a CRISPR system for targeting nucleic acid sufficient to form a nucleic acid targeting complex including a guide sequence to be tested can be provided in a host cell with appropriate target nucleic acid sequence, such as by transfection with vectors encoding components of the nucleic acid targeting complex followed by an assessment of the preferred targeting (e.g., cleavage) within the target nucleic acid sequence as for example, by the analysis described in this application.

The cleavage of the target nucleic acid sequence can be determined in vitro by providing the target nucleic acid sequence or by the components of the nucleic acid targeting complex including the guide sequence to be tested and the control guide sequence other than the test guide sequence and comparing the binding or degree of cleavage of the target sequence in the case of reactions with the test and control guide sequence. Other analyses are possible and can be performed by specialists in the field.

The guide sequence and the guide RNA for targeting the nucleic acid can be selected to target any target sequence of the nucleic acid. The target sequence may be DNA. The target sequence can be any RNA sequence. In some embodiments the target sequence may be a sequence within a RNA molecule selected from a group consisting of a matrix RNA (mRNA), pre-mRNA, ribosomal RNA (rRNA), transport RNA (tRNA), micro-RNA (miRNA), small interfering RNA (siRNA), small nuclear RNA (snRNA), small nucleolar RNA (snoRNA), double-stranded RNA (dsRNA), non-coding RNA (ncRNA), long non-coding RNA (IncRNA) and small cytoplasmic RNA (scRNA).

In some embodiments the guide RNA or crRNA may contain or consist of a direct repeat (DR) sequence and a guide sequence and/or spacer sequence.

The "tracrRNA" sequence includes any polynucleotide sequence characterized by sufficient complementarity with the crRNA sequence to allow hybridization. In some embodiments the crRNA sequence and the paired tracr sequence are contained in the same transcript, therefore when hybridization occurs between them a transcript in one or up amount with a secondary structure such as a "hairpin" forms.

The terms used in this technical field describe the protein entering the CRISPR/Cas system as «Cas protein», «CRISPR/Cas protein», «Cas family protein», «CRISPR/Cas system protein», «Cas family protein of CRISPR/Cas system», «Cas nuclease», «CRISPR/Cas nuclease», «CRISPR/Cas family nuclease», «CRISPR/Cas system nuclease», «Cas family nuclease of CRISPR/Cas system», «Cas endonuclease», «CRISPR/Cas endonuclease», «CRISPR/Cas family endonuclease», «CRISPR/Cas system endonuclease», «Cas family endonuclease of CRISPR/Cas system» and in this application if otherwise is not stated, the listed definitions are interchangeable.

Nucleases as a group of enzymes hydrolyse the phosphodiester bond between nucleic acid subunits. There are several types of nucleases depending on their specificity and activity: exonucleases and endonucleases, ribonucleases and deoxyribonucleases, nickases and some others.

The method disclosed in this application provides preparation of recombinant endonucleases of the Cas family of the CRISPR/Cas system of type I, type II or type III. The suitable CRISPR/Cas proteins can be selected from Cas1, Cas1b, Cas2, Cas3, Cas4, Cas5, Cas5e (CasD), Cas6, Cas6e, Cas6f, Cas7, Cas8al, Cas8a2, Cas8b, Cas8c, Cas9, Cas9D10A, Cas12a (Cpf1), CaslO, CaslOd, CasF, CasG, CasH, Csyl, Csy2, Csy3, Csel (CasA), Cse2 (CasB), Cse3 (CasE), Cse4 (CasC), Csel, Csc2, Csa5, Csn2, Csm2, Csm3, Csm4, Csm5, Csm6, Cmrl, Cmr3, Cmr4, Cmr5, Cmr6, Csbl, Csb2, Csb3, Csxl7, Csxl4, CsxlO, Csxl6, CsaX, Csx3, Cszl, Csxl5, Csf1, Csf2, Csf3, Csf4, Cul966 and others.

Cas nuclease can origin from Streptococcus pyogenes, Streptococcus thermophilus, Streptococcus sp., Nocardiopsis dassonvillei, Streptomyces pristinaespiralis, Streptomyces viridochromogenes, Streptosporangium roseum, Alicy clobacdlus acidocaldarius, Bacillus pseudomycoides, Bacillus selenitireducens, Exiguobacterium sibiricum, Lactobacillus delbrueckii, Lactobacillus salivarius, Microscilla marina, Burkholderiales bacterium, Polaromonas naphthalenivorans, Polaromonas sp., Crocosphaera watsonii, Cyanothece sp., Microcystis aeruginosa, Synechococcus sp., Acetohalobium arabaticum, Ammonifex degensii, Caldicelulosiruptor becscii, Candidatus Desulforudis, Clostridium botulinum, Clostridium difficile, Finegoldia magna, Natranaerobius thermophilus, Pelotomaculum thermopropionicum, Acidithiobacillus caldus, Acidithiobacillus ferrooxidans, Allochromatium vinosum, Marinobacter sp., Nitrosococcus halophilus, Nitrosococcus watsoni, Pseudoalteromonas haloplanktis, Ktedonobacter racemifer, Methanohalobium evestigatum, Anabaena variabilis, Nodularia spumigena, Nostoc sp., Arthrospira maxima, Arthrospira platensis, Arthrospira sp., Lyngbya sp., Microcoleus chthonoplastes, Oscillatoria sp., Petrotoga mobilis, Thermosipho africanus 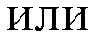 Acaryochloris marina, Pseudomonas aeruginosa, Acinetobacter baumannii, Salmonella enterica, Streptococcus agalactiae, Escherichia coli, Streptococcus suis, Salinispora arenicola, Klebsiella pneumoniae, Neisseria meningitidis, Vibrio cholerae, Leptospira interrogans, Listeria monocytogenes, Fusobacterium nucleatum, Enterococcus faecalis, Salinispora pacifica, Pasteurella multocida, Moraxella catarrhalis, Leptospira santarosai, Bifidobacterium longum, Serratia marcescens, Clostridioides difficile, Bifidobacterium breve, Methanosarcina mazei, Yersinia pseudotuberculosis, Fusobacterium necrophorum, Propionibacterium freudenreichii, Campylobacter coli, Clostridium novyi, Corynebacterium ulcerans, Salinispora tropica, Lactobacillus paracasei, Aggregatibacter actinomycet., Treponema pedis, Corynebacterium striatum, Lactobacillus rhamnosus, Xenorhabdus nematophila, Campylobacter jejuni, Lachnospiraceae bacterium, Treponema denticola, Staphylococcus aureus, Bacillus halodurans.

According to the present invention Cas nucleases can be obtained from a wild-type Cas protein or from a fragment thereof or from a modified Cas protein to change one or more biological or physico-chemical properties by adding functional elements to increase the activity, affinity, solubility, stability of the nuclease, to facilitate penetration into the cell or its compartments, etc. Either Cas protein domains not involved in RNA-directed cleavage can be removed from the protein in order to reduce the size of the modified Cas protein compared to the wild type.

Preferred variants include the RNA-directed endonucleases of the CRISPR/Cas system: SPCas9 - Cas9 nuclease from Streptococcus pyogenes, STCas9 - Cas9 nuclease from Streptococcus thermophilus, SPCas9D10A - Cas9 nickase from Streptococcus pyogenes and AsCpfl - Cpfl nuclease from Acidaminococcus, the amino acid sequences of which can be supplemented from the N-end and/or C-end:
- 6×His - with polyhistidine sequence to improve the purification efficiency;
- SV40 NLS - with the sequence of the nuclear localization signal of large T-antigen of the simian virus SV40 for effective protein delivery to the target cell nucleus;
- Nucleoplasmin NLS - with the sequence of the nucleoplasmin nuclear localization signal;
- TAT - with the sequence of the penetrating peptide TAT of the human immunodeficiency virus (HIV-1).

The most preferable are RNA-directed endonucleases characterized by having or containing an amino acid sequence selected from:
- SEQ ID NO: 1,
- SEQ ID NO: 2,
- SEQ ID NO: 3,
- SEQ ID NO: 4,
- SEQ ID NO: 5,
- SEQ ID NO: 6,
- SEQ ID NO: 7
- SEQ ID NO: 8
- or identical to any of them at least by 95%, 96%, 97%, 98% or 99% and also encoded by nucleic acids characterized by having or containing a nucleotide sequence selected from
- SEQ ID NO: 9,
- SEQ ID NO: 10,
- SEQ ID NO: 11,
- SEQ ID NO: 12,
- SEQ ID NO: 13,
- SEQ ID NO: 14,
- SEQ ID NO: 15,
- SEQ ID NO: 16
- or identical to any of them at least by 80%, 81%, 82%, 83%, 84%, 85%, 86%, 87%, 88%, 89%, 90%, 91%, 92%, 93%, 94%, 95%, 96%, 97%, 98% or 99%,
- or complementary to any of them,
- or hybridizing with any of them under strict conditions,
   which can
- be associated with regulatory sequences,
- be a part of an expression cassette, vector, or plasmid for expression, cloning, or integration into the genome, or
- be a part of the producing cell.

In this context the term "strict hybridization conditions" refers to the parameters known to a specialist in the appropriate field, including a variation in the hybridization temperature depending on the length of the nucleic acid. Parameters of nucleic acid hybridization can be found in references that combine such methods, Sambrook J. et al., Molecular Cloning, a Laboratory Manual. Cold Spring Harbor, Laboratory Press, New York, 1989 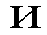 Ausubel M. et al., Current protocols in molecular biology, edited by Volumes 1 and 2. John Wiley & Sons, Inc., Media, PA, 1988). For example, strict hybridization conditions, in this context, may refer to hybridization at 65°C in the hybridization buffer (3,5×SSC, 0,02% Ficoll, 0,02% polyvinylpyrrolidone, 0,02% bovine serum albumin, 2,5 mM NaH₂PO₄, 0,5% SDS, 2 mM EDTA, pH 7).

According to the present invention a method for obtaining a recombinant nuclease preparation of the Cas family of the CRISPR/Cas system as described in the nearest analogue (Nandhakishore Rajagopalan, Sateesh Kagale, Pankaj Bhowmik and Halim Song, A Two-Step Method for Obtaining Highly Pure Cas9 Nuclease for Genome Editing, Biophysical, and Structural Studies, 2018, v.1, n.17, p.1-8) bases on the transformation of competent E. coli cells by an expression plasmid coding the target protein with further cultivation of the transformed producing cells under the circumstances, ensuring the expressions of the indicated recombinant nuclease in the culture medium Luria-Bertrani (LB) with the addition of ampicillin and usage of isopropyl-β-D-thiogalactoside (IPTG) as an inducer of expression of the target protein and isolation of the target protein from the soluble fraction using two-stage chromatographic purification.

However, the achievement of unexpected technical result is ensured by following differences in the present invention compared to the method mentioned in the closest analogue:
the competent Rosetta-gami B (DE3) E. coli cells are used as producing cells of the Cas family recombinant nuclease of the CRISPR/Cas system;
MgSO₄ and glucose are added to the culture medium and producing cells are grown until the starting culture is obtained and after that the starting culture is diluted to optical density of OD₆₀₀≈0,3 and the glucose content is reduced in the indicated medium at least in two times;
Then the producing cells are cultivated before they reach the optical density of OD₆₀₀≈1,5-1,9, IPTG is added and incubated at 22°C to get the effective expression of the target protein;
at the end of cultivation after centrifugation the cells are resuspended in a Na₂HPO₄/NaH₂PO₄-based lysis buffer and destroyed by ultrasonic disintegration followed by centrifugation to separate the fraction of soluble cytoplasm containing Cas family recombinant nuclease of the CRISPR/Cas system from the insoluble fraction of inclusion bodies.

To obtain the starting culture 2 mm MgSO₄ and 2% glucose preferably are added to the LB culture medium with 100 mkg / ml of ampicillin and then diluted in the LB culture medium with 100 mkg / ml of ampicillin, supplemented with 2 mm MgSO₄ and 1% glucose.

Also, the producing cells are grown preferably at 37°C for 16-20 hours to obtain a starting culture and after dilution they are cultured at 37°C until an optical density of OD600≈1.5-1.9 is reached and after adding IPTG they are incubated at 22°C for 20-24 hours.

The cells are resuspended preferably in a lysis buffer containing 20 mM Na₂HPO₄/NaH₂PO₄ with pH 6,6-8,0.

Centrifugation at the end of cultivation is carried out preferably at 6,000 g for 10 minutes and/or centrifugation to separate the fraction of soluble cytoplasm is carried out at 10,000 g for one hour.

The achievement of unexpected technical result is ensured by following differences in the present invention compared to the method mentioned in the closest analogue:
NaCl and imidazole are added to purify the fraction of soluble cytoplasm containing the target protein obtained after centrifugation and the first stage of chromatographic purification by means of metal-affinity chromatography, while the elution of the target protein is carried out with a buffer containing Na₂HPO₄/NaH₂PO₄, NaCl and imizadole;
At the second stage of chromatographic purification the target protein in the specified buffer is diluted at least 20 times with a buffer with Tris-HCl, while the conductivity of the solution does not exceed 5 mS / cm and subjects to cation exchange chromatography followed by the elution of the target protein with a buffer containing Tris-HCl and NaCl with DTT and EDTA added to the specified buffer to stabilize the target protein.

At the first stage of chromatographic purification NaCl is added to the specified fraction of soluble cytoplasm preferably to a final concentration of 500 mM and imidazole is added preferably to a final concentration of 10 mM and the elution of the target protein is carried out preferably with a buffer with pH 6,6-8,0, containing 20 mM Na₂HPO₄/NaH₂PO₄, 500 mM NaCl and 250 mM of imizadole.

Chelating Sepharose FF is used preferably as a chromatography resin at the first stage of the chromatographic purification.

At the second phase of chromatographic purification the target protein in the buffer is diluted at least 20 times preferably with a buffer with pH 8.2 containing 20 mM Tris-HCl with conductivity solution of 3-5 mS/cm, elution of the target protein is carried out preferably with a buffer with pH 8.2 containing 20 mM Tris-HCl and 600-1000 mM NaCl.

SP Sepharose FF is used preferably as chromatography resin at the second stage of chromatographic purification.

Up to 2 mM DTT and 0.2 mM EDTA need to be added to the buffer to stabilize the target protein.

The achievement of unexpected technical result in the present invention is granted by following differences in comparation with the closest analogue, including additional purification of the target protein from bacterial endotoxin carried out by:
adding to the specified target protein solution obtained after chromatography of the detergent Triton X-114 for removing bacterial endotoxin through transition of Triton X-114 from the water-insoluble fraction to the water-soluble one and back, with the formation of micelles and the subsequent separation of water-soluble and water-insoluble fractions by centrifugation,
then again Triton X-114 is added to the water-soluble fraction obtained after centrifugation with consequent transition from the water-insoluble fraction to the water-soluble one and back, with the formation of micelles and separation by centrifugation,
at the same time, bacterial endotoxins removal from the recombinant nuclease preparation of the Cas family of the CRISPR/CAS system to a level that allows using this preparation in vivo, is performed by the steps of the Triton X-114 addings and are repeated for at least 6 times preferably from 6 to 9 times.

After the final centrifugation at the end of the additional purification stage, the aqueous phase including the purified recombinant nuclease of the Cas family is selected and if necessary, freezing or lyophilization is carried out with the addition of auxiliary substances for freezing or lyophilization, respectively.

At the stage of additional purification from endotoxins centrifugation is carried out preferably at 10,000 g for 10 minutes at 23 ° C.

Additional purification of the recombinant nuclease of the Cas family from endotoxins is carried out preferably by performing the following steps:
adding Triton X-114 detergent up to 1% to the target protein solution obtained after the chromatography purification stages;
placing the resulting solution on ice until absolute transparency of the solution is reached followed by holding at 37 ° C until absolute opacity of the solution is achieved in order to enrich the solution with micelles;
subsequent separation of the aqueous and micellar fractions of the solution by the centrifugation;
adding Triton X-114 detergent up to 1% to the upper aqueous phase obtained after centrifugation and repeating the treatment until a recombinant nuclease preparation of the Cas family with a bacterial endotoxin content is reduced to a level that allows using preparation in vivo.

Herewith, the solution is placed on ice and / or kept at 37°C, preferably for at least 15 minutes.

According to the proposed method, a recombinant nuclease of the Cas family of the CRISPR/Cas system characterized by an amino acid sequence selected from SEQ ID NO: 1-8 can be obtained.

Recombinant nuclease of the Cas family of the CRISPR/Cas system can be obtained by using a coding nucleotide sequence selected from SEQ ID NO: 9-16.

According to the proposed method, a recombinant nuclease preparation of the Cas family of the CRISPR/Cas system substantially free of bacterial endotoxins is obtained to be used in the CRISPR/Cas system.

Substantially, the bacterial endotoxin-free recombinant nuclease preparation of the Cas family of the CRISPR/Cas system contains less than 1.5 EU/ml of bacterial endotoxins, preferably 0.3 EU / ml.

The preparation can be in liquid form as an aqueous phase and include a purified recombinant nuclease of the Cas family obtained after final centrifugation at the end of the additional purification stage.

The recombinant nuclease preparation of the Cas family includes preferably the specified nuclease with an endotoxin content of 0.3-1.5 EU/ml in a buffer solution containing Tris-HCl, NaCl, DTT and EDTA, the most preferable are 20 mM Tris-HCl, 600-1000 mM NaCl, 2 mM DTT, 0,2 mM EDTA with pH 8,2.

The preparation may be in a dry form as a freeze-dried preparation preferably, obtained after final centrifugation at the end of the additional purification step by a freeze-dry of an aqueous phase comprising a purified recombinant nuclease of the Cas family.

A lyophilized recombinant nuclease preparation of the Cas family is prepared preferably by adding lyophilization excipients selected from glycerol or trehalose, glycerol at a concentration of 10-50% preferably or trehalose at a concentration of 50-500 mM to a buffer solution containing recombinant nuclease of the Cas family with 0,3-1,5 EU/ml, Tris-HCl, NaCl, DTT and EDTA, the most preferable are 20 mM Tris-HCl, 600-1000 mM NaCl, 2 mM DTT, 0,2 mM EDTA with pH 8,2, with subsequent lyophilization.

Depending on the purpose of use, the concentration of recombinant nuclease of the Cas family varies widely in the composition of the preparation from 0.3 mg/ml to 3 mg/ml.

Obtained recombinant nuclease preparation of the Cas family of the CRISPR/Cas system can be used in the CRISPR/Cas system as part of a kit with the user manuals.

The kit may additionally include a target-specific guide RNA. In this case, the recombinant nuclease preparation of the CRISPR/CAS family as part of the kit can be kept with a target-specific guide RNA in one container or separately in different containers.

Preparations and kits according to the present invention can be used in the CRISPR/CAS system in various fields of technology related to genome editing: for development of cellular models of hereditary diseases, functional screening of genomes, studying epigenomes and visualizing cellular processes, in the food industry for obtaining enriched food, in agriculture for creating new breeds of animals and plant varieties, as well as in medicine for the diagnosis, prevention and treatment of various diseases.

### BRIEF DESCRIPTION OF THE DRAWINGS

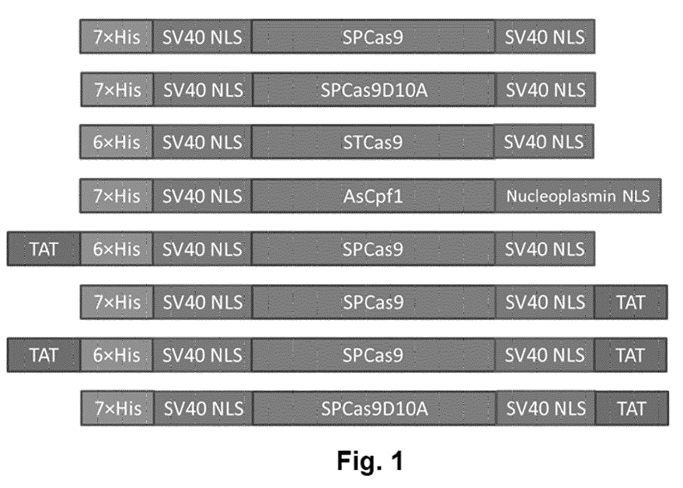

Fig. 1. Schemes of recombinant proteins of CRISPR/CAS family.
   6×His - polyhistidine sequence;
   SV40 NLS - signal of nuclear localization of the large T-antigen of the simian virus SV40 for effective protein delivery to the target cell nucleus;
   Nucleoplasmin NLS - signal of nuclear localization of nucleoplasmin, the first identified molecular chaperone;
   TAT - peptide of the human immunodeficiency virus;
   SPCas9 - Cas9 nuclease from *Streptococcus pyogenes;*
   SPCas9D10A - Cas9 nickase from *Streptococcus pyogenes;*
   STCas9 - Cas9 nuclease from *Streptococcus thermophilus;*
   AsCpfl - Cpfl nuclease from *Acidaminococcus;*
   TATSPCas9 - Cas9 nuclease from *Streptococcus pyogenes,* fused protein with TAT-peptide at N-end;
   SPCas9TAT - Cas9 nuclease from *Streptococcus pyogenes,* fused protein with TAT-peptide at C-end;
   TATSPCas9TAT - Cas9 nuclease from *Streptococcus pyogenes,* fused protein with TAT-peptide at N- and C-ends;
   SPCas9D10ATAT - Cas9 nickase from *Streptococcus pyogenes,* fused protein with TAT-peptide at C-end.

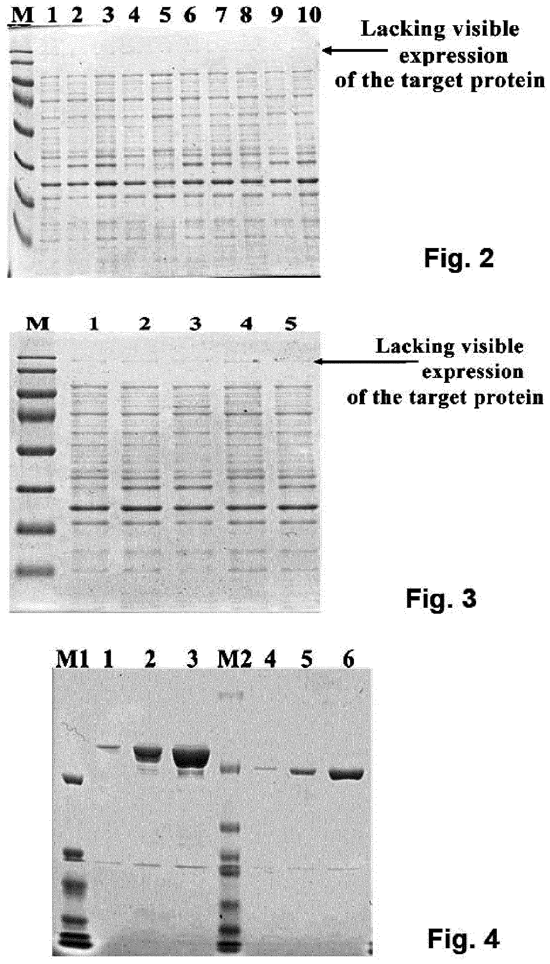

Fig. 2. Electropherogram of the soluble fractions containing recombinant CRISPR/CAS family proteins obtained during expression in BL21(DE3) E. coli cells, where numbers 1-10 mark the following:
   1 - soluble fraction of BL21(DE3) E. coli non-transformed with plasmid DNA pAM, carrying the gene, which contains the sequence SEQ ID NO: NO: 9-16;
   2 - soluble fraction of BL21(DE3) E. coli transformed with plasmid DNA pAM-SPCas9, non-induced with addition of IPTG;
   3 - soluble fraction of BL21(DE3) E. coli transformed with plasmid DNA pAM-SPCas9, induced with addition of IPTG, cultivated at room temperature for 4 hours;
   4 - soluble fraction of BL21(DE3) E. coli transformed with plasmid DNA pAM-SPCas9, induced with addition of IPTG, cultivated at room temperature for 20 hours;
   5 - soluble fraction of BL21(DE3) E. coli transformed with plasmid DNA pAM-SPCas9D10A, non-induced with addition of IPTG;
   6 - soluble fraction of BL21(DE3) E. coli transformed with plasmid DNA pAM-SPCas9D10A, induced with addition of IPTG, cultivated at room temperature for 4 hours;
   7 - soluble fraction of BL21(DE3) E. coli transformed with plasmid DNA pAM-SPCas9D10A, induced with addition of IPTG, cultivated at room temperature for 20 hours;
   8 - soluble fraction of BL21(DE3) E. coli transformed with plasmid DNA pAM-AsCpf1, non-induced with addition of IPTG;
   9 - soluble fraction of BL21(DE3) E. coli transformed with plasmid DNA pAM-AsCpf1, induced with addition of IPTG, cultivated at room temperature for 4 hours;
   10 - soluble fraction of BL21(DE3) E. coli transformed with plasmid DNA pAM-AsCpfl, induced with addition of IPTG, cultivated at room temperature for 20 hours; The letter M indicates molecular weight standards,
      the arrow marks the area of the gel where the recombinant proteins of the CRISPR/CAS family were calculated to be located.
Fig. 3. Electropherogram of soluble fractions containing recombinant CRISPR/CAS family proteins obtained during expression in E. coli Rosetta-gami B (DE3) cells using a starting culture grown in Luria-Bertani broth that does not contain 2 mM MgSO₄ and 2% glucose, where the numbers 1-5 denote:
   1 - soluble fraction of Rosetta-gami B (DE3) E. coli non-transformed with plasmid DNA pAM carrying the gene, containing the sequence SEQ ID NO: NO: 9-16;
   2 - soluble fraction of Rosetta-gami B (DE3) E. coli transformed with plasmid DNA pAM carrying the gene, containing the sequence SEQ ID NO: NO: 9, non-induced with addition of IPTG;
   3 - soluble fraction of Rosetta-gami B (DE3) E. coli transformed with plasmid DNA pAM carrying the gene, containing the sequence SEQ ID NO: NO: 9, induced with addition of IPTG, cultivated at room temperature for 20 hours;
   4 - soluble fraction of Rosetta-gami B (DE3) E. coli transformed with plasmid DNA pAM, carrying the gene, containing the sequence SEQ ID NO: NO: 10, non-induced with addition of IPTG;
   5 - soluble fraction of Rosetta-gami B (DE3) E. coli transformed with plasmid DNA pAM, carrying the gene, containing the sequence SEQ ID NO: NO: 10, induced with addition of IPTG, cultivated at room temperature for 20 hours;
      the letter M indicates the molecular weight standards.
   The arrow marks the area of the gel where the recombinant proteins of the CRISPR/CAS family were calculated to be located.
Fig. 4. Electropherogram of the protein preparations containing recombinant proteins of the CRISPR/CAS family SPCas9 and STCas9, obtained during chromatographic purification; where the numbers 1-6 denote the following:
   1 - preparation SPCas9 obtained by chromatographic protein purification from the soluble cytoplasm of the producing strain induced when OD₆₀₀=0,6-1,0;
   2 - preparation SPCas9 obtained by chromatographic protein purification from the soluble cytoplasm of the producing strain induced when OD₆₀₀=1,1-1,4;
   3 - preparation SPCas9 obtained by chromatographic protein purification from the soluble cytoplasm of the producing strain induced when OD₆₀₀=1,5-1,9;
   4 - preparation STCas9 obtained by chromatographic protein purification from the soluble cytoplasm of the producing strain induced when OD₆₀₀=0,6-1,0;
   5 - preparation STCas9 obtained by chromatographic protein purification from the soluble cytoplasm of the producing strain induced when OD₆₀₀=1,1-1,4;
   6 - preparation STCas9 obtained by chromatographic protein purification from the soluble cytoplasm of the producing strain induced when OD₆₀₀=1,5-1,9;
      the letters M1 and M2 indicate the markers of the molecular weights.
Fig. 5. Chromatographic purification profile of recombinant protein SPCas9 belonging to CRISPR/CAS family on Chelating sepharose using buffer with pH=6,6; where the numbers 1-5 denote the following process stages:
   1 - sample loading;
   2 - column wash to remove the unbound proteins;
   3 - protein elution with solution containing 50 mM of imidazole;
   4 - protein elution with solution containing 250 mM of imidazole;
   5 - column wash with solution containing 1 M of sodium chloride.
Fig. 6. Chromatographic purification profile of recombinant protein SPCas9 belonging to CRISPR/CAS family on Chelating sepharose using buffer with pH=7,3; where the numbers 1-5 denote the following process stages:
   1 - sample loading;
   2 - column wash to remove the unbound proteins;
   3 - protein elution with solution containing 50 mM of imidazole;
   4 - protein elution with solution containing 250 mM of imidazole;
   5 - column wash with solution containing 1 M of sodium chloride.
Fig. 7. Chromatographic purification profile of recombinant protein SPCas9 belonging to CRISPR/CAS family on Chelating sepharose using buffer with pH=8,0; where the numbers 1-5 denote the following process stages:
   1 - sample loading;
   2 - column wash to remove the unbound proteins;
   3 - protein elution with solution containing 50 mM of imidazole;
   4 - protein elution with solution containing 250 mM of imidazole;
   5 - column wash with solution containing 1 M of sodium chloride.
Fig. 8. Electropherogram of chromatographic fractions containing recombinant protein SPCas9 of CRISPR/CAS family obtained by purification on Chelating sepharose; where the numbers 1-4 denote the following fractions:
   1 - soluble fraction of Rosetta-gami B (DE3) Escherichia coli transformed with plasmid DNA pAM carrying the gene, containing the sequence SEQ ID NO: NO: 9;
   2 - flow-through (a chromatographic fraction containing all proteins not bound to the column);
   3 - eluate, containing 50 mM of imidazole;
   4 - eluate, containing 250 mM of imidazole;
      the letter M indicates the standards of the molecular weights. The arrows mark the position of the recombinant protein SPCas9 of the CRISPR/CAS family in the gel.

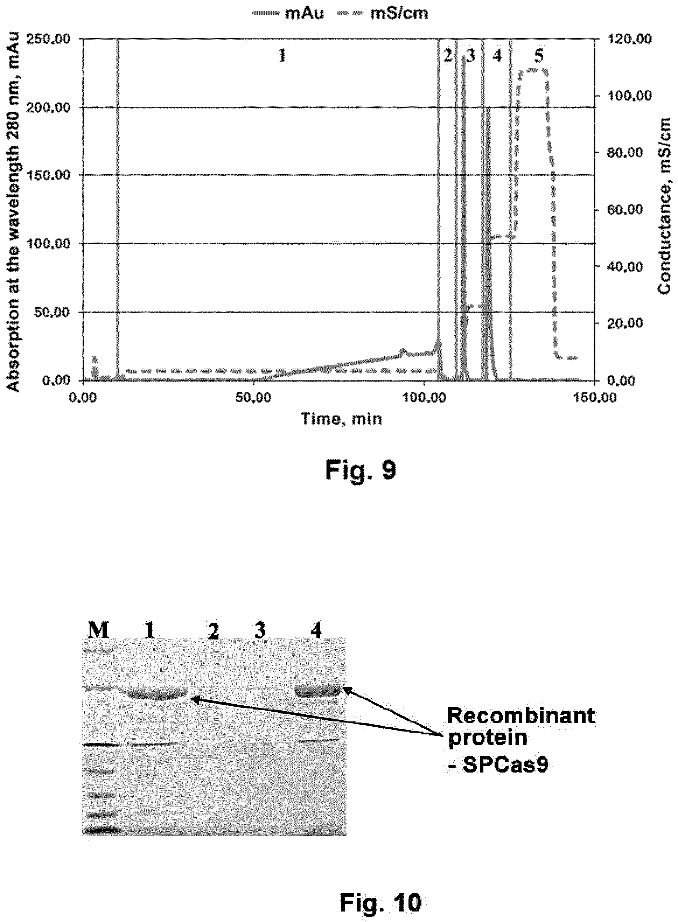

Fig. 9. Chromatographic purification profile of recombinant protein SPCas9 belonging to CRISPR/CAS family on SP Sepharose; where the numbers 1-5 denote the following process stages:
   1 - sample loading;
   2 - column wash to remove the unbound proteins;
   3 - protein elution with solution containing 300 mM of sodium chloride;
   4 - protein elution with solution containing 600 mM of sodium chloride;
   5 - column wash with solution containing 1 M of sodium chloride.
Fig. 10. Electropherogram of chromatographic fractions containing recombinant protein SPCas9 of CRISPR/CAS family, obtained by purification on SP Sepharose; where the numbers 1-4 denote the following fractions:
   1 - eluate from the first stage purification step on Chelating sepharose containing the recombinant protein SPCas9 (250 mM of imidazole);
   2 - flow-through (a chromatographic fraction containing all proteins not bound to the column);
   3 - eluate containing 300 mM of sodium chloride;
   4 - eluate containing 600 mM of sodium chloride;
      the letter M indicates the standards of the molecular weights. The arrows mark the position of the recombinant protein SPCas9 of the CRISPR/CAS family in the gel.

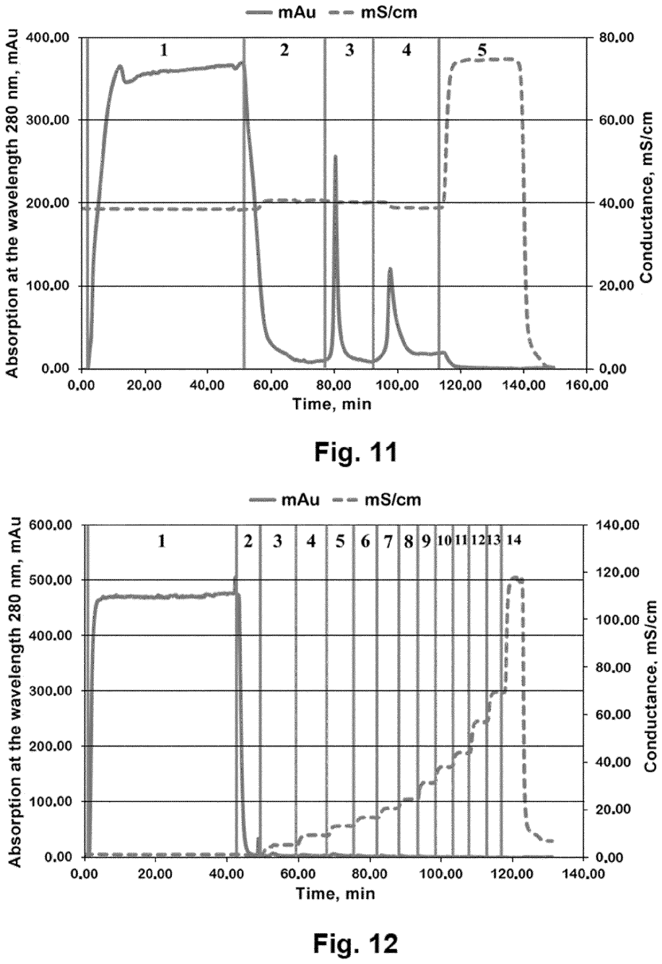

Fig. 11. Chromatographic purification profile of recombinant protein SPCas9D10A belonging to CRISPR/CAS family on Chelating sepharose using buffer with pH=6,6; protein expression was conducted at the temperature of 27°C for 20 hours; where the numbers 1-5 denote the following process stages:
   1 - sample loading;
   2 - column wash to remove the unbound proteins;
   3 - protein elution with solution containing 50 mM of imidazole;
   4 - protein elution with solution containing 250 mM of imidazole;
   5 - column wash with solution containing 1 M of sodium chloride.
Fig. 12. Chromatographic purification profile of recombinant protein SPCas9D10A belonging to CRISPR/CAS family on SP Sepharose; protein expression was conducted at the temperature of 27°C for 20 hours; where the numbers 1-14 denote the following process stages:
   1 - sample loading;
   2 - column wash to remove the unbound proteins;
   3 - protein elution with solution containing 50 mM of sodium chloride;
   4 - protein elution with solution containing 100 mM of sodium chloride;
   5 - protein elution with solution containing 150 mM of sodium chloride;
   6 - protein elution with solution containing 200 mM of sodium chloride;
   7 - protein elution with solution containing 250 mM of sodium chloride;
   8 - protein elution with solution containing 300 mM of sodium chloride;
   9 - protein elution with solution containing 400 mM of sodium chloride;
   10 - protein elution with solution containing 500 mM of sodium chloride;
   11 - protein elution with solution containing 600 mM of sodium chloride;
   12 - protein elution with solution containing 800 mM of sodium chloride;
   13 - protein elution with solution containing 1 M of sodium chloride;
   14 - column wash with solution containing 2 M of sodium chloride.

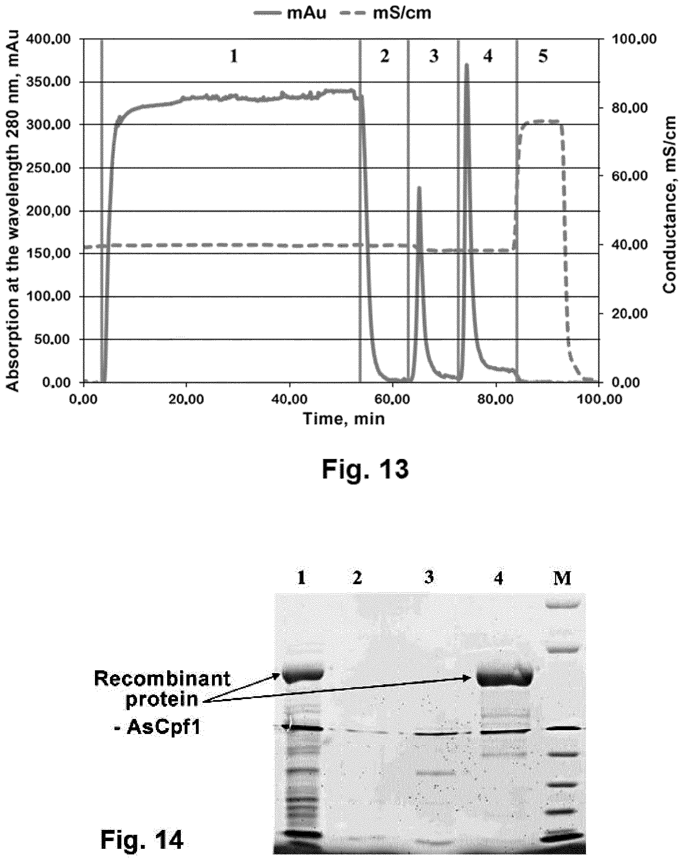

Fig. 13. Chromatographic purification profile of recombinant protein AsCpfl belonging to CRISPR/CAS family on Chelating sepharose using buffer with pH=6,6; where the numbers 1-5 denote the following process stages:
   1 - sample loading;
   2 - column wash to remove the unbound proteins;
   3 - protein elution with solution containing 50 mM of imidazole;
   4 - protein elution with solution containing 250 mM of imidazole;
   5 - column wash with solution containing 1 M of sodium chloride.
Fig. 14. Electropherogram of chromatographic fractions containing recombinant protein AsCpfl of CRISPR/CAS family obtained by purification on Chelating sepharose; where the numbers 1-4 denote the following fractions:
   1 - soluble fraction of Rosetta-gami B (DE3) Escherichia coli transformed with plasmid DNA pAM carrying the gene, containing the sequence SEQ ID NO: NO: 12;
   2 - flow-through (a chromatographic fraction containing all proteins not bound to the column);
   3 - eluate containing 50 mM of imidazole;
   4 - eluate containing 250 mM of imidazole;
      The letter M indicates the standards of the molecular weights,
   The arrows indicate the location of the recombinant protein AsCpfl of the CRISPR/CAS family in the gel.

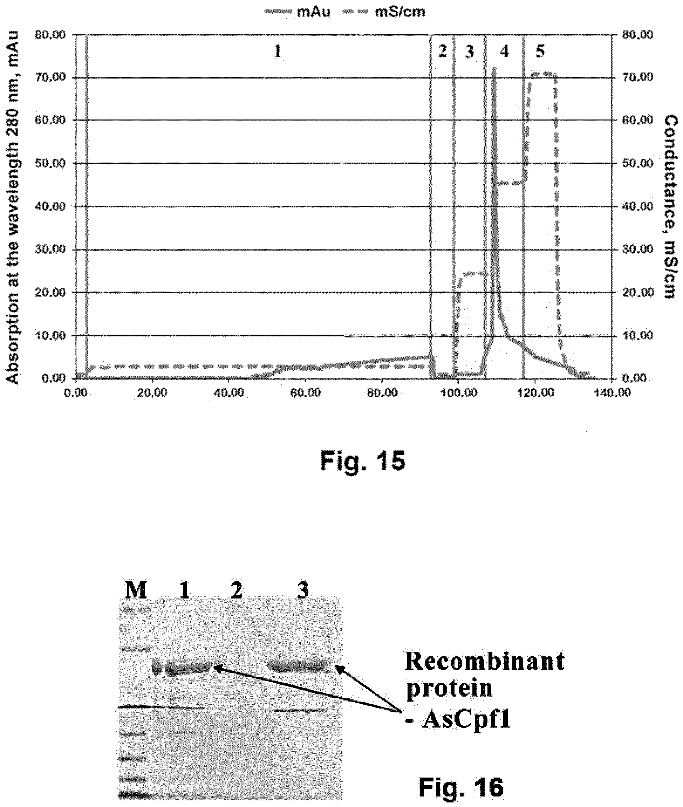

Fig. 15. Chromatographic purification profile of recombinant protein AsCpfl belonging to CRISPR/CAS family on SP Sepharose; where the numbers 1-5 denote the following process stages:
   1 - sample loading;
   2 - column wash to remove the unbound proteins;
   3 - protein elution with solution containing 300 mM of sodium chloride;
   4 - protein elution with solution containing 600 mM of sodium chloride;
   5 - column wash with solution containing 1 M of sodium chloride.
Fig. 16. Electropherogram of chromatographic fractions containing recombinant protein AsCpfl of the CRISPR/CAS family, obtained by purification on SP Sepharose; where the numbers 1-3 denote the following fractions:
   1 - eluate from the first stage purification step on Chelating sepharose containing the recombinant protein AsCpfl (250 mM of imidazole);
   2 - flow-through (a chromatographic fraction containing all proteins not bound to the column);
   3 - eluate containing 600 mM of sodium chloride;
      The letter M indicates the standards of the molecular weights,
   The arrows indicate the location of the recombinant protein AsCpfl of CRISPR/CAS family in the gel.

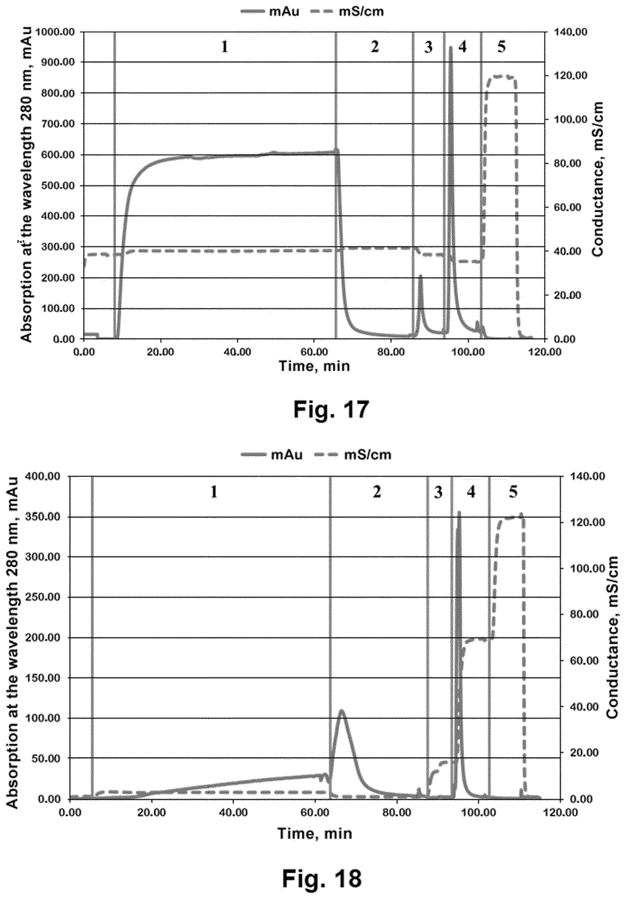

Fig. 17. Chromatographic purification profile of recombinant protein STCas9 belonging to CRISPR/CAS family on Chelating sepharose using buffer with pH=6,6; where the numbers 1-5 denote the following process stages:
   1 - sample loading;
   2 - column wash to remove the unbound proteins;
   3 - protein elution with solution containing 50 mM of imidazole;
   4 - protein elution with solution containing 250 mM of imidazole;
   5 - column wash with solution containing 1 M of sodium chloride.
Fig. 18. Chromatographic purification profile of recombinant protein STCas9 belonging to CRISPR/CAS family on SP Sepharose; where the numbers 1-5 denote the following process stages:
   1 - sample loading;
   2 - column wash to remove the unbound proteins;
   3 - protein elution with solution containing 300 mM of sodium chloride;
   4 - protein elution with solution containing 1 M of sodium chloride;
   5 - column wash with solution containing 2 M of sodium chloride.

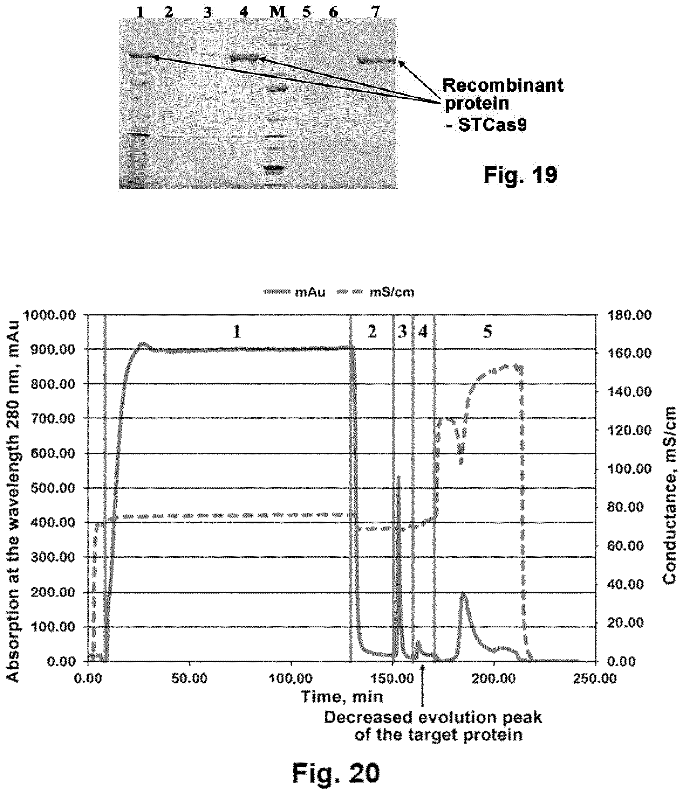

Fig. 19. Electropherogram of chromatographic fractions containing recombinant protein STCas9 of the CRISPR/CAS family, obtained by purification on Chelating sepharose and SP Sepahrose; where the numbers 1-7 denote the following fractions:
   1 - soluble fraction of Rosetta-gami B (DE3) Escherichia coli transformed with plasmid DNA pAM carrying the gene, containing the sequence SEQ ID NO: NO: 11;
   2 - first stage flow-through (a chromatographic fraction containing all proteins not bound to the column with Chelating Sepharose);
   3 - first stage eluate containing 50 mM of imidazole;
   4 - first stage eluate containing 250 mM of imidazole;
   5 - second stage flow-through (a chromatographic fraction containing all proteins not bound to the column with SP Sepharose);
   6 - second stage eluate containing 300 mM of sodium chloride;
   7 - second stage eluate containing 1 M of sodium chloride;
      The letter M indicates the standards of the molecular weights,
   The arrows indicate the location of the recombinant protein STCas9 of the CRISPR/CAS family in the gel.
Fig. 20. Chromatographic purification profile of recombinant protein SPCas9 belonging to CRISPR/CAS family on Chelating sepharose (disintegration and chromatography were carried out in buffer with sodium chloride concentration of 1 M); where the numbers 1-5 denote the following process stages:
   1 - sample loading;
   2 - column wash to remove the unbound proteins;
   3 - protein elution with solution containing 50 mM of imidazole;
   4 - protein elution with solution containing 250 mM of imidazole;
   5 - column wash with solution containing 2 M of sodium chloride.

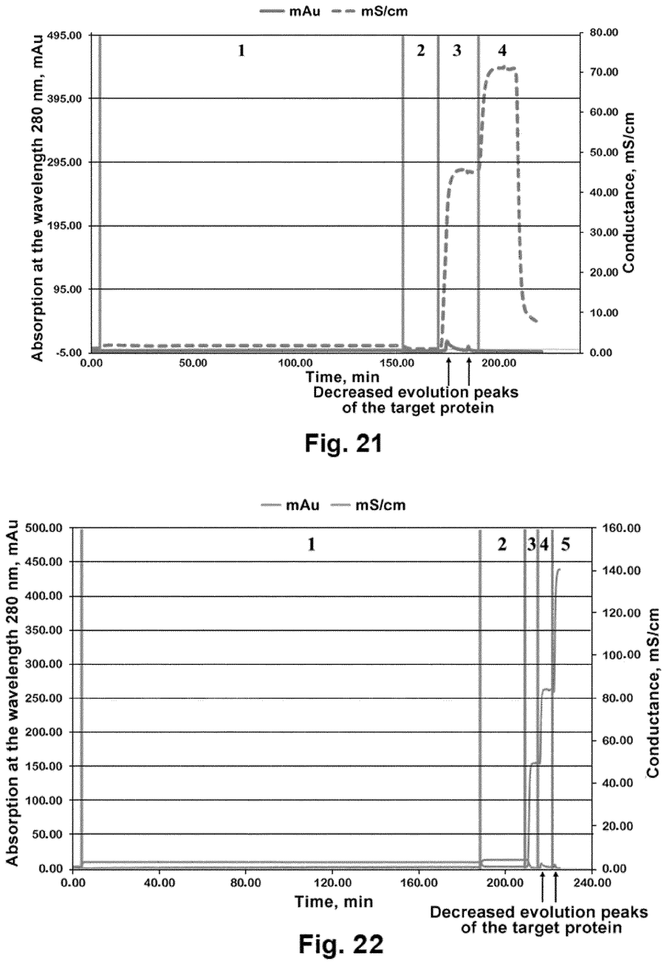

Fig. 21. Chromatographic purification profile of recombinant protein SPCas9 belonging to CRISPR/CAS family on SP Sepharose (disintegration of the protein producing cells was carried out in buffer with pH=9,5); where the numbers 1-4 denote the following process stages:
   1 - sample loading;
   2 - column wash to remove the unbound proteins;
   3 - protein elution with solution containing 600 mM of sodium chloride;
   4 - column wash with solution containing 1 M of sodium chloride.
Fig. 22. Chromatographic purification profile of recombinant protein SPCas9 belonging to CRISPR/CAS family on SP Sepharose (chromatography was carried out in buffer with pH=4,0); where the numbers 1-5 denote the following process stages:
   1 - sample loading;
   2 - column wash to remove the unbound proteins;
   3 - protein elution with solution containing 600 mM of sodium chloride;
   4 - protein elution with solution containing 1,2 M of sodium chloride;
   5 - column wash with solution containing 2,4 M of sodium chloride.

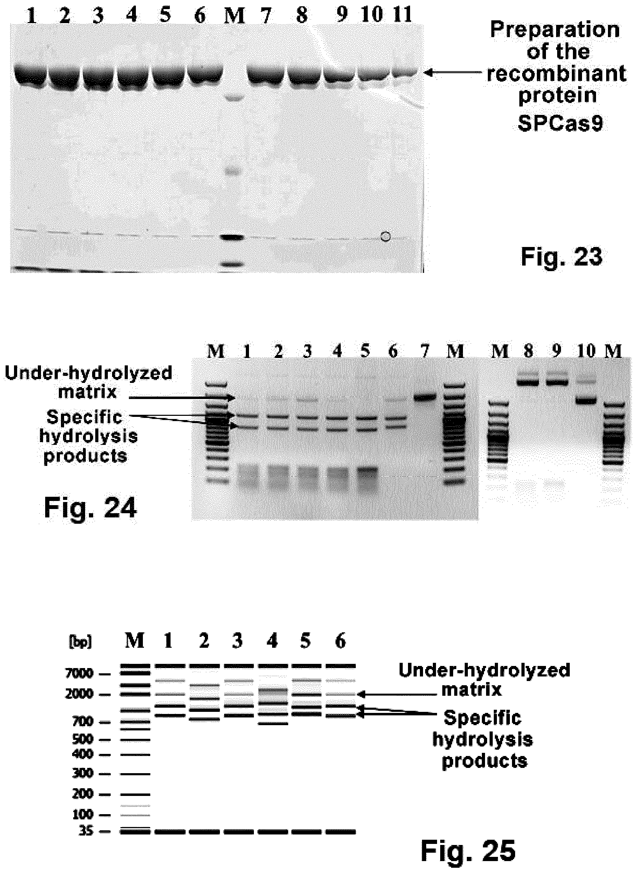

Fig. 23. Electropherogram of the preparations of the recombinant protein SPCas9 of the CRISPR/CAS family containing excipients for lyophilization, such as glycerol and trehalose; where the numbers 1-11 denote the preparations:
   1 - containing 50mM of trehalose;
   2 - containing 100mM of trehalose;
   3 - containing 150mM of trehalose;
   4 - containing 200mM of trehalose;
   5 - containing 250mM of trehalose;
   6 - containing 500mM of trehalose;
   7 - containing 10% of glycerol;
   8 - containing 20% of glycerol;
   9 - containing 30% of glycerol;
   10 - containing 40% of glycerol;
   11 - containing 50% of glycerol;
      The letter M indicates the standards of the molecular weights,
   The arrow indicates the location of the preparation of the recombinant protein SPCas9 of the CRISPR/Cas family in the gel.
Fig. 24. Electropherogram of DNA fragments treated with CRISPR/Cas family proteins in presence of the target-specific guide RNA; where the numbers 1-10 denote the following samples:
   1 - hydrolysis products after treatment with SPCas9;
   2 - hydrolysis products treatment with TATSPCas9;
   3 - hydrolysis products after treatment with SPCas9TAT;
   4 - hydrolysis products after treatment with TATSPCas9TAT;
   5 - hydrolysis products after treatment with EnGen Cas9 (NEB);
   6 - hydrolysis products after treatment with AsCpfl;
   7 - non-hydrolysed DNA matrix, obtained by PCR;
   8 - hydrolysis products after treatment with SPCas9D10A;
   9 - hydrolysis products after treatment with SPCas9D10ATAT;
   10 - non-hydrolysed DNA matrix (supercoiled plasmid DNA);
   The letter M indicates the standards of the molecular weights.
Fig. 25. Analysis of DNA fragments treated with recombinant proteins of the CRISPR/Cas family in the presence of target-specific guide RNA performed on an Agilent Bioanalyzer 2100; where the numbers 1-6 denote the following samples:
   1 - EnGen Cas9 (NEB),
   2 - TATSPCas9,
   3 - TATSPCas9TAT,
   4 - SPCas9TAT,
   5 - AsCpfl,
   6 - SPCas9;
   The letter M indicates the standards of the molecular weights.
Table 1. Features of the obtained expression constructions.
Table 2. Recombinant proteins of the CRISPR/Cas family.
Table 3. The content of bacterial endotoxins in solutions of recombinant proteins of the CRISPR/Cas family reduced with the help of the detergent Triton X-114 on the example of proteins SPCas9, STCas9, AsCpfl.
Table 4. Efficiency of specific hydrolysis of target DNA by recombinant CRISPR/CAS family proteins in the presence of target-specific guide RNA.

### DESCRIPTION OF EMBODIMENTS

### EXAMPLE 1: CONSTRUCTION OF PLASMID DNAs FOR EXPRESSION OF THE RECOMBINANT PROTEINS OF CRISPR/CAS FAMILY IN E. COLI.

To produce recombinant proteins of CRISPR/CAS family in E. coli commercially available pET22b plasmid (Novagen, USA) was used as a vector plasmid DNA to construct the expression plasmid DNAs carrying the gene containing one of the SEQ ID NO: NO: 9-16 sequences. The pET22b map is available on site https://www.snapgene.com/resources/plasmidfiles/?set=pet and duet vectors (novagen)& plasmid=pET-22b(+). Before using pET22b as a vector an additional site for recognition of NsiI restriction endonuclease was introduced into the multiple cloning site with the QuikChange XL Site-Directed Mutagenesis Kit (Agilent, USA). The commercially available plasmids from the repository (Addgene, USA) were used as sources of fragments for cloning and these fragments were obtained by means of PCR with use of the developed primers (tab. 1). The structure of the cloned fragments is schematically represented on Fig. 1. The primers used for PCR contain NsiI nuclease restriction sites (on 5'-end) and NotI (on 3'-end) and also DNA fragments coding the sequences of the poly-histidine tract (on 5'-end), signal of the nuclear localization of the big T-antigen of simian virus SV40 (on 5'-end) for the efficient protein delivery to the target cells and the sequence coding the TAT - peptide of the human immunodeficiency virus (on 5'- and/or 3'-end).

The products of the amplification obtained during the PCR with the developed primers (tab. 1) were cleaved by NsiI and NotI restriction endonucleases (except for the fragment coding the STCas9 nuclease, where NdeI and AgeI were used) and then they were ligated into the agarose gel purified vector plasmid DNA modified pET22b(NsiI) cleaved by appropriate restriction endonucleases. The single clones containing pET22b(NsiI) plasmid with the target insert were obtained using chemically competent MACH1-T1 E. coli strain (Thermo Fisher Scientific, USA). The clones containing the insert of the required length were identified by means of the PCR with the primers T7 forward and T7 reverse (GenTerra, Russia). The plasmid DNA was purified and the correctness of the inserted sequence was verified using Sanger sequencing.

As a result, the expression plasmid DNA carrying the gene containing one of SEQ ID NO: NO: 9-16 sequences were obtained for production of the recombinant proteins of CRISPR/CAS family in E. coli (tab. 1, tab. 2):
1. pAM-SPCas9 sized 9852 bps carrying the gene, containing the sequence SEQ ID NO: NO: 9. It codes the Cas9 nuclease from *Streptococcus pyogenes* with the amino acid sequence SEQ ID NO: NO: 1. pAM-SPCas9 contains 33 unique restriction sites including *BamH*I (7016), *EcoR*I (8833), *Not*I (9167), *Nsi*I (4964), *Sal*I (5551), *Sma*I (6379), *Xba*I (4918).
2. pAM-SPCa9D10A sized 9852 bps carrying the gene, containing the sequence SEQ ID NO: NO: 10. It codes the Cas9 nickase from *Streptococcus pyogenes* with the amino acid sequence from SEQ ID NO: NO: 2. pAM-SPCas9D10A contains 34 unique restriction sites including *BamH*I (7016), *EcoR*I (8833), *Not*I (9167), *Nsi*I (4964), *Sal*I (5551), *Sma*I (6379), *Xba*I (4918).
3. pAM-STCas9 sized 8873 bps carrying the gene, containing the sequence SEQ ID NO: NO: 11. It codes the Cas9 nuclease from *Streptococcus thermophilus* with the amino acid sequence SEQ ID NO: NO: 3. pAM-STCas9 contains 24 unique restriction sites including *Age*I (8444), *Nco*I (5966), *Nde*I (4958), *Pvu*I (772), *Xba*I(4918).
4. pAM-AsCpf1 sized 9405 bps carrying the gene, containing the sequence SEQ ID NO: NO: 12. It codes the Cpfl nuclease from *Acidaminococcus* with the amino acid sequence SEQ ID NO: NO: 4. pAM-AsCpf1 contains 28 unique restriction sites, including *BssH*II (3719), *Nde*I (4958), *Not*I (8990), *Nsi*I (4964), *Xba*I(4918), *Xho*I (8998).
5. pAM-TATSPCas9 sized 9654 bps carrying the gene, containing the sequence SEQ ID NO: NO: 13. It codes the Cas9 nuclease from *Streptococcus pyogenes* (fused protein with TAT-peptide on N-end) with the amino acid sequence SEQ ID NO: NO: 5. pAM-TATSPCas9 contains 33 unique restriction sites, including *BamH*I (2094), *EcoR*I (3911), *Not*I (4275), *Nsi*I (0), *Xbal* (9608).
6. pAM-SPCas9TAT sized 9612 bps carrying the gene, containing the sequence SEQ ID NO: NO: 14. It codes the Cas9 nuclease from *Streptococcus pyogenes* (fused protein with TAT-peptide on C-end) with the amino acid sequence SEQ ID NO: NO: 6. pAM-SPCas9TAT contains 33 unique restriction sites, including *BamH*I (7016), *EcoR*I (8833), *Not*I (9197), *Nsi*I (4964), *Xba*I (4918).
7. pAM-TATSPCas9TAT sized 9654 bps carrying the gene, containing the sequence SEQ ID NO: NO: 15. It codes the Cas9 nuclease from *Streptococcus pyogenes* (fused protein with TAT-peptide on N- and C-ends) with the amino acid sequence SEQ ID NO: NO: 7. pAM-TATSPCas9TAT contains 33 unique restriction sites, including *BamH*I (7058), *EcoR*I (8875), *Not*I (9239), *Nsi*I (4964), *Xba*I(4918).
8. pAM-SPCa9D10ATAT sized 9612 bps carrying the gene, containing the sequence SEQ ID NO: NO: 16. It codes the Cas9 nickase from *Streptococcus pyogenes* (fused protein with TAT-peptide on C-end) with the emino acid sequence SEQ ID NO: NO: 8. pAM-SPCa9D10ATAT contains 33 unique restriuction sites, including *BamH*I (7016), *EcoR*I (8833), *Not*I (9197), *Nsi*I (4964), *Xba*I(4918).

### EXAMPLE 2: EXPRESSION AND OBTAINING OF THE SOLUBLE FRACTION OF THE RECOMBINANT PROTEINS OF CRISPR/CAS FAMILY IN E. COLI.

Recombinant CRISPR/CAS family protein-producing strains for pilot expression were obtained by chemical transformation of competent BL21(DE3) E. coli cells using pAM plasmid DNA (Table 1), carrying the gene containing one of the SEQ ID NO: NO: 9-16 sequences. Pilot expression was performed in Luria-Bertani broth (LB) containing 100 mkg / ml of ampicillin, 2 mM MgSO₄ and 1% glucose. To analyse the expression of recombinant CRISPR/CAS family protein soluble fraction of the cytoplasm was isolated from protein-producing cells and analysed by electrophoresis in 7.5% polyacrylamide gel. After electrophoresis, the gel was stained with Coomassie R-250 according to the standard Lammley method and scanned (Epson, Japan). During the pilot expression it was not manageable to obtain the visible expression of the proteins SPCas9, SPCas9D10A and AsCpfl in the producing strains, obtained based on the parental strain BL21(DE3) E. coli and this can be proved by absence of the visible bands with the molecular weight of 161836,42 Da (the calculated molecular weight of SPCas9) in the lanes 2-4, 161792,41 Da (the calculated molecular weight of SPCas9D10A) in the lanes 5-7 and 155028,12 Da (the calculated molecular weight of AsCpfl) in the ways 8-10 in a stained acrylamide gel when matched with a molecular weight marker (from top to bottom 180, 130, 100, 70, 55, 40, 35, 25, 15, 10 kDa, PageRuler^{™} Prestained Protein Ladder, 10 to 180 kDa, # 26616, USA) (fig. 2).

Due to the absence of expression of the target proteins in the BL21(DE3) strain of E. coli it was decided to change the parental expression strain to Rosetta-gami B (DE3) E. coli, having the genotype: F- ompT hsdSB (rB- mB-) gal dcm lacY1 ahpC (DE3) gor522::Tn10 trxBpRARE (CamR, KanR, TetR) able to efficiently express the target proteins coded with the sequences, which are not codon-optimized for E. coli and able to form the disulphide bonds of the produced target protein in the bacterial cytoplasm.

The producing strains of the recombinant CRISPR/CAS family proteins were obtained by method of chemical transformation of competent Rosetta-gami B (DE3) E. coli cells with use of plasmid DNA pAM, carrying the gene containing one of the sequences SEQ ID NO: NO: 9-16.

Starting culture of the recombinant protein-producing cells was obtained by inoculation of the separate clone of the producing strain into the Luria-Bertani broth (LB), containing 100 mkg/ml of ampicillin, 2 mM MgSO₄ and 2% of glucose. Cultivation of the starting culture was carried out at 37°C overnight with constant stirring. In the course of the experiments could be stated that the absence of MgSO₄ and glucose in the culture medium leads to a lack of expression of the target product as indicated by the absence of visible bands with molecular weight 161836,42 Da (the calculated molecular weight of SPCas9) in the lane 3 and 161792,41 Da (the calculated molecular weight of SPCas9D10A) in the lane 5 in a stained acrylamide gel when matched with a molecular weight marker (from top to bottom 180, 130, 100, 70, 55, 40, 35, 25, 15, 10 kDa, PageRuler^{™} Prestained Protein Ladder, 10 to 180 kDa, # 26616, USA) (fig. 3, the arrow indicates the area of the polyacrylamide gel where the bands of the calculated molecular weight should be located).

Next day the cells were diluted to the optical density of OD₆₀₀≈0,3 in the culture medium LB containing 100 mkg/ml of ampicillin, 2 mM MgSO₄ and 1% of glucose and were grown at 37°C with constant stirring until the optical density OD₆₀₀≈1,5-1,9 was obtained, since it was shown that target protein yield greatly increased if IPTG was added when OD₆₀₀ was 1,5-1,9 (fig. 4).

When the optical density of OD₆₀₀≈1,5-1,9 was achieved the isopropyl-β-D-thiogalactoside (IPTG) was added to the culture to final concentration of 0,5 mM for expression induction. After that protein-producing cells were incubated at 22°C for 20-24 hours with constant stirring.

After the cultivation, the cells were collected using centrifugation (6000 g, 10 min), then the obtained cell pellets were resuspended in the lysis buffer containing 20 mM Na₂HPO₄/NaH₂PO₄ pH 6,6-8,0.

The cells of the producing strain, resuspended in the lysis buffer containing 20 mM Na₂HPO₄/NaH₂PO₄ pH 6,6-8,0, were destructed using ultrasound disintegration technique (see for example, the method of the ultrasound disintegration described in the dissertation by Lebedev L.R., Development of technologies, to obtain the preparations of the natural and recombinant proteins from the prokaryotic cells, Synopsis of Thesis, Berdsk, 1998, 136p.).

Separation of the soluble fraction containing the recombinant protein of the CRISPR/CAS family from the insoluble fraction of the inclusion bodies in the solution, obtained after the ultrasound disintegration was carried out by centrifugation (10 000 g, 1 hour). The soluble fraction containing the recombinant protein of the CRISPR/CAS family obtained in such a way was subjected to the chromatographic purification.

### EXAMPLE 3. CHROMATOGRAPHIC PURIFICATION OF THE RECOMBINANT PROTEINS OF THE CRISPR/CAS FAMILY FROM THE SOLUBLE FRACTION OF E. COLI

Sodium chloride to a final concentration of 500 mM and imidazole to a final concentration of 10 mM were added to the soluble fraction obtained by ultrasonic disintegration and centrifugation methods containing a recombinant protein of the CRISPR/CAS family.

At the 1 stage of chromatographic purification, the solution prepared in this way was subjected to a metal-affinity chromatography on the chromatography resin Chelating Sepharose FF (GE Healthcare, Great Britain).

To prepare a chromatographic column with Chelating Sepharose FF metal-affinity chromatography resin charged with divalent nickel ions 10 ml of Chelating Sepharose FF metal-affinity resin were placed into the XK 16/20 Column (GE Healthcare, UK) and washed with 20 column volumes of deionized water. The column was charged with nickel ions by washing with 5 column volumes of 200 mM solution of nickel chloride, followed by washing with 20 column volumes of deionized water.

Before starting the process of chromatographic purification of recombinant protein of the CRISPR/CAS family from the soluble fraction of E. coli containing it, the column was equilibrated with 5 column volumes of buffer containing 20 mM Na₂HPO₄/NaH₂PO₄ pH 6,6-8,0, 500 mM of sodium chloride, 10 mM of imidazole. The soluble fraction containing the recombinant protein of the CRISPR/CAS family, 500 mM of sodium chloride and 10 mM of imidazole was applied to the metal-affinity chromatography resin Chelating Sepharose FF, charged with divalent nickel ions, at a flow rate of 10 ml/min.

After loading, all proteins not binding with the Chelating Sepharose FF resin were removed by washing the column with 5-10 column volumes of buffer containing 20 mM Na₂HPO₄/NaH₂PO₄ pH 6,6-8,0, 500 mM of sodium chloride, 10 mM of imidazole. Removal of protein impurities bound to the Chelating Sepharose FF resin was carried out by washing the column with 5-10 column volumes of the buffer, containing 20 mM of Na₂HPO₄/NaH₂PO₄ pH 6,6-8,0, 500 mM of sodium chloride, 50 mM of imidazole. Elution of the CAS-proteins was carried out with the buffer containing 20 mM of Na₂HPO₄/NaH₂PO₄ pH 6,6-8,0, 500 mM of sodium chloride, 250 mM of imidazole.

After the target protein elution, the chromatographic column containing the metal-affinity chromatography resin Chelating Sepharose FF was washed with the buffer containing 20 mM of Na₂HPO₄/NaH₂PO₄ pH 6,6-8,0, 1 M of sodium chloride. The chromatographic column was stored in the 20% ethanol solution at +4°C after being washed.

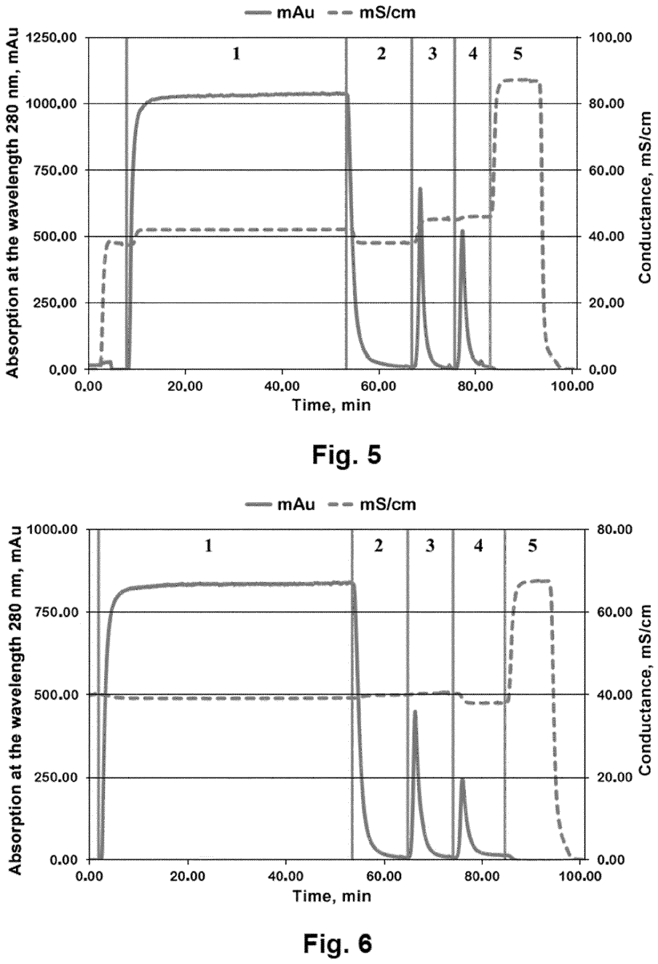

Typical chromatographic purification profiles of recombinant proteins belonging to CRISPR/CAS family on the Chelating Sepharose FF resin are shown on the examples of the protein purification profiles SPCas9, AsCpfl and STCas9 on Fig. 5, 6, 7, 13 and 17 respectively.

Chromatographic fractions containing the recombinant CRISPR/CAS family proteins obtained during chromatographic purification on Chelating Sepharose FF resin were analysed with the electrophoresis in 7.5% polyacrylamide gel.

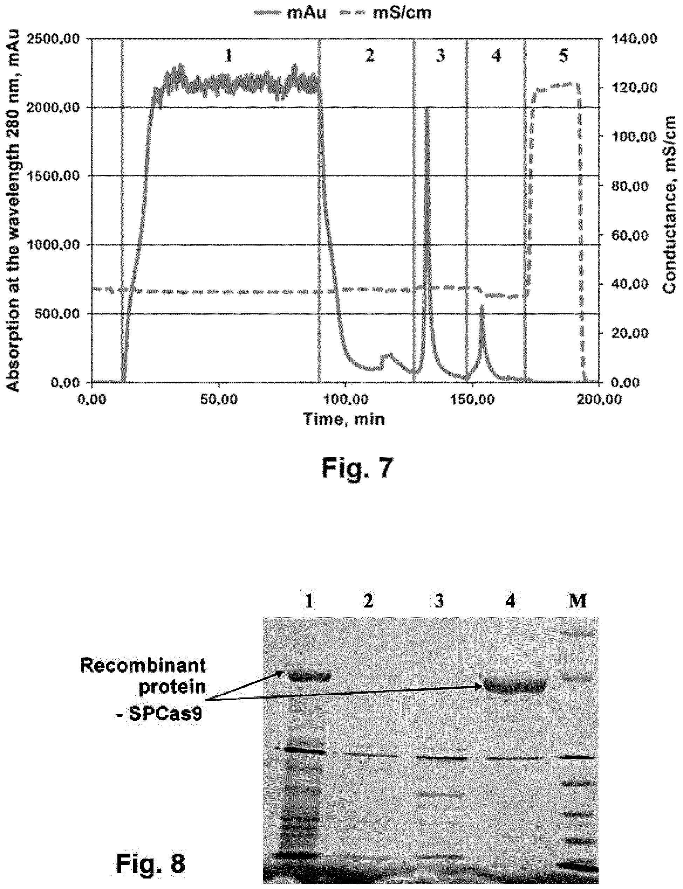

After electrophoresis, the gel was stained with Coomassie R-250 according to the standard Lammley method and scanned (Epson, Japan). Typical electropherograms of chromatographic fractions containing recombinant proteins of the CRISPR/CAS family are shown on examples of electropherograms of chromatographic fractions obtained during protein purification of SPCas9, AsCpfl and STCas9 on the Chelating Sepharose FF resin on the Fig. 8, 14 and 19 respectively.

The target proteins were found in the chromatographic fractions with the 20 mM of Na₂HPO₄/NaH₂PO₄ pH 6,6-8,0, 500 mM of sodium chloride, 250 mM of imidazole and this was proved by the presence of the visible bands with molecular weight of 161836,42 Da (with the calculated molecular weight of the SPCas9) in the lane 4 on the fig. 8, 155028,12 Da (the calculated molecular weight of the AsCpfl) in the lane 4 on fig. 14, and also 135860,17 Da (the calculated molecular weight of the STCas9) in the lane 4 on fig. 19 in stained polyacrylamide gels, when matched with a marker of molecular masses (from top to bottom 200, 150, 100, 85, 60, 50, 40, 30, 25, 20, 15, 10 kDa, the kit of protein markers (10-200 kDa) M35, Sibenzyme, Russia).

At the 2 stage of the chromatographic purification, the recombinant protein of the CRISPR/CAS family in the buffer containing 20 mM of Na₂HPO₄/NaH₂PO₄ pH 6,6-8,0, 500 mM of sodium chloride, 250 mM of imidazole was subjected to the ion exchange chromatography performed on a SP Sepharose FF chromatography resin (GE Healthcare, Great Britain).

To prepare a chromatographic column with the ion exchange chromatography resin SP Sepharose FF, 10 ml of the ion exchange resin SP Sepharose FF was placed into the XK 16/20 Column (GE Healthcare, UK) and washed with 20 column volumes of deionized water.

Before starting the process of chromatographic purification of the recombinant protein of the CRISPR/CAS family from eluate obtained during the chromatographic purification on the Chelating Sepharose FF resin, the column was equilibrated with 5 column volumes of buffer containing 20 mM Tris-HCl pH 8.2. Eluate obtained during the metal-affinity chromatographic purification step was diluted with buffer nor less than 20 times with mentioned buffer containing 20 mM Tris-HCl pH 8,2 up to the final conductivity of the solution 3-5 mS/cm and then applied on the cation exchange chromatography resin SP Sepharose FF with the flow rate of 10 ml/min.

After loading, all proteins not binding with the SP Sepharose FF resin were removed by washing the column with 5-10 column volumes of buffer containing 20 mM Tris-HCl ph 8.2. Removal of protein impurities bounded to the SP Sepharose FF resin was carried out by washing the column with 5-10 column volumes of buffer containing 20 mM Tris-HCl pH 8.2, 300 mM of sodium chloride.

Elution of the recombinant protein of the CRISPR/CAS family was carried out with the buffer containing 20 mM Tris-HCl pH 8,2, 600 mM - 1 M of sodium chloride.

After the target protein elution, the chromatographic column containing the SP Sepharose FF resin was washed with the buffer containing 20 mM Tris-HCl pH 8,2, 1-2 M of sodium chloride. The washed column was preserved in a 20% ethanol solution containing 200 mM sodium acetate and stored at +4 ° C.

Typical chromatographic purification profiles of recombinant proteins of the CRISPR/CAS family on the SP Sepharose FF resin are shown on the examples of protein SPCas9, AsCpfl and STCas9 purification profiles on Fig. 9, 15 and 18 respectively.

Chromatographic fractions containing recombinant proteins of the CRISPR/CAS family obtained during chromatographic purification on the SP Sepharose FF resin were analysed using electrophoresis in 7.5% polyacrylamide gel.

After electrophoresis, the gel was stained with Coomassie R-250 according to the standard Lammley method, and scanned (Epson, Japan).

Typical electropherograms of chromatographic fractions containing recombinant proteins of the CRISPR/CAS family are shown on the examples of electropherograms of chromatographic fractions obtained during protein SPCas9, AsCpfl and STCas9 purification on the SP Sepharose FF resin on Fig. 10, 16 and 19 respectively.

The target proteins were found in the chromatographic fractions containing 20 mM Tris-HCl pH 8,2, 600 mM - 1 M of sodium chloride and this was proved by presence of visible bands with molecular weight of 161836,42 Da (the calculated molecular weight of SPCas9) in the lane 4 on Fig. 10, 155028,12 Da (the calculated molecular weight of AsCpfl) in the lane 3 on Fig. 16, and also 135860,17 Da (the calculated molecular weight of STCas9) in the lane 7 on Fig. 19 in stained polyacrylamide gels, when matched with a marker of molecular masses (from top to bottom 200, 150, 100, 85, 60, 50, 40, 30, 25, 20, 15, 10 kDa, the Kit of protein markers (10-200 kDa) M35, Sibenzyme, Russia).

When the CRISPR/CAS family protein expression is conducted at 27°C for 20-24 hours the 1 stage chromatographic purification takes place without any deviations from the protocol of the steps of sample loading, washing of unbound proteins, elution with a solution, containing 50 mM of imidazole, elution with a solution, containing 250 mM of imidazole and washing with a solution containing 1 M of sodium chloride and this can be seen of Fig. 11.

But at the 2 stage of purification on the SP Sepharose FF resin visible protein aggregation (opalescence of the solution) occurs when eluate from the 1 stage was transferred to the binding buffer containing 20 mM Tris-HCl pH 8,2. As a result of aggregation, the recombinant protein of the CRISPR/CAS family expressed at 27°C for 20-24 hours, loses its ability to be sorbed on the SP Sepharose FF resin as can be seen on Fig. 12, when at the loading stage (indicated by the number 1 in Fig. 12) there is an increase in absorption at 280 nm wavelength (mAu), and on the elution stages (indicated by the numbers 3-13 in Fig. 12) there is no such growth.

### EXAMPLE 4. REMOVAL OF BACTERIAL ENDOTOXINS FROM SOLUTIONS OF THE RECOMBINANT PROTEINS OF CRISPR/CAS FAMILY.

Various methods are recommended for removing bacterial endotoxins from the recombinant protein solutions. To remove bacterial endotoxins from the recombinant protein solutions of the CRISPR/CAS family different approaches were used such as changes in the ionic strength of the solution (increasing the content of sodium chloride in the solution), changes in pH at various stages of the chromatographic purification, using a specialized affinity chromatography resin and using Triton X-114 detergent.

Proteins of the CRISPR/CAS family have sufficiently high values of the isoelectric point (pI > 9) so in the solutions with physiological pH = 6.5-8.0, they have a high positive total charge. This leads to strong binding of negatively charged bacterial endotoxins of destroyed bacterial cell walls to the proteins of the CRISPR/CAS family isolated from E. coli cells.

The high ionic strength of the solution is known to be able to prevent the strong binding of bacterial endotoxins to the protein surface. Therefore, one of the options for removing bacterial endotoxins involves the disintegration of bacterial cells of the producing strain in the presence of sodium chloride in the final concentration of 1 M. No changes were made into the chromatographic purification protocol, except that in the first stage of purification (on the Chelating sepharose resin) all buffer solutions contain sodium chloride at a final concentration of 1 M. It was shown that this modified protocol does not allow obtaining a purified protein due to the loss of material during the chromatographic purification, as can be seen by the decrease in the peak (absorption at 280 nm) when the protein was eluted with a solution containing 250 mM of imidazole (Figure 20, the elution stage with a solution containing 250 mM of imidazole, indicated by the number 4).

Proteins of the CRISPR/CAS family have sufficiently high values of the isoelectric point (pl > 9) so in solutions with pH > pl (for example, pH ≥ 9.5) they must have a total negative charge. This total negative charge should prevent binding of the proteins of the CRISPR/CAS family to negatively charged bacterial endotoxins.

This approach has also been implemented to remove bacterial endotoxins from solutions of the recombinant CRISPR/CAS family proteins. For that aim the disintegration of the bacterial cells of the producing strain was carried out at pH = 9.5. No changes were made into the chromatographic purification protocol, except that at 1 stage of purification (on the Chelating sepharose resin) all buffer solutions had a pH = 9.5. It was shown that this modified protocol does not allow obtaining a purified protein due to the loss of material during the chromatographic purification, as can be seen by the decrease in peaks (absorption at 280 nm) during protein elution with a solution containing 600 mM of sodium chloride (figure 21, the elution stage with a solution containing 600 mM of sodium chloride, indicated by the number 3).

The chromatographic purification on the cation-exchange chromatography resin SP Sepharose at low pH < 5 is known to be able to contribute to the removal of bacterial endotoxins from recombinant protein solutions. This approach was implemented to remove bacterial endotoxins from the solutions of the recombinant CRISPR/CAS family proteins. No changes were made into the protocol of cell disintegration of the producing strain. No changes were made into the chromatographic purification protocol, except that at the 2 stage of purification (on the SP Sepharose resin) all buffer solutions had pH = 4. It was shown that this modified protocol does not allow obtaining a purified protein due to the loss of material during the chromatographic purification, as can be seen from the reduced peaks (absorption at 280 nm) during protein elution with a solution containing 600 mM of sodium chloride (Figure 22, the elution stage with a solution containing 600 mM of sodium chloride, indicated by the number 4).

An attempt was made to remove bacterial endotoxins from solutions of recombinant proteins of the CRISPR/CAS family using a specialized affinity chromatography resin Polymyxin B-agarose (Sigma-Aldrich, USA). For this aim a 100 µl of Polymyxin B-agarose was placed into the recombinant CRISPR/CAS protein solution and incubated for 1 hour under constant stirring at room temperature, after what the Polymyxin B-agarose resin was removed by centrifugation. This final purification method resulted in complete precipitation of the recombinant CRISPR / CAS SpCas9 protein on the surface of the Polymyxin B-agarose affinity resin. Thus, this method was not suitable for removing bacterial endotoxins because does not allow obtaining a purified protein due to the loss of material due to its precipitation on the surface of the affinity chromatography resin Polymyxin B-agarose.

The removal of bacterial endotoxins from solutions of recombinant CRISPR/CAS family proteins was carried out using Triton X-114 detergent. Triton X-114 has the unique attribute of a low turbidity point (23°C) allowing it to be used in a number of biochemical applications that require solubilisation and separation of proteins.

To remove the bacterial endotoxins from the solutions of the CRISPR/CAS family recombinant proteins by the Triton X-114 detergent, Triton X-114 detergent was added to the protein solution in the buffer containing 20 mM Tris-HCl pH 8,2, 0,6-1 M of sodium chloride, 2 mM DTT and 0,2 mM EDTA to final concentration of 1%. Solution was mixed thoroughly on the vortex until visible turbidity. After that, the solution was placed on ice bath for 15 minutes until becoming completely transparent. Then the solution was placed in a thermostat at 37 ° C for 15 minutes until becoming completely opaque (indicative that the solution was enriched with micelles containing bacterial endotoxins). At the end of incubation, the separation of the aqueous and micellar fractions was carried out by the centrifugation at 10,000 g for 10 minutes at 23°C. The upper water phase was taken into a clean test tube. The purification procedure was repeated 6-9 times to remove bacterial endotoxins to a level allowing using protein preparation in vivo.

The content of bacterial endotoxins in protein solutions was determined by using a semiquantitative LAL reagent gel clot test with the sensitivity of 0.03 EU / ml (LAL Center, Russia). This method is recommended by the Ministry of Health of the Russian Federation and is described in the OFS.1.2.4.0006.15 Bacterial endotoxins of the General Pharmacopoeia Article. The threshold pyrogenic dose is considered to be a dose equal to 5 EU / kg per 1 hour for the test drug preparation, if it administered to the patient by any parenteral route other than intrathecal.

When applied, the developed protocol for removal of the bacterial endotoxins from solutions of recombinant proteins, we obtained the CRISPR/CAS family proteins containing 0.3-1.5 EU / ml (Table 3). For comparison the commercially available protein EnGen^{™} Cas9 NLS, *S. pyogenes* (M0646, NEB, USA) was used. Information about EnGen^{™} Cas9 NLS is available on the following site - "https://international.neb.com" or from the publications sources (see, for example, Philippe E. Mangeot, Valérie Risson, Floriane Fusil, Aline Marnef, Emilie Laurent, Juliana Blin, Virginie Mournetas, Emmanuelle Massouridès, Thibault J. M. Sohier, Antoine Corbin, Fabien Aubé, Marie Teixeira, Christian Pinset, Laurent Schaeffer, Gaëlle Legube, François-Loïc Cosset, Els Verhoeyen, Théophile Ohlmann and Emiliano P. Ricci, Genome editing in primary cells and in vivo using viral-derived Nanoblades loaded with Cas9-sgRNA ribonucleoproteins, Nat Commun., 2019; v.10, p:45). The content of the bacterial endotoxins in the preparation of the commercially available protein EnGen^{™} Cas9 NLS, *S. pyogenes* (M0646, NEB, USA) was 3-30 EU / ml, being 10-20 times higher than the content of bacterial endotoxins in solutions of the recombinant proteins of the CRISPR/CAS family obtained by the method described in the present invention.

As a result of the chromatographic purification and removal of bacterial endotoxins, preparations of recombinant proteins of the CRISPR/CAS family of the following composition were obtained:
1. recombinant nuclease Cas9, *Streptococcus pyogenes* (SPCas9), in buffer solution, containing 20 mM Tris-HCl pH 8,2, 600 mM NaCl, 2 mM DTT, 0,2 mM EDTA, 0,3-1,5 EU/ml;
2. recombinant nickase Cas9, *Streptococcus pyogenes* (SPCas9D10A), in buffer solution, containing 20 mM Tris-HCl pH 8,2, 600 mM NaCl, 2 mM DTT, 0,2 mM EDTA, 0,3-1,5 EU/ml;
3. recombinant nuclease Cas9, *Streptococcus thermophilus* (STCas9), in buffer solution, containing 20 mM Tris-HCl pH 8,2, 1000 mM NaCl, 2 mM DTT, 0,2 mM EDTA, 0,3-1,5 EU/ml;
4. recombinant nuclease AsCpfl, *Acidaminococcus* (AsCpfl), in buffer solution, containing 20 mM Tris-HCl pH 8,2, 600 mM NaCl, 2 mM DTT, 0,2 mM EDTA, 0,3-1,5 EU/ml;
5. recombinant nuclease Cas9, *Streptococcus pyogenes* (TATSPCas9), fused protein with TAT-peptide on N-end in buffer solution, containing 20 mM Tris-HCl pH 8,2, 1000 mM NaCl, 2 mM DTT, 0,2 mM EDTA, 0,3-1,5 EU/ml;
6. recombinant nuclease Cas9, *Streptococcus pyogenes* (SPCas9TAT), fused protein with TAT-peptide on C-end in buffer solution, containing 20 mM Tris-HCl pH 8,2, 1000 mM NaCl, 2 mM DTT, 0,2 mM EDTA, 0,3-1,5 EU/ml;
7. recombinant nuclease Cas9, *Streptococcus pyogenes* (TATSPCas9TAT), fused protein with TAT-peptide on N- and C-ends in buffer solution, containing 20 mM Tris-HCl pH 8,2, 1000 mM NaCl, 2 mM DTT, 0,2 mM EDTA, 0,3-1,5 EU/ml;
8. recombinant nickase Cas9, *Streptococcus pyogenes* (SPCas9D10ATAT), fused protein with TAT-peptide on C-end in buffer solution, containing 20 mM Tris-HCl pH 8,2, 1000 mM NaCl, 2 mM DTT, 0,2 mM EDTA, 0,3-1,5 EU/ml.

Also, lyophilization of the obtained preparations of recombinant proteins of the CRISPR/CAS family was performed (see, for example, the methods of lyophilization described in the article by Blynskaya E. V., Tishkov S. V. and Alekseev K. V., Technological approaches to improving the lyophilization process of protein and peptide drug preparations, Russian Biotherapeutic Journal 2017, v.16, n.1, p.6-11 or in the article by Horn J, Schanda J and Friess W, Impact of fast and conservative freeze-drying on product quality of protein-mannitol-sucrose-glycerol lyophilizates, Eur J Pharm Biopharm., 2018, v.127, p:342-354). In this case for the preparations of the recombinant proteins of the CRISPR/CAS family located in buffer solution containing 20 mM Tris-HCl pH 8,2, 600-1000 mM NaCl, 2 mM DTT, 0,2 mM EDTA, 0,3-1,5 EU/ml, the auxiliary substances were added for the lyophilization. For example, glycerol at a concentration of 10-50% was added as a tonicity modifier or trehalose at a concentration of 50-500 mM was added as a cryo-/lyoprotector and samples were lyophilized using freeze-drying machine (Labconco, Usa).

The preparations obtained this way were highly stable in prepared solutions containing excipients for lyophilisation and this can be seen during electrophoresis in 7.5% polyacrylamide gel and proved by the presence of visible bands with the molecular weight of 161836,42 Da (the calculated molecular weight of SPCas9) in lanes 1-11 on Fig. 23 in stained polyacrylamide gels when matched with a marker of molecular weights (from top to bottom 116, 66,2, 45, 35, 25, 18,4, 14,4 kDa, Unstained Protein Molecular Weight Marker, #26610, Thermo Fisher Scientific, USA).

Freeze-dried preparations of the recombinant CRISPR/CAS family proteins containing lyophilization auxiliaries such as glycerol and trehalose retain their specific activity for a long period of time (more than a year when being stored from 4 to 20° C).

### EXAMPLE 5. TESTING THE BIOLOGICAL ACTIVITY OF THE OBTAINED RECOMBINANT CRISPR/CAS FAMILY PROTEINS

When the developed protocols for the chromatographic purification and removal of the bacterial endotoxins were used, the solutions of recombinant proteins of the CRISPR/CAS family did not lose their specific activity. The specific activity of the CRISPR/CAS family proteins is manifested in the ability to introduce double-and single-stranded breaks into DNA matrices containing sequences recognized by CAS proteins. Recognition of the "target" occurs due to the presence of guide RNA (gRNA).

The standard protocol for testing the specific activity of CRISPR/CAS family proteins consists of several stages:
1. Preparation of DNA-matrix to test the specific activity of the CRISPR/CAS family proteins.
2. Preparation of the guide RNA.
3. Creation of a ribonucleoprotein complex containing the CRISPR/CAS family protein and a guide RNA.
4. Testing of a specific activity of the CRISPR/CAS family proteins and visualization of experimental results.

A standard protocol for testing the activity of CRISPR/CAS family proteins with some modifications was used (Anders C and Jinek M., In vitro enzymology of Cas9, Methods Enzymol., 2014, v.546, p:1-20).

The 1 stage involves obtaining a DNA matrix designed to test the specific activity of proteins of the CRISPR/CAS family. The DNA matrix is prepared in accordance with the target selected for editing. As a rule, the DNA matrix is a product of amplification of a gene site by polymerase chain reaction containing a sequence for recognition to which the CAS protein is "programmed" with the help of a guide RNA. Hydrolysis of the DNA matrix should lead to the formation of DNA fragments distinguishable from each other in length.

The pGEM-T-CCR5 plasmid containing a fragment of the gene encoding the human chemokine receptor CCR5 was used to prepare the DNA matrix. The DNA matrix was synthesized by PCR using primers M13for and M13rev (GenTerra, Russia). The length of the DNA matrix is 2000 nucleotide pairs; the length of fragments formed as a result of specific hydrolysis by CRISPR/CAS family proteins is 1200 and 800 nucleotide pairs.

Guide RNA preparation was performed by in vitro transcription using commercially available reagent kits (HiScribe^{™} T7 High Yield RNA Synthesis Kit, NEB, USA). Modifications of the manufacturer's protocol introduced by the authors consist of a method of purification of the transcribed guide RNA obtained in vitro allowing increasing the yield of the reaction product and obtaining the desired concentration of the final preparation of the guide RNA.

To prepare the guide RNA, a DNA fragment encoding the guide RNA was synthesized by PCR using primers T7 forward and M13rev (GenTerra, Russia) and the length of the fragment is 250 nucleotide pairs. This DNA fragment was used as a matrix in the in vitro transcription reaction. The products of the in vitro transcription reaction were precipitated from the reaction mixture by adding of sodium chloride to a final concentration of 400 mM and an equal volume of isopropyl alcohol.

Assembly of ribonucleoprotein complex containing a protein of the CRISPR/CAS family and a guide RNA was carried out according to a standard protocol with some modifications (Anders C and Jinek M., In vitro enzymology of Cas9, Methods Enzymol., 2014, v.546, p: 1-20).

The guide RNA (in an amount of 500 ng) was heated at 90 ° C for 5 minutes and allowed to cool down slowly to the room temperature (incubation at room temperature for at least 10 minutes) immediately before combining with the CAS protein preparation. Such heating is necessary for the formation of the correct conformation of the hairpin contained in the guide RNA. Many manufacturers of commercial CAS protein preparations skip this step when preparing a ribonucleoprotein complex.

To form the ribonucleoprotein complex 500 ng of CAS protein and the prepared guide RNA were mixed and incubated for 15 minutes at the room temperature. After the incubation time has elapsed a DNA matrix was added to the mixture containing the ribonucleoprotein complex. Incubation of the ribonucleoprotein complex and the target DNA was carried out at a temperature of 37°C for 60 minutes. At the end of the reaction, the unbound RNA was removed from the mixture using a RNase A solution. Hydrolysis products were visualized by electrophoresis in agarose gel (Figure 24).

To test the biological activity of recombinant proteins of the CRISPR/CAS family SPCas9D10A and SPCas9D10ATAT having nickase activity (the ability to introduce single-strand breaks in double-stranded DNA matrices) the described above protocol with minor changes was used. PCR DNA-matrix was replaced with the super-coiled plasmid pGEM-T-CCR5, which turns into a relaxed form as a result of specific hydrolysis and changes its mobility on electrophoresis. Hydrolysis products were visualized by electrophoresis in agarose gel (Figure 24).

Quantitative assessment of the hydrolysis rate was carried out by using a commercially available kit High Sensitivity DNA Assay (Agilent, USA) on the Bioanalyzer 2100 (Agilent, USA). The results of the analysis are shown in Fig. 25; the calculation of the quantitative data of the analysis presented in Table 4.

The results of the experiments can indicate that recombinant proteins of the CRISPR/CAS family obtained using the technology described in the invention do not lose their specific activity and unexpectedly even have increased activity in comparison with the commercially available preparation EnGen Cas9. The level of activity can be placed in the following order SPCas9 > TATSPCas9TAT > EnGen Cas9 > TATSPCas9 > AsCpfl > SPCas9TAT (Tab. 4).

Therefore, the method implementation according to the present invention made possible to achieve a yield of at least 20 milligrams of recombinant protein of the CRISPR/Cas family from 1 litre of culture of the producer strain and to obtain preparation of recombinant nuclease of the Cas family with a reduced content of bacterial endotoxins (0.3-1.5 EU/ml) allowing using of this preparation in vivo.

**Table 1.**

| Protein | Name of plasmid DNA | Expression vector | Source of cloned DNA | 5'-end primer | 3'-end primer | Restriction sites (5'- and 3'-) |
|---|---|---|---|---|---|---|
| Nuclease Cas9 (class 2, type 2, Cas9), *Streptococcus pyogenes* | pAM-SPCas9 | pET22b(NsiI) | Addgene #41815 (the physical map is available on site https://www.addgene .org/41815/) | **1 stage Cas9 (NsiI NLS)** - 5'-ggcagccccaagaagaagaggaaggtgat ggacaagaagtactccattgggc- 3' | **Cas9 (NLS NotI**) **rev** - 5'-ccgtttaaactcattactgcggc cgcagggatcgaacc-3' | NsiI NotI |
| | | | | **2 stage Cas9 (6His)** - 5'-tatatatgcatcatcaccaccaccatcac ggc agccccaagaagaagaggaaggtgatgga caagaagt - 3 ' | | |
| Nickase Cas9 (class 2, type 2, Cas9), *Streptococcus pyogenes* | pAM-SPCa9D 1 0A | pET22b(NsiI) | Addgene #41816 (the physical map is available on site https://www.addgene .org/41816/) | **1 stage Cas9 (NsiI NLS)** - 5'-ggcagccccaagaagaagaggaaggtgat ggacaagaagtactccattgggc-3' | **Cas9 (NLS NotI**) **rev** - 5'-ccgtttaaactcattactgcggc cgcagggatcgaacc-3' | NsiI NotI |
| | | | | **2 stage Cas9 (6His)** - 5'-tatatatgcatcatcaccaccaccatcacggcagccccaagaagaagaggaaggtgatgga caagaagt -3' | | |
| Nuclease Cas9 (class 2, type 2, Cas9), *Streptococcus thermophilus* | pAM-STCas9 | pET22b(NsiI) | Addgene #48669 (the physical map is available on site https://www.addgene .org/48669/) | **1 stage Stl-Cas-forl(NLS)** - 5'-ccaagaagaagaggaaggtgggcgcgga gccaccatgagcgacctg-3' | **St1-Cas** - 5'-tatatatatatagcggccgcact cgagaccggtagggatcgaa cc-3' | NdeI AgeI |
| | | | | **2 stage Stl-Cas-for2(6His)** - 5'-tatatatacatatgctcgagcatcaccaccac catcac ggcagccccaagaagaagaggaa ggtggg-3 ' | | |
| Nuclease Cpfl (class 2, type 5, Cas12a), Acidaminococ cus | pAM -AsCpf1 | pET22b(NsiI) | Addgene #pTE4396 (the physical map is available on site https://www.addgene .org/7 4041/) | **1 stage AsCpf1-for- NLS** - 5'-ggcagccccaagaagaagaggaaggtgat gacacagttcgag;ggc-3' | **AsCpf1-rev (NotI-XhoI)** - 5'-tatataagggctcgagcggcc gcaccttatcactttttcttttttgc ctggc-3' | NsiI NotI |
| | | | | **2 stage AsCpf1-for 6His** - 5'-tatatcatatgcatcatcaccaccaccatcac ggcagccccaagaagaagaggaagg-3' | | |
| Nuclease Cas9 (class 2, type 2, Cas9), *Streptococcus pyogenes,* fused protein with TAT-peptide on N-end | pAM-TATSPCas9 | pET22b(NsiI) | pAM-SPCas9 | **TAT-for** - 5'-tatatatgcatggccgcaaaaaacgccgcca gcgccgccgcccgcagggcagccatcacc accaccatcacgg-3' | **Seq4-Cas9** - 5'-gatttgggaccccagttctt-3 ' | NsiI Notl |
| Nuclease Cas9 (class 2, type 2, Cas9), *Streptococcus pyogenes,* fused protein with TAT-peptide on C-end | pAM -SPCas9TA T | pET22b(NsiI) | pAM-SPCas9 | **Seq5-Cas9-**5' -cagatttcagaaaggactttcagttt-3' | **TAT-Rev** - 5'-atatatgc ggccgcttatcactg cgggcggcggcgctggcggc gttttttgc ggccactgcccacc ttcctcttcttcttggggtcagcc -3' | NsiI Notl |
| Nuclease Cas9 (class 2, type 2, Cas9), *Streptococcus pyogenes,* fused protein with TAT-peptide on N- and C-ends | pAM-TATSPCas9TAT | pET22b(NsiI) | pAM-TATSPCas9 | **Seq5-Cas9-**5'-cagatttcagaaaggactttcagttt-3' | **TAT-Rev** - 5'-atatatgcggccgcttatcactg cgggcggcggcgctggcggc gttttttgcggccactgcccacc ttcctcttcttcttggggtcagcc -3' | NsiI NotI |
| Nickase Cas9 (class 2, type 2, Cas9), *Streptococcus pyogenes,* fused protein with TAT-peptide on C-end | pAM-SPCa9D10ATAT | pET22b(NsiI) | pAM-SPCa9D10A | **Seq5-Cas9-**5' -cagatttcagaaaggactttcagttt - 3 ' | **TAT-Rev** - 5'-atatatgcggcc gcttatcactg cgggcggcggcgctggcggc gttttttgc ggccactgcccacc ttcctcttcttcttggggtcagcc -3' | NsiI NotI |

**Table 2.**

| Item | Protein | Protein name, abbreviation | Amino acid sequence identifier | Nucleotide sequence identifier |
|---|---|---|---|---|
| 1 | Nuclease Cas9 (class 2, type 2, Cas9), *Streptococcus pyogenes* | SPCas9 | SEQ ID NO: 1 | SEQ ID NO: 9 |
| 2 | Nickase Cas9 (class 2, type 2, Cas9), *Streptococcus pyogenes* | SPCas9D 10A | SEQ ID NO: 2 | SEQ ID NO: 10 |
| 3 | Nuclease Cas9 (class 2, type 2, Cas9), *Streptococcus thermophilus* | STCas9 | SEQ ID NO: 3 | SEQ ID NO: 11 |
| 4 | Nuclease Cpfl (class 2, type 5, Cas12a), Acidaminococcus | AsCpfl | SEQ ID NO: 4 | SEQ ID NO: 12 |
| 5 | Nuclease Cas9 (class 2, type 2, Cas9), *Streptococcus pyogenes,* fused protein with TAT-peptide on N-end | TATSPCas9 | SEQ ID NO: 5 | SEQ ID NO: 13 |
| 6 | Nuclease Cas9 (class 2, type 2, Cas9), *Streptococcus pyogenes,* fused protein with TAT-peptide on C-end | SPCas9TAT | SEQ ID NO: 6 | SEQ ID NO: 14 |
| 7 | Nuclease Cas9 (class 2, type 2, Cas9), *Streptococcus pyogenes,* fused protein with TAT-peptide on N- and C-ends | TATSPCas9TAT | SEQ ID NO: 7 | SEQ ID NO: 15 |
| 8 | Nickase Cas9 (class 2, type 2, Cas9), *Streptococcus pyogenes,* fused protein with TAT-peptide on C-end | SPCas9D10ATAT | SEQ ID NO: 8 | SEQ ID NO: 16 |

**Table 3.**

| | **SPCas9** | **STCas9** | **AsCpf1** |
|---|---|---|---|
| Three-fold treatment Triton X-114 | N.t. | ≥ 6 EU/ml | N.t. |
| Six-fold treatment Triton X-114 | ≥ 3 EU/ml | 0,3-1,5 EU/ml | N.t. |
| Nine-fold treatment Triton X-114 | 0,3-1,5 EU/ml | N.t. | 0,3-1,5 EU/ml |

| | | | |
|---|---|---|---|
| N. t. - not tested | | | |

**Table 4.**

| | **EnGen Cas9 (NEB)** | **SPCas9** | **TATSPCas9** | **SPCas9TAT** | **TATSPCas9TAT** | **AsCpf1** |
|---|---|---|---|---|---|---|
| Product of special hydrolysis No 1 | 36,25% | 36,85% | 34,78% | 30,65% | 38,24% | 34,67% |
| Product of special hydrolysis No 2 | 47,5% | 50,00% | 44,93% | 40,32% | 48,53% | 44,00% |
| Non-hydrolysed matrix | 16,25% | 13,15% | 20,29% | 29,03% | 13,23% | 21,33% |

SEQUENCE LISTING
<110> UCHREZHDENIE NAUKI "CENTRALNYI NAUCHNO-ISSLEDOVATELSKIY INSTITUT
EPIDEMIOLOGII" FEDERALNOY SLUZHBY PO NADZORU V SPHERE ZASCHITY PRAV
POTREBITELEI I BLAGOPOLUCHIJA CHELOVEKA
<120> Method for production of recombinant CAS nuclease, substantially free
of bacterial endotoxins, resulting preparation produced by this method and
kit containing it for use in the CRISPR / Cas system
<160> NUMBER OF SEQ ID NOS: 16
<210> 1
<211> 1396
<212> AA (amino acid)
<213> artificial
<400> SEQUENCE 1 (SPCas9):
Met His His His His His His His Gly Ser Pro Lys Lys Lys Arg Lys
1               5                   10                  15

Val Met Asp Lys Lys Tyr Ser Ile Gly Leu Asp Ile Gly Thr Asn Ser
            20                  25                  30

Val Gly Trp Ala Val Ile Thr Asp Glu Tyr Lys Val Pro Ser Lys Lys
        35                  40                  45

Phe Lys Val Leu Gly Asn Thr Asp Arg His Ser Ile Lys Lys Asn Leu
    50                  55                  60

Ile Gly Ala Leu Leu Phe Asp Ser Gly Glu Thr Ala Glu Ala Thr Arg
65                  70                  75                  80

Leu Lys Arg Thr Ala Arg Arg Arg Tyr Thr Arg Arg Lys Asn Arg Ile
                85                  90                  95

Cys Tyr Leu Gln Glu Ile Phe Ser Asn Glu Met Ala Lys Val Asp Asp
            100                 105                 110

Ser Phe Phe His Arg Leu Glu Glu Ser Phe Leu Val Glu Glu Asp Lys
        115                 120                 125

Lys His Glu Arg His Pro Ile Phe Gly Asn Ile Val Asp Glu Val Ala
    130                 135                 140

Tyr His Glu Lys Tyr Pro Thr Ile Tyr His Leu Arg Lys Lys Leu Val
145                 150                 155                 160

Asp Ser Thr Asp Lys Ala Asp Leu Arg Leu Ile Tyr Leu Ala Leu Ala
                165                 170                 175


His Met Ile Lys Phe Arg Gly His Phe Leu Ile Glu Gly Asp Leu Asn
            180                 185                 190

Pro Asp Asn Ser Asp Val Asp Lys Leu Phe Ile Gln Leu Val Gln Thr
        195                 200                 205

Tyr Asn Gln Leu Phe Glu Glu Asn Pro Ile Asn Ala Ser Gly Val Asp
    210                 215                 220

Ala Lys Ala Ile Leu Ser Ala Arg Leu Ser Lys Ser Arg Arg Leu Glu
225                 230                 235                 240

Asn Leu Ile Ala Gln Leu Pro Gly Glu Lys Lys Asn Gly Leu Phe Gly
                245                 250                 255

Asn Leu Ile Ala Leu Ser Leu Gly Leu Thr Pro Asn Phe Lys Ser Asn

            260                 265                 270

Phe Asp Leu Ala Glu Asp Ala Lys Leu Gln Leu Ser Lys Asp Thr Tyr
        275                 280                 285

Asp Asp Asp Leu Asp Asn Leu Leu Ala Gln Ile Gly Asp Gln Tyr Ala
    290                 295                 300

Asp Leu Phe Leu Ala Ala Lys Asn Leu Ser Asp Ala Ile Leu Leu Ser
305                 310                 315                 320

Asp Ile Leu Arg Val Asn Thr Glu Ile Thr Lys Ala Pro Leu Ser Ala
                325                 330                 335

Ser Met Ile Lys Arg Tyr Asp Glu His His Gln Asp Leu Thr Leu Leu
            340                 345                 350

Lys Ala Leu Val Arg Gln Gln Leu Pro Glu Lys Tyr Lys Glu Ile Phe
        355                 360                 365

Phe Asp Gln Ser Lys Asn Gly Tyr Ala Gly Tyr Ile Asp Gly Gly Ala
    370                 375                 380

Ser Gln Glu Glu Phe Tyr Lys Phe Ile Lys Pro Ile Leu Glu Lys Met
385                 390                 395                 400

Asp Gly Thr Glu Glu Leu Leu Val Lys Leu Asn Arg Glu Asp Leu Leu
                405                 410                 415

Arg Lys Gln Arg Thr Phe Asp Asn Gly Ser Ile Pro His Gln Ile His
            420                 425                 430

Leu Gly Glu Leu His Ala Ile Leu Arg Arg Gln Glu Asp Phe Tyr Pro
        435                 440                 445

Phe Leu Lys Asp Asn Arg Glu Lys Ile Glu Lys Ile Leu Thr Phe Arg
    450                 455                 460

Ile Pro Tyr Tyr Val Gly Pro Leu Ala Arg Gly Asn Ser Arg Phe Ala
465                 470                 475                 480

Trp Met Thr Arg Lys Ser Glu Glu Thr Ile Thr Pro Trp Asn Phe Glu
                485                 490                 495

Glu Val Val Asp Lys Gly Ala Ser Ala Gln Ser Phe Ile Glu Arg Met
            500                 505                 510

Thr Asn Phe Asp Lys Asn Leu Pro Asn Glu Lys Val Leu Pro Lys His
        515                 520                 525

Ser Leu Leu Tyr Glu Tyr Phe Thr Val Tyr Asn Glu Leu Thr Lys Val
    530                 535                 540

Lys Tyr Val Thr Glu Gly Met Arg Lys Pro Ala Phe Leu Ser Gly Glu
545                 550                 555                 560

Gln Lys Lys Ala Ile Val Asp Leu Leu Phe Lys Thr Asn Arg Lys Val
                565                 570                 575

Thr Val Lys Gln Leu Lys Glu Asp Tyr Phe Lys Lys Ile Glu Cys Phe
            580                 585                 590

Asp Ser Val Glu Ile Ser Gly Val Glu Asp Arg Phe Asn Ala Ser Leu

595                 600                 605

Gly Thr Tyr His Asp Leu Leu Lys Ile Ile Lys Asp Lys Asp Phe Leu
    610                 615                 620

Asp Asn Glu Glu Asn Glu Asp Ile Leu Glu Asp Ile Val Leu Thr Leu
625                 630                 635                 640

Thr Leu Phe Glu Asp Arg Glu Met Ile Glu Glu Arg Leu Lys Thr Tyr
                645                 650                 655

Ala His Leu Phe Asp Asp Lys Val Met Lys Gln Leu Lys Arg Arg Arg
            660                 665                 670

Tyr Thr Gly Trp Gly Arg Leu Ser Arg Lys Leu Ile Asn Gly Ile Arg
        675                 680                 685

Asp Lys Gln Ser Gly Lys Thr Ile Leu Asp Phe Leu Lys Ser Asp Gly
    690                 695                 700

Phe Ala Asn Arg Asn Phe Met Gln Leu Ile His Asp Asp Ser Leu Thr
705                 710                 715                 720

Phe Lys Glu Asp Ile Gln Lys Ala Gln Val Ser Gly Gln Gly Asp Ser
                725                 730                 735

Leu His Glu His Ile Ala Asn Leu Ala Gly Ser Pro Ala Ile Lys Lys
            740                 745                 750

Gly Ile Leu Gln Thr Val Lys Val Val Asp Glu Leu Val Lys Val Met
        755                 760                 765

Gly Arg His Lys Pro Glu Asn Ile Val Ile Glu Met Ala Arg Glu Asn
    770                 775                 780

Gln Thr Thr Gln Lys Gly Gln Lys Asn Ser Arg Glu Arg Met Lys Arg
785                 790                 795                 800

Ile Glu Glu Gly Ile Lys Glu Leu Gly Ser Gln Ile Leu Lys Glu His
                805                 810                 815

Pro Val Glu Asn Thr Gln Leu Gln Asn Glu Lys Leu Tyr Leu Tyr Tyr
            820                 825                 830

Leu Gln Asn Gly Arg Asp Met Tyr Val Asp Gln Glu Leu Asp Ile Asn
        835                 840                 845

Arg Leu Ser Asp Tyr Asp Val Asp His Ile Val Pro Gln Ser Phe Leu
    850                 855                 860

Lys Asp Asp Ser Ile Asp Asn Lys Val Leu Thr Arg Ser Asp Lys Asn
865                 870                 875                 880

Arg Gly Lys Ser Asp Asn Val Pro Ser Glu Glu Val Val Lys Lys Met
                885                 890                 895

Lys Asn Tyr Trp Arg Gln Leu Leu Asn Ala Lys Leu Ile Thr Gln Arg
            900                 905                 910

Lys Phe Asp Asn Leu Thr Lys Ala Glu Arg Gly Gly Leu Ser Glu Leu
        915                 920                 925

Asp Lys Ala Gly Phe Ile Lys Arg Gln Leu Val Glu Thr Arg Gln Ile

930                 935                 940

Thr Lys His Val Ala Gln Ile Leu Asp Ser Arg Met Asn Thr Lys Tyr
945                 950                 955                 960

Asp Glu Asn Asp Lys Leu Ile Arg Glu Val Lys Val Ile Thr Leu Lys
                965                 970                 975

Ser Lys Leu Val Ser Asp Phe Arg Lys Asp Phe Gln Phe Tyr Lys Val
            980                 985                 990

Arg Glu Ile Asn Asn Tyr His His Ala His Asp Ala Tyr Leu Asn Ala
        995                 1000                1005

Val Val Gly Thr Ala Leu Ile Lys Lys Tyr Pro Lys Leu Glu Ser Glu
    1010                1015                1020

Phe Val Tyr Gly Asp Tyr Lys Val Tyr Asp Val Arg Lys Met Ile Ala
1025                1030                1035                1040

Lys Ser Glu Gln Glu Ile Gly Lys Ala Thr Ala Lys Tyr Phe Phe Tyr
                1045                1050                1055

Ser Asn Ile Met Asn Phe Phe Lys Thr Glu Ile Thr Leu Ala Asn Gly
            1060                1065                1070

Glu Ile Arg Lys Arg Pro Leu Ile Glu Thr Asn Gly Glu Thr Gly Glu
        1075                1080                1085

Ile Val Trp Asp Lys Gly Arg Asp Phe Ala Thr Val Arg Lys Val Leu
    1090                1095                1100

Ser Met Pro Gln Val Asn Ile Val Lys Lys Thr Glu Val Gln Thr Gly
1105                1110                1115                1120

Gly Phe Ser Lys Glu Ser Ile Leu Pro Lys Arg Asn Ser Asp Lys Leu
                1125                1130                1135

Ile Ala Arg Lys Lys Asp Trp Asp Pro Lys Lys Tyr Gly Gly Phe Asp
            1140                1145                1150

Ser Pro Thr Val Ala Tyr Ser Val Leu Val Val Ala Lys Val Glu Lys
        1155                1160                1165

Gly Lys Ser Lys Lys Leu Lys Ser Val Lys Glu Leu Leu Gly Ile Thr
    1170                1175                1180

Ile Met Glu Arg Ser Ser Phe Glu Lys Asn Pro Ile Asp Phe Leu Glu
1185                1190                1195                1200

Ala Lys Gly Tyr Lys Glu Val Lys Lys Asp Leu Ile Ile Lys Leu Pro
                1205                1210                1215

Lys Tyr Ser Leu Phe Glu Leu Glu Asn Gly Arg Lys Arg Met Leu Ala
            1220                1225                1230

Ser Ala Gly Glu Leu Gln Lys Gly Asn Glu Leu Ala Leu Pro Ser Lys
        1235                1240                1245

Tyr Val Asn Phe Leu Tyr Leu Ala Ser His Tyr Glu Lys Leu Lys Gly
    1250                1255                1260

Ser Pro Glu Asp Asn Glu Gln Lys Gln Leu Phe Val Glu Gln His Lys

1265                1270                1275                1280

His Tyr Leu Asp Glu Ile Ile Glu Gln Ile Ser Glu Phe Ser Lys Arg
                1285                1290                1295

Val Ile Leu Ala Asp Ala Asn Leu Asp Lys Val Leu Ser Ala Tyr Asn
            1300                1305                1310

Lys His Arg Asp Lys Pro Ile Arg Glu Gln Ala Glu Asn Ile Ile His
        1315                1320                1325

Leu Phe Thr Leu Thr Asn Leu Gly Ala Pro Ala Ala Phe Lys Tyr Phe
    1330                1335                1340

Asp Thr Thr Ile Asp Arg Lys Arg Tyr Thr Ser Thr Lys Glu Val Leu
1345                1350                1355                1360

Asp Ala Thr Leu Ile His Gln Ser Ile Thr Gly Leu Tyr Glu Thr Arg
                1365                1370                1375

Ile Asp Leu Ser Gln Leu Gly Gly Asp Ser Arg Ala Asp Pro Lys Lys
            1380                1385                1390

Lys Arg Lys Val
        1395
<210> 2
<211> 1396
<212> AA (amino acid)
<213> artificial
<400> SEQUENCE 2 (SPCas9D10A):
Met His His His His His His His Gly Ser Pro Lys Lys Lys Arg Lys
1               5                   10                  15

Val Met Asp Lys Lys Tyr Ser Ile Gly Leu Ala Ile Gly Thr Asn Ser
            20                  25                  30

Val Gly Trp Ala Val Ile Thr Asp Glu Tyr Lys Val Pro Ser Lys Lys
        35                  40                  45
Phe Lys Val Leu Gly Asn Thr Asp Arg His Ser Ile Lys Lys Asn Leu
    50                  55                  60

Ile Gly Ala Leu Leu Phe Asp Ser Gly Glu Thr Ala Glu Ala Thr Arg
65                  70                  75                  80

Leu Lys Arg Thr Ala Arg Arg Arg Tyr Thr Arg Arg Lys Asn Arg Ile
                85                  90                  95

Cys Tyr Leu Gln Glu Ile Phe Ser Asn Glu Met Ala Lys Val Asp Asp
            100                 105                 110

Ser Phe Phe His Arg Leu Glu Glu Ser Phe Leu Val Glu Glu Asp Lys
        115                 120                 125

Lys His Glu Arg His Pro Ile Phe Gly Asn Ile Val Asp Glu Val Ala
    130                 135                 140

Tyr His Glu Lys Tyr Pro Thr Ile Tyr His Leu Arg Lys Lys Leu Val
145                 150                 155                 160

Asp Ser Thr Asp Lys Ala Asp Leu Arg Leu Ile Tyr Leu Ala Leu Ala
                165                 170                 175

His Met Ile Lys Phe Arg Gly His Phe Leu Ile Glu Gly Asp Leu Asn

            180                 185                 190

Pro Asp Asn Ser Asp Val Asp Lys Leu Phe Ile Gln Leu Val Gln Thr
        195                 200                 205

Tyr Asn Gln Leu Phe Glu Glu Asn Pro Ile Asn Ala Ser Gly Val Asp
    210                 215                 220

Ala Lys Ala Ile Leu Ser Ala Arg Leu Ser Lys Ser Arg Arg Leu Glu
225                 230                 235                 240

Asn Leu Ile Ala Gln Leu Pro Gly Glu Lys Lys Asn Gly Leu Phe Gly
                245                 250                 255

Asn Leu Ile Ala Leu Ser Leu Gly Leu Thr Pro Asn Phe Lys Ser Asn
            260                 265                 270

Phe Asp Leu Ala Glu Asp Ala Lys Leu Gln Leu Ser Lys Asp Thr Tyr
        275                 280                 285

Asp Asp Asp Leu Asp Asn Leu Leu Ala Gln Ile Gly Asp Gln Tyr Ala
    290                 295                 300

Asp Leu Phe Leu Ala Ala Lys Asn Leu Ser Asp Ala Ile Leu Leu Ser
305                 310                 315                 320

Asp Ile Leu Arg Val Asn Thr Glu Ile Thr Lys Ala Pro Leu Ser Ala
                325                 330                 335

Ser Met Ile Lys Arg Tyr Asp Glu His His Gln Asp Leu Thr Leu Leu
            340                 345                 350

Lys Ala Leu Val Arg Gln Gln Leu Pro Glu Lys Tyr Lys Glu Ile Phe
        355                 360                 365

Phe Asp Gln Ser Lys Asn Gly Tyr Ala Gly Tyr Ile Asp Gly Gly Ala
    370                 375                 380

Ser Gln Glu Glu Phe Tyr Lys Phe Ile Lys Pro Ile Leu Glu Lys Met
385                 390                 395                 400

Asp Gly Thr Glu Glu Leu Leu Val Lys Leu Asn Arg Glu Asp Leu Leu
                405                 410                 415

Arg Lys Gln Arg Thr Phe Asp Asn Gly Ser Ile Pro His Gln Ile His
            420                 425                 430

Leu Gly Glu Leu His Ala Ile Leu Arg Arg Gln Glu Asp Phe Tyr Pro
        435                 440                 445

Phe Leu Lys Asp Asn Arg Glu Lys Ile Glu Lys Ile Leu Thr Phe Arg
    450                 455                 460

Ile Pro Tyr Tyr Val Gly Pro Leu Ala Arg Gly Asn Ser Arg Phe Ala
465                 470                 475                 480

Trp Met Thr Arg Lys Ser Glu Glu Thr Ile Thr Pro Trp Asn Phe Glu
                485                 490                 495

Glu Val Val Asp Lys Gly Ala Ser Ala Gln Ser Phe Ile Glu Arg Met
            500                 505                 510

Thr Asn Phe Asp Lys Asn Leu Pro Asn Glu Lys Val Leu Pro Lys His

515                 520                 525

Ser Leu Leu Tyr Glu Tyr Phe Thr Val Tyr Asn Glu Leu Thr Lys Val
    530                 535                 540

Lys Tyr Val Thr Glu Gly Met Arg Lys Pro Ala Phe Leu Ser Gly Glu
545                 550                 555                 560

Gln Lys Lys Ala Ile Val Asp Leu Leu Phe Lys Thr Asn Arg Lys Val
                565                 570                 575

Thr Val Lys Gln Leu Lys Glu Asp Tyr Phe Lys Lys Ile Glu Cys Phe
            580                 585                 590

Asp Ser Val Glu Ile Ser Gly Val Glu Asp Arg Phe Asn Ala Ser Leu
        595                 600                 605

Gly Thr Tyr His Asp Leu Leu Lys Ile Ile Lys Asp Lys Asp Phe Leu
    610                 615                 620

Asp Asn Glu Glu Asn Glu Asp Ile Leu Glu Asp Ile Val Leu Thr Leu
625                 630                 635                 640

Thr Leu Phe Glu Asp Arg Glu Met Ile Glu Glu Arg Leu Lys Thr Tyr
                645                 650                 655

Ala His Leu Phe Asp Asp Lys Val Met Lys Gln Leu Lys Arg Arg Arg
            660                 665                 670

Tyr Thr Gly Trp Gly Arg Leu Ser Arg Lys Leu Ile Asn Gly Ile Arg
        675                 680                 685

Asp Lys Gln Ser Gly Lys Thr Ile Leu Asp Phe Leu Lys Ser Asp Gly
    690                 695                 700

Phe Ala Asn Arg Asn Phe Met Gln Leu Ile His Asp Asp Ser Leu Thr
705                 710                 715                 720

Phe Lys Glu Asp Ile Gln Lys Ala Gln Val Ser Gly Gln Gly Asp Ser
                725                 730                 735

Leu His Glu His Ile Ala Asn Leu Ala Gly Ser Pro Ala Ile Lys Lys
            740                 745                 750

Gly Ile Leu Gln Thr Val Lys Val Val Asp Glu Leu Val Lys Val Met
        755                 760                 765

Gly Arg His Lys Pro Glu Asn Ile Val Ile Glu Met Ala Arg Glu Asn
    770                 775                 780

Gln Thr Thr Gln Lys Gly Gln Lys Asn Ser Arg Glu Arg Met Lys Arg
785                 790                 795                 800

Ile Glu Glu Gly Ile Lys Glu Leu Gly Ser Gln Ile Leu Lys Glu His
                805                 810                 815

Pro Val Glu Asn Thr Gln Leu Gln Asn Glu Lys Leu Tyr Leu Tyr Tyr
            820                 825                 830

Leu Gln Asn Gly Arg Asp Met Tyr Val Asp Gln Glu Leu Asp Ile Asn
        835                 840                 845

Arg Leu Ser Asp Tyr Asp Val Asp His Ile Val Pro Gln Ser Phe Leu

850                 855                 860

Lys Asp Asp Ser Ile Asp Asn Lys Val Leu Thr Arg Ser Asp Lys Asn
865                 870                 875                 880

Arg Gly Lys Ser Asp Asn Val Pro Ser Glu Glu Val Val Lys Lys Met
                885                 890                 895

Lys Asn Tyr Trp Arg Gln Leu Leu Asn Ala Lys Leu Ile Thr Gln Arg
            900                 905                 910

Lys Phe Asp Asn Leu Thr Lys Ala Glu Arg Gly Gly Leu Ser Glu Leu
        915                 920                 925

Asp Lys Ala Gly Phe Ile Lys Arg Gln Leu Val Glu Thr Arg Gln Ile
    930                 935                 940

Thr Lys His Val Ala Gln Ile Leu Asp Ser Arg Met Asn Thr Lys Tyr
945                 950                 955                 960

Asp Glu Asn Asp Lys Leu Ile Arg Glu Val Lys Val Ile Thr Leu Lys
                965                 970                 975

Ser Lys Leu Val Ser Asp Phe Arg Lys Asp Phe Gln Phe Tyr Lys Val
            980                 985                 990

Arg Glu Ile Asn Asn Tyr His His Ala His Asp Ala Tyr Leu Asn Ala
        995                 1000                1005

Val Val Gly Thr Ala Leu Ile Lys Lys Tyr Pro Lys Leu Glu Ser Glu
    1010                1015                1020

Phe Val Tyr Gly Asp Tyr Lys Val Tyr Asp Val Arg Lys Met Ile Ala
1025                1030                1035                1040

Lys Ser Glu Gln Glu Ile Gly Lys Ala Thr Ala Lys Tyr Phe Phe Tyr
                1045                1050                1055

Ser Asn Ile Met Asn Phe Phe Lys Thr Glu Ile Thr Leu Ala Asn Gly
            1060                1065                1070

Glu Ile Arg Lys Arg Pro Leu Ile Glu Thr Asn Gly Glu Thr Gly Glu
        1075                1080                1085

Ile Val Trp Asp Lys Gly Arg Asp Phe Ala Thr Val Arg Lys Val Leu
    1090                1095                1100

Ser Met Pro Gln Val Asn Ile Val Lys Lys Thr Glu Val Gln Thr Gly
1105                1110                1115                1120

Gly Phe Ser Lys Glu Ser Ile Leu Pro Lys Arg Asn Ser Asp Lys Leu
                1125                1130                1135

Ile Ala Arg Lys Lys Asp Trp Asp Pro Lys Lys Tyr Gly Gly Phe Asp
            1140                1145                1150

Ser Pro Thr Val Ala Tyr Ser Val Leu Val Val Ala Lys Val Glu Lys
        1155                1160                1165

Gly Lys Ser Lys Lys Leu Lys Ser Val Lys Glu Leu Leu Gly Ile Thr
    1170                1175                1180

Ile Met Glu Arg Ser Ser Phe Glu Lys Asn Pro Ile Asp Phe Leu Glu

1185                1190                1195                1200

Ala Lys Gly Tyr Lys Glu Val Lys Lys Asp Leu Ile Ile Lys Leu Pro
                1205                1210                1215

Lys Tyr Ser Leu Phe Glu Leu Glu Asn Gly Arg Lys Arg Met Leu Ala
            1220                1225                1230

Ser Ala Gly Glu Leu Gln Lys Gly Asn Glu Leu Ala Leu Pro Ser Lys
        1235                1240                1245

Tyr Val Asn Phe Leu Tyr Leu Ala Ser His Tyr Glu Lys Leu Lys Gly
    1250                1255                1260

Ser Pro Glu Asp Asn Glu Gln Lys Gln Leu Phe Val Glu Gln His Lys
1265                1270                1275                1280

His Tyr Leu Asp Glu Ile Ile Glu Gln Ile Ser Glu Phe Ser Lys Arg
                1285                1290                1295

Val Ile Leu Ala Asp Ala Asn Leu Asp Lys Val Leu Ser Ala Tyr Asn
            1300                1305                1310

Lys His Arg Asp Lys Pro Ile Arg Glu Gln Ala Glu Asn Ile Ile His
        1315                1320                1325

Leu Phe Thr Leu Thr Asn Leu Gly Ala Pro Ala Ala Phe Lys Tyr Phe
    1330                1335                1340

Asp Thr Thr Ile Asp Arg Lys Arg Tyr Thr Ser Thr Lys Glu Val Leu
1345                1350                1355                1360

Asp Ala Thr Leu Ile His Gln Ser Ile Thr Gly Leu Tyr Glu Thr Arg
                1365                1370                1375

Ile Asp Leu Ser Gln Leu Gly Gly Asp Ser Arg Ala Asp Pro Lys Lys
            1380                1385                1390

Lys Arg Lys Val
        1395

<210> 3
<211> 1155
<212> AA (amino acid)
<213> artificial
<400> SEQUENCE 3 (STCas9):

Met Leu Glu His His His His His His Gly Ser Pro Lys Lys Lys Arg
1               5                   10                  15

Lys Val Gly Arg Gly Ala Thr Met Ser Asp Leu Val Leu Gly Leu Asp
            20                  25                  30

Ile Gly Ile Gly Ser Val Gly Val Gly Ile Leu Asn Lys Val Thr Gly
        35                  40                  45

Glu Ile Ile His Lys Asn Ser Arg Ile Phe Pro Ala Ala Gln Ala Glu
    50                  55                  60

Asn Asn Leu Val Arg Arg Thr Asn Arg Gln Gly Arg Arg Leu Ala Arg
65                  70                  75                  80

Arg Lys Lys His Arg Arg Val Arg Leu Asn Arg Leu Phe Glu Glu Ser

                85                  90                  95

Gly Leu Ile Thr Asp Phe Thr Lys Ile Ser Ile Asn Leu Asn Pro Tyr
            100                 105                 110

Gln Leu Arg Val Lys Gly Leu Thr Asp Glu Leu Ser Asn Glu Glu Leu
        115                 120                 125

Phe Ile Ala Leu Lys Asn Met Val Lys His Arg Gly Ile Ser Tyr Leu
    130                 135                 140

Asp Asp Ala Ser Asp Asp Gly Asn Ser Ser Val Gly Asp Tyr Ala Gln
145                 150                 155                 160

Ile Val Lys Glu Asn Ser Lys Gln Leu Glu Thr Lys Thr Pro Gly Gln
                165                 170                 175

Ile Gln Leu Glu Arg Tyr Gln Thr Tyr Gly Gln Leu Arg Gly Asp Phe
            180                 185                 190

Thr Val Glu Lys Asp Gly Lys Lys His Arg Leu Ile Asn Val Phe Pro
        195                 200                 205

Thr Ser Ala Tyr Arg Ser Glu Ala Leu Arg Ile Leu Gln Thr Gln Gln
    210                 215                 220

Glu Phe Asn Pro Gln Ile Thr Asp Glu Phe Ile Asn Arg Tyr Leu Glu
225                 230                 235                 240

Ile Leu Thr Gly Lys Arg Lys Tyr Tyr His Gly Pro Gly Asn Glu Lys
                245                 250                 255

Ser Arg Thr Asp Tyr Gly Arg Tyr Arg Thr Ser Gly Glu Thr Leu Asp
            260                 265                 270

Asn Ile Phe Gly Ile Leu Ile Gly Lys Cys Thr Phe Tyr Pro Asp Glu
        275                 280                 285

Phe Arg Ala Ala Lys Ala Ser Tyr Thr Ala Gln Glu Phe Asn Leu Leu
    290                 295                 300

Asn Asp Leu Asn Asn Leu Thr Val Pro Thr Glu Thr Lys Lys Leu Ser
305                 310                 315                 320

Lys Glu Gln Lys Asn Gln Ile Ile Asn Tyr Val Lys Asn Glu Lys Ala
                325                 330                 335

Met Gly Pro Ala Lys Leu Phe Lys Tyr Ile Ala Lys Leu Leu Ser Cys
            340                 345                 350

Asp Val Ala Asp Ile Lys Gly Tyr Arg Ile Asp Lys Ser Gly Lys Ala
        355                 360                 365

Glu Ile His Thr Phe Glu Ala Tyr Arg Lys Met Lys Thr Leu Glu Thr
    370                 375                 380

Leu Asp Ile Glu Gln Met Asp Arg Glu Thr Leu Asp Lys Leu Ala Tyr
385                 390                 395                 400

Val Leu Thr Leu Asn Thr Glu Arg Glu Gly Ile Gln Glu Ala Leu Glu
                405                 410                 415

His Glu Phe Ala Asp Gly Ser Phe Ser Gln Lys Gln Val Asp Glu Leu

            420                 425                 430

Val Gln Phe Arg Lys Ala Asn Ser Ser Ile Phe Gly Lys Gly Trp His
        435                 440                 445

Asn Phe Ser Val Lys Leu Met Met Glu Leu Ile Pro Glu Leu Tyr Glu
    450                 455                 460

Thr Ser Glu Glu Gln Met Thr Ile Leu Thr Arg Leu Gly Lys Gln Lys
465                 470                 475                 480

Thr Thr Ser Ser Ser Asn Lys Thr Lys Tyr Ile Asp Glu Lys Leu Leu
                485                 490                 495

Thr Glu Glu Ile Tyr Asn Pro Val Val Ala Lys Ser Val Arg Gln Ala
            500                 505                 510

Ile Lys Ile Val Asn Ala Ala Ile Lys Glu Tyr Gly Asp Phe Asp Asn
        515                 520                 525

Ile Val Ile Glu Met Ala Arg Glu Thr Asn Glu Asp Asp Glu Lys Lys
    530                 535                 540

Ala Ile Gln Lys Ile Gln Lys Ala Asn Lys Asp Glu Lys Asp Ala Ala
545                 550                 555                 560

Met Leu Lys Ala Ala Asn Gln Tyr Asn Gly Lys Ala Glu Leu Pro His
                565                 570                 575

Ser Val Phe His Gly His Lys Gln Leu Ala Thr Lys Ile Arg Leu Trp
            580                 585                 590

His Gln Gln Gly Glu Arg Cys Leu Tyr Thr Gly Lys Thr Ile Ser Ile
        595                 600                 605

His Asp Leu Ile Asn Asn Ser Asn Gln Phe Glu Val Asp His Ile Leu
    610                 615                 620

Pro Leu Ser Ile Thr Phe Asp Asp Ser Leu Ala Asn Lys Val Leu Val
625                 630                 635                 640

Tyr Ala Thr Ala Asn Gln Glu Lys Gly Gln Arg Thr Pro Tyr Gln Ala
                645                 650                 655

Leu Asp Ser Met Asp Asp Ala Trp Ser Phe Arg Glu Leu Lys Ala Phe
            660                 665                 670

Val Arg Glu Ser Lys Thr Leu Ser Asn Lys Lys Lys Glu Tyr Leu Leu
        675                 680                 685

Thr Glu Glu Asp Ile Ser Lys Phe Asp Val Arg Lys Lys Phe Ile Glu
    690                 695                 700

Arg Asn Leu Val Asp Thr Leu Tyr Ala Ser Arg Val Val Leu Asn Ala
705                 710                 715                 720

Leu Gln Glu His Phe Arg Ala His Lys Ile Asp Thr Lys Val Ser Val
                725                 730                 735

Val Arg Gly Gln Phe Thr Ser Gln Leu Arg Arg His Trp Gly Ile Glu
            740                 745                 750

Lys Thr Arg Asp Thr Tyr His His His Ala Val Asp Ala Leu Ile Ile

755                 760                 765

Ala Ala Ser Ser Gln Leu Asn Leu Trp Lys Lys Gln Lys Asn Thr Leu
    770                 775                 780

Val Ser Tyr Ser Glu Asp Gln Leu Leu Asp Ile Glu Thr Gly Glu Leu
785                 790                 795                 800

Ile Ser Asp Asp Glu Tyr Lys Glu Ser Val Phe Lys Ala Pro Tyr Gln
                805                 810                 815

His Phe Val Asp Thr Leu Lys Ser Lys Glu Phe Glu Asp Ser Ile Leu
            820                 825                 830

Phe Ser Tyr Gln Val Asp Ser Lys Phe Asn Arg Lys Ile Ser Asp Ala
        835                 840                 845

Thr Ile Tyr Ala Thr Arg Gln Ala Lys Val Gly Lys Asp Lys Ala Asp
    850                 855                 860

Glu Thr Tyr Val Leu Gly Lys Ile Lys Asp Ile Tyr Thr Gln Asp Gly
865                 870                 875                 880

Tyr Asp Ala Phe Met Lys Ile Tyr Lys Lys Asp Lys Ser Lys Phe Leu
                885                 890                 895

Met Tyr Arg His Asp Pro Gln Thr Phe Glu Lys Val Ile Glu Pro Ile
            900                 905                 910

Leu Glu Asn Tyr Pro Asn Lys Gln Ile Asn Asp Lys Gly Lys Glu Val
        915                 920                 925

Pro Cys Asn Pro Phe Leu Lys Tyr Lys Glu Glu His Gly Tyr Ile Arg
    930                 935                 940

Lys Tyr Ser Lys Lys Gly Asn Gly Pro Glu Ile Lys Ser Leu Lys Tyr
945                 950                 955                 960

Tyr Asp Ser Lys Leu Gly Asn His Ile Asp Ile Thr Pro Lys Asp Ser
                965                 970                 975

Asn Asn Lys Val Val Leu Gln Ser Val Ser Pro Trp Arg Ala Asp Val
            980                 985                 990

Tyr Phe Asn Lys Thr Thr Gly Lys Tyr Glu Ile Leu Gly Leu Lys Tyr
        995                 1000                1005

Ala Asp Leu Gln Phe Asp Lys Gly Thr Gly Thr Tyr Lys Ile Ser Gln
    1010                1015                1020

Glu Lys Tyr Asn Asp Ile Lys Lys Lys Glu Gly Val Asp Ser Asp Ser
1025                1030                1035                1040

Glu Phe Lys Phe Thr Leu Tyr Lys Asn Asp Leu Leu Leu Val Lys Asp
                1045                1050                1055

Thr Glu Thr Lys Glu Gln Gln Leu Phe Arg Phe Leu Ser Arg Thr Met
            1060                1065                1070

Pro Lys Gln Lys His Tyr Val Glu Leu Lys Pro Tyr Asp Lys Gln Lys
        1075                1080                1085

Phe Glu Gly Gly Glu Ala Leu Ile Lys Val Leu Gly Asn Val Ala Asn

1090                    1095                    1100

Ser Gly Gln Cys Lys Lys Gly Leu Gly Lys Ser Asn Ile Ser Ile Tyr
1105                    1110                    1115                    1120

Lys Val Arg Thr Asp Val Leu Gly Asn Gln His Ile Ile Lys Asn Glu
                1125                    1130                    1135

Gly Asp Lys Pro Lys Leu Asp Phe Ser Arg Ala Asp Pro Lys Lys Lys
            1140                    1145                    1150

Arg Lys Val
        1155

<210> 4
<211> 1340
<212> AA (amino acid)
<213> artificial
<400> SEQUENCE 4 (AsCpf1):

Met His His His His His His His Gly Ser Pro Lys Lys Lys Arg Lys
1               5                   10                  15

Val Met Thr Gln Phe Glu Gly Phe Thr Asn Leu Tyr Gln Val Ser Lys
            20                  25                  30

Thr Leu Arg Phe Glu Leu Ile Pro Gln Gly Lys Thr Leu Lys His Ile
        35                  40                  45

Gln Glu Gln Gly Phe Ile Glu Glu Asp Lys Ala Arg Asn Asp His Tyr
    50                  55                  60

Lys Glu Leu Lys Pro Ile Ile Asp Arg Ile Tyr Lys Thr Tyr Ala Asp
65                  70                  75                  80

Gln Cys Leu Gln Leu Val Gln Leu Asp Trp Glu Asn Leu Ser Ala Ala
                85                  90                  95

Ile Asp Ser Tyr Arg Lys Glu Lys Thr Glu Glu Thr Arg Asn Ala Leu
            100                 105                 110

Ile Glu Glu Gln Ala Thr Tyr Arg Asn Ala Ile His Asp Tyr Phe Ile
        115                 120                 125

Gly Arg Thr Asp Asn Leu Thr Asp Ala Ile Asn Lys Arg His Ala Glu
    130                 135                 140

Ile Tyr Lys Gly Leu Phe Lys Ala Glu Leu Phe Asn Gly Lys Val Leu
145                 150                 155                 160

Lys Gln Leu Gly Thr Val Thr Thr Thr Glu His Glu Asn Ala Leu Leu
                165                 170                 175

Arg Ser Phe Asp Lys Phe Thr Thr Tyr Phe Ser Gly Phe Tyr Glu Asn
            180                 185                 190

Arg Lys Asn Val Phe Ser Ala Glu Asp Ile Ser Thr Ala Ile Pro His
        195                 200                 205

Arg Ile Val Gln Asp Asn Phe Pro Lys Phe Lys Glu Asn Cys His Ile
    210                 215                 220

Phe Thr Arg Leu Ile Thr Ala Val Pro Ser Leu Arg Glu His Phe Glu

225                 230                 235                 240

Asn Val Lys Lys Ala Ile Gly Ile Phe Val Ser Thr Ser Ile Glu Glu
                245                 250                 255

Val Phe Ser Phe Pro Phe Tyr Asn Gln Leu Leu Thr Gln Thr Gln Ile
            260                 265                 270

Asp Leu Tyr Asn Gln Leu Leu Gly Gly Ile Ser Arg Glu Ala Gly Thr
        275                 280                 285

Glu Lys Ile Lys Gly Leu Asn Glu Val Leu Asn Leu Ala Ile Gln Lys
    290                 295                 300

Asn Asp Glu Thr Ala His Ile Ile Ala Ser Leu Pro His Arg Phe Ile
305                 310                 315                 320

Pro Leu Phe Lys Gln Ile Leu Ser Asp Arg Asn Thr Leu Ser Phe Ile
                325                 330                 335

Leu Glu Glu Phe Lys Ser Asp Glu Glu Val Ile Gln Ser Phe Cys Lys
            340                 345                 350

Tyr Lys Thr Leu Leu Arg Asn Glu Asn Val Leu Glu Thr Ala Glu Ala
        355                 360                 365

Leu Phe Asn Glu Leu Asn Ser Ile Asp Leu Thr His Ile Phe Ile Ser
    370                 375                 380

His Lys Lys Leu Glu Thr Ile Ser Ser Ala Leu Cys Asp His Trp Asp
385                 390                 395                 400

Thr Leu Arg Asn Ala Leu Tyr Glu Arg Arg Ile Ser Glu Leu Thr Gly
                405                 410                 415

Lys Ile Thr Lys Ser Ala Lys Glu Lys Val Gln Arg Ser Leu Lys His
            420                 425                 430

Glu Asp Ile Asn Leu Gln Glu Ile Ile Ser Ala Ala Gly Lys Glu Leu
        435                 440                 445

Ser Glu Ala Phe Lys Gln Lys Thr Ser Glu Ile Leu Ser His Ala His
    450                 455                 460

Ala Ala Leu Asp Gln Pro Leu Pro Thr Thr Leu Lys Lys Gln Glu Glu
465                 470                 475                 480

Lys Glu Ile Leu Lys Ser Gln Leu Asp Ser Leu Leu Gly Leu Tyr His
                485                 490                 495

Leu Leu Asp Trp Phe Ala Val Asp Glu Ser Asn Glu Val Asp Pro Glu
            500                 505                 510

Phe Ser Ala Arg Leu Thr Gly Ile Lys Leu Glu Met Glu Pro Ser Leu
        515                 520                 525

Ser Phe Tyr Asn Lys Ala Arg Asn Tyr Ala Thr Lys Lys Pro Tyr Ser
    530                 535                 540

Val Glu Lys Phe Lys Leu Asn Phe Gln Met Pro Thr Leu Ala Ser Gly
545                 550                 555                 560

Trp Asp Val Asn Lys Glu Lys Asn Asn Gly Ala Ile Leu Phe Val Lys

565                 570                 575

Asn Gly Leu Tyr Tyr Leu Gly Ile Met Pro Lys Gln Lys Gly Arg Tyr
            580                 585                 590

Lys Ala Leu Ser Phe Glu Pro Thr Glu Lys Thr Ser Glu Gly Phe Asp
        595                 600                 605

Lys Met Tyr Tyr Asp Tyr Phe Pro Asp Ala Ala Lys Met Ile Pro Lys
    610                 615                 620

Cys Ser Thr Gln Leu Lys Ala Val Thr Ala His Phe Gln Thr His Thr
625                 630                 635                 640

Thr Pro Ile Leu Leu Ser Asn Asn Phe Ile Glu Pro Leu Glu Ile Thr
                645                 650                 655

Lys Glu Ile Tyr Asp Leu Asn Asn Pro Glu Lys Glu Pro Lys Lys Phe
            660                 665                 670

Gln Thr Ala Tyr Ala Lys Lys Thr Gly Asp Gln Lys Gly Tyr Arg Glu
        675                 680                 685

Ala Leu Cys Lys Trp Ile Asp Phe Thr Arg Asp Phe Leu Ser Lys Tyr
    690                 695                 700

Thr Lys Thr Thr Ser Ile Asp Leu Ser Ser Leu Arg Pro Ser Ser Gln
705                 710                 715                 720

Tyr Lys Asp Leu Gly Glu Tyr Tyr Ala Glu Leu Asn Pro Leu Leu Tyr
                725                 730                 735

His Ile Ser Phe Gln Arg Ile Ala Glu Lys Glu Ile Met Asp Ala Val
            740                 745                 750

Glu Thr Gly Lys Leu Tyr Leu Phe Gln Ile Tyr Asn Lys Asp Phe Ala
        755                 760                 765

Lys Gly His His Gly Lys Pro Asn Leu His Thr Leu Tyr Trp Thr Gly
    770                 775                 780

Leu Phe Ser Pro Glu Asn Leu Ala Lys Thr Ser Ile Lys Leu Asn Gly
785                 790                 795                 800

Gln Ala Glu Leu Phe Tyr Arg Pro Lys Ser Arg Met Lys Arg Met Ala
                805                 810                 815

His Arg Leu Gly Glu Lys Met Leu Asn Lys Lys Leu Lys Asp Gln Lys
            820                 825                 830

Thr Pro Ile Pro Asp Thr Leu Tyr Gln Glu Leu Tyr Asp Tyr Val Asn
        835                 840                 845

His Arg Leu Ser His Asp Leu Ser Asp Glu Ala Arg Ala Leu Leu Pro
    850                 855                 860

Asn Val Ile Thr Lys Glu Val Ser His Glu Ile Ile Lys Asp Arg Arg
865                 870                 875                 880

Phe Thr Ser Asp Lys Phe Phe Phe His Val Pro Ile Thr Leu Asn Tyr
                885                 890                 895

Gln Ala Ala Asn Ser Pro Ser Lys Phe Asn Gln Arg Val Asn Ala Tyr

            900                 905                 910

Leu Lys Glu His Pro Glu Thr Pro Ile Ile Gly Ile Asp Arg Gly Glu
        915                 920                 925

Arg Asn Leu Ile Tyr Ile Thr Val Ile Asp Ser Thr Gly Lys Ile Leu
    930                 935                 940

Glu Gln Arg Ser Leu Asn Thr Ile Gln Gln Phe Asp Tyr Gln Lys Lys
945                 950                 955                 960

Leu Asp Asn Arg Glu Lys Glu Arg Val Ala Ala Arg Gln Ala Trp Ser
                965                 970                 975

Val Val Gly Thr Ile Lys Asp Leu Lys Gln Gly Tyr Leu Ser Gln Val
            980                 985                 990

Ile His Glu Ile Val Asp Leu Met Ile His Tyr Gln Ala Val Val Val
        995                 1000                1005

Leu Glu Asn Leu Asn Phe Gly Phe Lys Ser Lys Arg Thr Gly Ile Ala
    1010                1015                1020

Glu Lys Ala Val Tyr Gln Gln Phe Glu Lys Met Leu Ile Asp Lys Leu
1025                1030                1035                1040

Asn Cys Leu Val Leu Lys Asp Tyr Pro Ala Glu Lys Val Gly Gly Val
                1045                1050                1055

Leu Asn Pro Tyr Gln Leu Thr Asp Gln Phe Thr Ser Phe Ala Lys Met
            1060                1065                1070

Gly Thr Gln Ser Gly Phe Leu Phe Tyr Val Pro Ala Pro Tyr Thr Ser
        1075                1080                1085

Lys Ile Asp Pro Leu Thr Gly Phe Val Asp Pro Phe Val Trp Lys Thr
    1090                1095                1100

Ile Lys Asn His Glu Ser Arg Lys His Phe Leu Glu Gly Phe Asp Phe
1105                1110                1115                1120

Leu His Tyr Asp Val Lys Thr Gly Asp Phe Ile Leu His Phe Lys Met
                1125                1130                1135

Asn Arg Asn Leu Ser Phe Gln Arg Gly Leu Pro Gly Phe Met Pro Ala
            1140                1145                1150

Trp Asp Ile Val Phe Glu Lys Asn Glu Thr Gln Phe Asp Ala Lys Gly
        1155                1160                1165

Thr Pro Phe Ile Ala Gly Lys Arg Ile Val Pro Val Ile Glu Asn His
    1170                1175                1180

Arg Phe Thr Gly Arg Tyr Arg Asp Leu Tyr Pro Ala Asn Glu Leu Ile
1185                1190                1195                1200

Ala Leu Leu Glu Glu Lys Gly Ile Val Phe Arg Asp Gly Ser Asn Ile
                1205                1210                1215

Leu Pro Lys Leu Leu Glu Asn Asp Asp Ser His Ala Ile Asp Thr Met
            1220                1225                1230

Val Ala Leu Ile Arg Ser Val Leu Gln Met Arg Asn Ser Asn Ala Ala

1235                1240                1245

Thr Gly Glu Asp Tyr Ile Asn Ser Pro Val Arg Asp Leu Asn Gly Val
    1250                1255                1260

Cys Phe Asp Ser Arg Phe Gln Asn Pro Glu Trp Pro Met Asp Ala Asp
1265                1270                1275                1280

Ala Asn Gly Ala Tyr His Ile Ala Leu Lys Gly Gln Leu Leu Leu Asn
                1285                1290                1295

His Leu Lys Glu Ser Lys Asp Leu Lys Leu Gln Asn Gly Ile Ser Asn
            1300                1305                1310

Gln Asp Trp Leu Ala Tyr Ile Gln Glu Leu Arg Asn Lys Arg Pro Ala
        1315                1320                1325

Ala Thr Lys Lys Ala Gly Gln Ala Lys Lys Lys Lys
    1330                1335                1340


<210> 5
<211> 1410
<212> AA (amino acid)
<213> artificial
<400> SEQUENCE 5 (TATSPCas9):
Met His Gly Arg Lys Lys Arg Arg Gln Arg Arg Arg Pro Gln Gly Ser
1               5                   10                  15

His His His His His His Gly Ser Pro Lys Lys Lys Arg Lys Val Met
            20                  25                  30

Asp Lys Lys Tyr Ser Ile Gly Leu Asp Ile Gly Thr Asn Ser Val Gly
        35                  40                  45

Trp Ala Val Ile Thr Asp Glu Tyr Lys Val Pro Ser Lys Lys Phe Lys
    50                  55                  60

Val Leu Gly Asn Thr Asp Arg His Ser Ile Lys Lys Asn Leu Ile Gly
65                  70                  75                  80

Ala Leu Leu Phe Asp Ser Gly Glu Thr Ala Glu Ala Thr Arg Leu Lys
                85                  90                  95

Arg Thr Ala Arg Arg Arg Tyr Thr Arg Arg Lys Asn Arg Ile Cys Tyr
            100                 105                 110

Leu Gln Glu Ile Phe Ser Asn Glu Met Ala Lys Val Asp Asp Ser Phe
        115                 120                 125

Phe His Arg Leu Glu Glu Ser Phe Leu Val Glu Glu Asp Lys Lys His
    130                 135                 140

Glu Arg His Pro Ile Phe Gly Asn Ile Val Asp Glu Val Ala Tyr His
145                 150                 155                 160

Glu Lys Tyr Pro Thr Ile Tyr His Leu Arg Lys Lys Leu Val Asp Ser
                165                 170                 175

Thr Asp Lys Ala Asp Leu Arg Leu Ile Tyr Leu Ala Leu Ala His Met
            180                 185                 190

Ile Lys Phe Arg Gly His Phe Leu Ile Glu Gly Asp Leu Asn Pro Asp

195                 200                 205

Asn Ser Asp Val Asp Lys Leu Phe Ile Gln Leu Val Gln Thr Tyr Asn
    210                 215                 220

Gln Leu Phe Glu Glu Asn Pro Ile Asn Ala Ser Gly Val Asp Ala Lys
225                 230                 235                 240

Ala Ile Leu Ser Ala Arg Leu Ser Lys Ser Arg Arg Leu Glu Asn Leu
                245                 250                 255

Ile Ala Gln Leu Pro Gly Glu Lys Lys Asn Gly Leu Phe Gly Asn Leu
            260                 265                 270

Ile Ala Leu Ser Leu Gly Leu Thr Pro Asn Phe Lys Ser Asn Phe Asp
        275                 280                 285

Leu Ala Glu Asp Ala Lys Leu Gln Leu Ser Lys Asp Thr Tyr Asp Asp
    290                 295                 300

Asp Leu Asp Asn Leu Leu Ala Gln Ile Gly Asp Gln Tyr Ala Asp Leu
305                 310                 315                 320

Phe Leu Ala Ala Lys Asn Leu Ser Asp Ala Ile Leu Leu Ser Asp Ile
                325                 330                 335

Leu Arg Val Asn Thr Glu Ile Thr Lys Ala Pro Leu Ser Ala Ser Met
            340                 345                 350

Ile Lys Arg Tyr Asp Glu His His Gln Asp Leu Thr Leu Leu Lys Ala
        355                 360                 365

Leu Val Arg Gln Gln Leu Pro Glu Lys Tyr Lys Glu Ile Phe Phe Asp
    370                 375                 380

Gln Ser Lys Asn Gly Tyr Ala Gly Tyr Ile Asp Gly Gly Ala Ser Gln
385                 390                 395                 400

Glu Glu Phe Tyr Lys Phe Ile Lys Pro Ile Leu Glu Lys Met Asp Gly
                405                 410                 415

Thr Glu Glu Leu Leu Val Lys Leu Asn Arg Glu Asp Leu Leu Arg Lys
            420                 425                 430

Gln Arg Thr Phe Asp Asn Gly Ser Ile Pro His Gln Ile His Leu Gly
        435                 440                 445

Glu Leu His Ala Ile Leu Arg Arg Gln Glu Asp Phe Tyr Pro Phe Leu
    450                 455                 460

Lys Asp Asn Arg Glu Lys Ile Glu Lys Ile Leu Thr Phe Arg Ile Pro
465                 470                 475                 480

Tyr Tyr Val Gly Pro Leu Ala Arg Gly Asn Ser Arg Phe Ala Trp Met
                485                 490                 495

Thr Arg Lys Ser Glu Glu Thr Ile Thr Pro Trp Asn Phe Glu Glu Val
            500                 505                 510

Val Asp Lys Gly Ala Ser Ala Gln Ser Phe Ile Glu Arg Met Thr Asn
        515                 520                 525

Phe Asp Lys Asn Leu Pro Asn Glu Lys Val Leu Pro Lys His Ser Leu
    530                 535                 540
Leu Tyr Glu Tyr Phe Thr Val Tyr Asn Glu Leu Thr Lys Val Lys Tyr
545                 550                 555                 560
Val Thr Glu Gly Met Arg Lys Pro Ala Phe Leu Ser Gly Glu Gln Lys
                565                 570                 575
Lys Ala Ile Val Asp Leu Leu Phe Lys Thr Asn Arg Lys Val Thr Val
            580                 585                 590
Lys Gln Leu Lys Glu Asp Tyr Phe Lys Lys Ile Glu Cys Phe Asp Ser
        595                 600                 605
Val Glu Ile Ser Gly Val Glu Asp Arg Phe Asn Ala Ser Leu Gly Thr
    610                 615                 620
Tyr His Asp Leu Leu Lys Ile Ile Lys Asp Lys Asp Phe Leu Asp Asn
625                 630                 635                 640
Glu Glu Asn Glu Asp Ile Leu Glu Asp Ile Val Leu Thr Leu Thr Leu
                645                 650                 655
Phe Glu Asp Arg Glu Met Ile Glu Glu Arg Leu Lys Thr Tyr Ala His
            660                 665                 670
Leu Phe Asp Asp Lys Val Met Lys Gln Leu Lys Arg Arg Arg Tyr Thr
        675                 680                 685
Gly Trp Gly Arg Leu Ser Arg Lys Leu Ile Asn Gly Ile Arg Asp Lys
    690                 695                 700
Gln Ser Gly Lys Thr Ile Leu Asp Phe Leu Lys Ser Asp Gly Phe Ala
705                 710                 715                 720
Asn Arg Asn Phe Met Gln Leu Ile His Asp Asp Ser Leu Thr Phe Lys
                725                 730                 735
Glu Asp Ile Gln Lys Ala Gln Val Ser Gly Gln Gly Asp Ser Leu His
            740                 745                 750
Glu His Ile Ala Asn Leu Ala Gly Ser Pro Ala Ile Lys Lys Gly Ile
        755                 760                 765
Leu Gln Thr Val Lys Val Val Asp Glu Leu Val Lys Val Met Gly Arg
    770                 775                 780
His Lys Pro Glu Asn Ile Val Ile Glu Met Ala Arg Glu Asn Gln Thr
785                 790                 795                 800
Thr Gln Lys Gly Gln Lys Asn Ser Arg Glu Arg Met Lys Arg Ile Glu
                805                 810                 815
Glu Gly Ile Lys Glu Leu Gly Ser Gln Ile Leu Lys Glu His Pro Val
            820                 825                 830
Glu Asn Thr Gln Leu Gln Asn Glu Lys Leu Tyr Leu Tyr Tyr Leu Gln
        835                 840                 845
Asn Gly Arg Asp Met Tyr Val Asp Gln Glu Leu Asp Ile Asn Arg Leu
    850                 855                 860

Ser Asp Tyr Asp Val Asp His Ile Val Pro Gln Ser Phe Leu Lys Asp
865                 870                 875                 880

Asp Ser Ile Asp Asn Lys Val Leu Thr Arg Ser Asp Lys Asn Arg Gly
                885                 890                 895

Lys Ser Asp Asn Val Pro Ser Glu Glu Val Val Lys Lys Met Lys Asn
            900                 905                 910

Tyr Trp Arg Gln Leu Leu Asn Ala Lys Leu Ile Thr Gln Arg Lys Phe
        915                 920                 925

Asp Asn Leu Thr Lys Ala Glu Arg Gly Gly Leu Ser Glu Leu Asp Lys
    930                 935                 940

Ala Gly Phe Ile Lys Arg Gln Leu Val Glu Thr Arg Gln Ile Thr Lys
945                 950                 955                 960

His Val Ala Gln Ile Leu Asp Ser Arg Met Asn Thr Lys Tyr Asp Glu
                965                 970                 975

Asn Asp Lys Leu Ile Arg Glu Val Lys Val Ile Thr Leu Lys Ser Lys
            980                 985                 990

Leu Val Ser Asp Phe Arg Lys Asp Phe Gln Phe Tyr Lys Val Arg Glu
        995                 1000                1005

Ile Asn Asn Tyr His His Ala His Asp Ala Tyr Leu Asn Ala Val Val
    1010                1015                1020

Gly Thr Ala Leu Ile Lys Lys Tyr Pro Lys Leu Glu Ser Glu Phe Val
1025                1030                1035                1040

Tyr Gly Asp Tyr Lys Val Tyr Asp Val Arg Lys Met Ile Ala Lys Ser
                1045                1050                1055

Glu Gln Glu Ile Gly Lys Ala Thr Ala Lys Tyr Phe Phe Tyr Ser Asn
            1060                1065                1070

Ile Met Asn Phe Phe Lys Thr Glu Ile Thr Leu Ala Asn Gly Glu Ile
        1075                1080                1085

Arg Lys Arg Pro Leu Ile Glu Thr Asn Gly Glu Thr Gly Glu Ile Val
    1090                1095                1100

Trp Asp Lys Gly Arg Asp Phe Ala Thr Val Arg Lys Val Leu Ser Met
1105                1110                1115                1120

Pro Gln Val Asn Ile Val Lys Lys Thr Glu Val Gln Thr Gly Gly Phe
                1125                1130                1135

Ser Lys Glu Ser Ile Leu Pro Lys Arg Asn Ser Asp Lys Leu Ile Ala
            1140                1145                1150

Arg Lys Lys Asp Trp Asp Pro Lys Lys Tyr Gly Gly Phe Asp Ser Pro
        1155                1160                1165

Thr Val Ala Tyr Ser Val Leu Val Val Ala Lys Val Glu Lys Gly Lys
    1170                1175                1180

Ser Lys Lys Leu Lys Ser Val Lys Glu Leu Leu Gly Ile Thr Ile Met
1185                1190                1195                1200

Glu Arg Ser Ser Phe Glu Lys Asn Pro Ile Asp Phe Leu Glu Ala Lys
                1205                1210                1215

Gly Tyr Lys Glu Val Lys Lys Asp Leu Ile Ile Lys Leu Pro Lys Tyr
            1220                1225                1230

Ser Leu Phe Glu Leu Glu Asn Gly Arg Lys Arg Met Leu Ala Ser Ala
        1235                1240                1245

Gly Glu Leu Gln Lys Gly Asn Glu Leu Ala Leu Pro Ser Lys Tyr Val
    1250                1255                1260

Asn Phe Leu Tyr Leu Ala Ser His Tyr Glu Lys Leu Lys Gly Ser Pro
1265                1270                1275                1280

Glu Asp Asn Glu Gln Lys Gln Leu Phe Val Glu Gln His Lys His Tyr
                1285                1290                1295

Leu Asp Glu Ile Ile Glu Gln Ile Ser Glu Phe Ser Lys Arg Val Ile
            1300                1305                1310

Leu Ala Asp Ala Asn Leu Asp Lys Val Leu Ser Ala Tyr Asn Lys His
        1315                1320                1325

Arg Asp Lys Pro Ile Arg Glu Gln Ala Glu Asn Ile Ile His Leu Phe
    1330                1335                1340

Thr Leu Thr Asn Leu Gly Ala Pro Ala Ala Phe Lys Tyr Phe Asp Thr
1345                1350                1355                1360

Thr Ile Asp Arg Lys Arg Tyr Thr Ser Thr Lys Glu Val Leu Asp Ala
                1365                1370                1375

Thr Leu Ile His Gln Ser Ile Thr Gly Leu Tyr Glu Thr Arg Ile Asp
            1380                1385                1390

Leu Ser Gln Leu Gly Gly Asp Ser Arg Ala Asp Pro Lys Lys Lys Arg
        1395                1400                1405

Lys Val
    1410

<210> 6
<211> 1410
<212> AA (amino acid)
<213> artificial
<400> SEQUENCE 6 (SPCas9TAT):

Met His His His His His His His Gly Ser Pro Lys Lys Lys Arg Lys
  1               5                   10                  15

Val Met Asp Lys Lys Tyr Ser Ile Gly Leu Asp Ile Gly Thr Asn Ser
            20                  25                  30

Val Gly Trp Ala Val Ile Thr Asp Glu Tyr Lys Val Pro Ser Lys Lys
        35                  40                  45

Phe Lys Val Leu Gly Asn Thr Asp Arg His Ser Ile Lys Lys Asn Leu
    50                  55                  60

Ile Gly Ala Leu Leu Phe Asp Ser Gly Glu Thr Ala Glu Ala Thr Arg

65                  70                  75                  80

Leu Lys Arg Thr Ala Arg Arg Arg Tyr Thr Arg Arg Lys Asn Arg Ile
                85                  90                  95

Cys Tyr Leu Gln Glu Ile Phe Ser Asn Glu Met Ala Lys Val Asp Asp
            100                 105                 110

Ser Phe Phe His Arg Leu Glu Glu Ser Phe Leu Val Glu Glu Asp Lys
        115                 120                 125

Lys His Glu Arg His Pro Ile Phe Gly Asn Ile Val Asp Glu Val Ala
    130                 135                 140

Tyr His Glu Lys Tyr Pro Thr Ile Tyr His Leu Arg Lys Lys Leu Val
145                 150                 155                 160

Asp Ser Thr Asp Lys Ala Asp Leu Arg Leu Ile Tyr Leu Ala Leu Ala
                165                 170                 175

His Met Ile Lys Phe Arg Gly His Phe Leu Ile Glu Gly Asp Leu Asn
            180                 185                 190

Pro Asp Asn Ser Asp Val Asp Lys Leu Phe Ile Gln Leu Val Gln Thr
        195                 200                 205

Tyr Asn Gln Leu Phe Glu Glu Asn Pro Ile Asn Ala Ser Gly Val Asp
    210                 215                 220

Ala Lys Ala Ile Leu Ser Ala Arg Leu Ser Lys Ser Arg Arg Leu Glu
225                 230                 235                 240

Asn Leu Ile Ala Gln Leu Pro Gly Glu Lys Lys Asn Gly Leu Phe Gly
                245                 250                 255

Asn Leu Ile Ala Leu Ser Leu Gly Leu Thr Pro Asn Phe Lys Ser Asn
            260                 265                 270

Phe Asp Leu Ala Glu Asp Ala Lys Leu Gln Leu Ser Lys Asp Thr Tyr
        275                 280                 285

Asp Asp Asp Leu Asp Asn Leu Leu Ala Gln Ile Gly Asp Gln Tyr Ala
    290                 295                 300

Asp Leu Phe Leu Ala Ala Lys Asn Leu Ser Asp Ala Ile Leu Leu Ser
305                 310                 315                 320

Asp Ile Leu Arg Val Asn Thr Glu Ile Thr Lys Ala Pro Leu Ser Ala
                325                 330                 335

Ser Met Ile Lys Arg Tyr Asp Glu His His Gln Asp Leu Thr Leu Leu
            340                 345                 350

Lys Ala Leu Val Arg Gln Gln Leu Pro Glu Lys Tyr Lys Glu Ile Phe
        355                 360                 365

Phe Asp Gln Ser Lys Asn Gly Tyr Ala Gly Tyr Ile Asp Gly Gly Ala
    370                 375                 380

Ser Gln Glu Glu Phe Tyr Lys Phe Ile Lys Pro Ile Leu Glu Lys Met
385                 390                 395                 400

Asp Gly Thr Glu Glu Leu Leu Val Lys Leu Asn Arg Glu Asp Leu Leu

405                 410                 415

Arg Lys Gln Arg Thr Phe Asp Asn Gly Ser Ile Pro His Gln Ile His
            420                 425                 430

Leu Gly Glu Leu His Ala Ile Leu Arg Arg Gln Glu Asp Phe Tyr Pro
        435                 440                 445

Phe Leu Lys Asp Asn Arg Glu Lys Ile Glu Lys Ile Leu Thr Phe Arg
    450                 455                 460

Ile Pro Tyr Tyr Val Gly Pro Leu Ala Arg Gly Asn Ser Arg Phe Ala
465                 470                 475                 480

Trp Met Thr Arg Lys Ser Glu Glu Thr Ile Thr Pro Trp Asn Phe Glu
                485                 490                 495

Glu Val Val Asp Lys Gly Ala Ser Ala Gln Ser Phe Ile Glu Arg Met
            500                 505                 510

Thr Asn Phe Asp Lys Asn Leu Pro Asn Glu Lys Val Leu Pro Lys His
        515                 520                 525

Ser Leu Leu Tyr Glu Tyr Phe Thr Val Tyr Asn Glu Leu Thr Lys Val
    530                 535                 540

Lys Tyr Val Thr Glu Gly Met Arg Lys Pro Ala Phe Leu Ser Gly Glu
545                 550                 555                 560

Gln Lys Lys Ala Ile Val Asp Leu Leu Phe Lys Thr Asn Arg Lys Val
                565                 570                 575

Thr Val Lys Gln Leu Lys Glu Asp Tyr Phe Lys Lys Ile Glu Cys Phe
            580                 585                 590

Asp Ser Val Glu Ile Ser Gly Val Glu Asp Arg Phe Asn Ala Ser Leu
        595                 600                 605

Gly Thr Tyr His Asp Leu Leu Lys Ile Ile Lys Asp Lys Asp Phe Leu
    610                 615                 620

Asp Asn Glu Glu Asn Glu Asp Ile Leu Glu Asp Ile Val Leu Thr Leu
625                 630                 635                 640

Thr Leu Phe Glu Asp Arg Glu Met Ile Glu Glu Arg Leu Lys Thr Tyr
                645                 650                 655

Ala His Leu Phe Asp Asp Lys Val Met Lys Gln Leu Lys Arg Arg Arg
            660                 665                 670

Tyr Thr Gly Trp Gly Arg Leu Ser Arg Lys Leu Ile Asn Gly Ile Arg
        675                 680                 685

Asp Lys Gln Ser Gly Lys Thr Ile Leu Asp Phe Leu Lys Ser Asp Gly
    690                 695                 700

Phe Ala Asn Arg Asn Phe Met Gln Leu Ile His Asp Asp Ser Leu Thr
705                 710                 715                 720

Phe Lys Glu Asp Ile Gln Lys Ala Gln Val Ser Gly Gln Gly Asp Ser
                725                 730                 735

Leu His Glu His Ile Ala Asn Leu Ala Gly Ser Pro Ala Ile Lys Lys

740                 745                 750

Gly Ile Leu Gln Thr Val Lys Val Val Asp Glu Leu Val Lys Val Met
        755                 760                 765

Gly Arg His Lys Pro Glu Asn Ile Val Ile Glu Met Ala Arg Glu Asn
    770                 775                 780

Gln Thr Thr Gln Lys Gly Gln Lys Asn Ser Arg Glu Arg Met Lys Arg
785                 790                 795                 800

Ile Glu Glu Gly Ile Lys Glu Leu Gly Ser Gln Ile Leu Lys Glu His
                805                 810                 815

Pro Val Glu Asn Thr Gln Leu Gln Asn Glu Lys Leu Tyr Leu Tyr Tyr
            820                 825                 830

Leu Gln Asn Gly Arg Asp Met Tyr Val Asp Gln Glu Leu Asp Ile Asn
        835                 840                 845

Arg Leu Ser Asp Tyr Asp Val Asp His Ile Val Pro Gln Ser Phe Leu
    850                 855                 860

Lys Asp Asp Ser Ile Asp Asn Lys Val Leu Thr Arg Ser Asp Lys Asn
865                 870                 875                 880

Arg Gly Lys Ser Asp Asn Val Pro Ser Glu Glu Val Val Lys Lys Met
                885                 890                 895

Lys Asn Tyr Trp Arg Gln Leu Leu Asn Ala Lys Leu Ile Thr Gln Arg
            900                 905                 910

Lys Phe Asp Asn Leu Thr Lys Ala Glu Arg Gly Gly Leu Ser Glu Leu
        915                 920                 925

Asp Lys Ala Gly Phe Ile Lys Arg Gln Leu Val Glu Thr Arg Gln Ile
    930                 935                 940

Thr Lys His Val Ala Gln Ile Leu Asp Ser Arg Met Asn Thr Lys Tyr
945                 950                 955                 960

Asp Glu Asn Asp Lys Leu Ile Arg Glu Val Lys Val Ile Thr Leu Lys
                965                 970                 975

Ser Lys Leu Val Ser Asp Phe Arg Lys Asp Phe Gln Phe Tyr Lys Val
            980                 985                 990

Arg Glu Ile Asn Asn Tyr His His Ala His Asp Ala Tyr Leu Asn Ala
        995                 1000                1005

Val Val Gly Thr Ala Leu Ile Lys Lys Tyr Pro Lys Leu Glu Ser Glu
    1010                1015                1020

Phe Val Tyr Gly Asp Tyr Lys Val Tyr Asp Val Arg Lys Met Ile Ala
1025                1030                1035                1040

Lys Ser Glu Gln Glu Ile Gly Lys Ala Thr Ala Lys Tyr Phe Phe Tyr
                1045                1050                1055

Ser Asn Ile Met Asn Phe Phe Lys Thr Glu Ile Thr Leu Ala Asn Gly
            1060                1065                1070

Glu Ile Arg Lys Arg Pro Leu Ile Glu Thr Asn Gly Glu Thr Gly Glu

1075                1080                1085

Ile Val Trp Asp Lys Gly Arg Asp Phe Ala Thr Val Arg Lys Val Leu
    1090                1095                1100

Ser Met Pro Gln Val Asn Ile Val Lys Lys Thr Glu Val Gln Thr Gly
1105                1110                1115                1120

Gly Phe Ser Lys Glu Ser Ile Leu Pro Lys Arg Asn Ser Asp Lys Leu
                1125                1130                1135

Ile Ala Arg Lys Lys Asp Trp Asp Pro Lys Lys Tyr Gly Gly Phe Asp
            1140                1145                1150

Ser Pro Thr Val Ala Tyr Ser Val Leu Val Val Ala Lys Val Glu Lys
        1155                1160                1165

Gly Lys Ser Lys Lys Leu Lys Ser Val Lys Glu Leu Leu Gly Ile Thr
    1170                1175                1180

Ile Met Glu Arg Ser Ser Phe Glu Lys Asn Pro Ile Asp Phe Leu Glu
1185                1190                1195                1200

Ala Lys Gly Tyr Lys Glu Val Lys Lys Asp Leu Ile Ile Lys Leu Pro
                1205                1210                1215

Lys Tyr Ser Leu Phe Glu Leu Glu Asn Gly Arg Lys Arg Met Leu Ala
            1220                1225                1230

Ser Ala Gly Glu Leu Gln Lys Gly Asn Glu Leu Ala Leu Pro Ser Lys
        1235                1240                1245

Tyr Val Asn Phe Leu Tyr Leu Ala Ser His Tyr Glu Lys Leu Lys Gly
    1250                1255                1260

Ser Pro Glu Asp Asn Glu Gln Lys Gln Leu Phe Val Glu Gln His Lys
1265                1270                1275                1280

His Tyr Leu Asp Glu Ile Ile Glu Gln Ile Ser Glu Phe Ser Lys Arg
                1285                1290                1295

Val Ile Leu Ala Asp Ala Asn Leu Asp Lys Val Leu Ser Ala Tyr Asn
            1300                1305                1310

Lys His Arg Asp Lys Pro Ile Arg Glu Gln Ala Glu Asn Ile Ile His
        1315                1320                1325

Leu Phe Thr Leu Thr Asn Leu Gly Ala Pro Ala Ala Phe Lys Tyr Phe
    1330                1335                1340

Asp Thr Thr Ile Asp Arg Lys Arg Tyr Thr Ser Thr Lys Glu Val Leu
1345                1350                1355                1360

Asp Ala Thr Leu Ile His Gln Ser Ile Thr Gly Leu Tyr Glu Thr Arg
                1365                1370                1375

Ile Asp Leu Ser Gln Leu Gly Gly Asp Ser Arg Ala Asp Pro Lys Lys
            1380                1385                1390

Lys Arg Lys Val Gly Ser Gly Arg Lys Lys Arg Arg Gln Arg Arg Arg
        1395                1400                1405

Pro Gln

    1410

<210> 7
<211> 1424
<212> AA (amino acid)
<213> artificial
<400> SEQUENCE 7 (TATSPCas9TAT):

Met His Gly Arg Lys Lys Arg Arg Gln Arg Arg Arg Pro Gln Gly Ser
1               5                   10                  15

His His His His His His Gly Ser Pro Lys Lys Lys Arg Lys Val Met
            20                  25                  30

Asp Lys Lys Tyr Ser Ile Gly Leu Asp Ile Gly Thr Asn Ser Val Gly
        35                  40                  45

Trp Ala Val Ile Thr Asp Glu Tyr Lys Val Pro Ser Lys Lys Phe Lys
    50                  55                  60

Val Leu Gly Asn Thr Asp Arg His Ser Ile Lys Lys Asn Leu Ile Gly
65                  70                  75                  80

Ala Leu Leu Phe Asp Ser Gly Glu Thr Ala Glu Ala Thr Arg Leu Lys
                85                  90                  95

Arg Thr Ala Arg Arg Arg Tyr Thr Arg Arg Lys Asn Arg Ile Cys Tyr
            100                 105                 110

Leu Gln Glu Ile Phe Ser Asn Glu Met Ala Lys Val Asp Asp Ser Phe
        115                 120                 125

Phe His Arg Leu Glu Glu Ser Phe Leu Val Glu Glu Asp Lys Lys His
    130                 135                 140

Glu Arg His Pro Ile Phe Gly Asn Ile Val Asp Glu Val Ala Tyr His
145                 150                 155                 160

Glu Lys Tyr Pro Thr Ile Tyr His Leu Arg Lys Lys Leu Val Asp Ser
                165                 170                 175

Thr Asp Lys Ala Asp Leu Arg Leu Ile Tyr Leu Ala Leu Ala His Met
            180                 185                 190

Ile Lys Phe Arg Gly His Phe Leu Ile Glu Gly Asp Leu Asn Pro Asp
        195                 200                 205

Asn Ser Asp Val Asp Lys Leu Phe Ile Gln Leu Val Gln Thr Tyr Asn
    210                 215                 220

Gln Leu Phe Glu Glu Asn Pro Ile Asn Ala Ser Gly Val Asp Ala Lys
225                 230                 235                 240

Ala Ile Leu Ser Ala Arg Leu Ser Lys Ser Arg Arg Leu Glu Asn Leu
                245                 250                 255

Ile Ala Gln Leu Pro Gly Glu Lys Lys Asn Gly Leu Phe Gly Asn Leu
            260                 265                 270

Ile Ala Leu Ser Leu Gly Leu Thr Pro Asn Phe Lys Ser Asn Phe Asp
        275                 280                 285

Leu Ala Glu Asp Ala Lys Leu Gln Leu Ser Lys Asp Thr Tyr Asp Asp
    290                 295                 300

Asp Leu Asp Asn Leu Leu Ala Gln Ile Gly Asp Gln Tyr Ala Asp Leu
305                 310                 315                 320

Phe Leu Ala Ala Lys Asn Leu Ser Asp Ala Ile Leu Leu Ser Asp Ile
                325                 330                 335

Leu Arg Val Asn Thr Glu Ile Thr Lys Ala Pro Leu Ser Ala Ser Met
            340                 345                 350

Ile Lys Arg Tyr Asp Glu His His Gln Asp Leu Thr Leu Leu Lys Ala
        355                 360                 365

Leu Val Arg Gln Gln Leu Pro Glu Lys Tyr Lys Glu Ile Phe Phe Asp
    370                 375                 380

Gln Ser Lys Asn Gly Tyr Ala Gly Tyr Ile Asp Gly Gly Ala Ser Gln
385                 390                 395                 400

Glu Glu Phe Tyr Lys Phe Ile Lys Pro Ile Leu Glu Lys Met Asp Gly
                405                 410                 415

Thr Glu Glu Leu Leu Val Lys Leu Asn Arg Glu Asp Leu Leu Arg Lys
            420                 425                 430

Gln Arg Thr Phe Asp Asn Gly Ser Ile Pro His Gln Ile His Leu Gly
        435                 440                 445

Glu Leu His Ala Ile Leu Arg Arg Gln Glu Asp Phe Tyr Pro Phe Leu
    450                 455                 460

Lys Asp Asn Arg Glu Lys Ile Glu Lys Ile Leu Thr Phe Arg Ile Pro
465                 470                 475                 480

Tyr Tyr Val Gly Pro Leu Ala Arg Gly Asn Ser Arg Phe Ala Trp Met
                485                 490                 495

Thr Arg Lys Ser Glu Glu Thr Ile Thr Pro Trp Asn Phe Glu Glu Val
            500                 505                 510

Val Asp Lys Gly Ala Ser Ala Gln Ser Phe Ile Glu Arg Met Thr Asn
        515                 520                 525

Phe Asp Lys Asn Leu Pro Asn Glu Lys Val Leu Pro Lys His Ser Leu
    530                 535                 540

Leu Tyr Glu Tyr Phe Thr Val Tyr Asn Glu Leu Thr Lys Val Lys Tyr
545                 550                 555                 560

Val Thr Glu Gly Met Arg Lys Pro Ala Phe Leu Ser Gly Glu Gln Lys
                565                 570                 575

Lys Ala Ile Val Asp Leu Leu Phe Lys Thr Asn Arg Lys Val Thr Val
            580                 585                 590

Lys Gln Leu Lys Glu Asp Tyr Phe Lys Lys Ile Glu Cys Phe Asp Ser
        595                 600                 605

Val Glu Ile Ser Gly Val Glu Asp Arg Phe Asn Ala Ser Leu Gly Thr
    610                 615                 620

Tyr His Asp Leu Leu Lys Ile Ile Lys Asp Lys Asp Phe Leu Asp Asn
625                 630                 635                 640

Glu Glu Asn Glu Asp Ile Leu Glu Asp Ile Val Leu Thr Leu Thr Leu
                645                 650                 655

Phe Glu Asp Arg Glu Met Ile Glu Glu Arg Leu Lys Thr Tyr Ala His
            660                 665                 670

Leu Phe Asp Asp Lys Val Met Lys Gln Leu Lys Arg Arg Arg Tyr Thr
        675                 680                 685

Gly Trp Gly Arg Leu Ser Arg Lys Leu Ile Asn Gly Ile Arg Asp Lys
    690                 695                 700

Gln Ser Gly Lys Thr Ile Leu Asp Phe Leu Lys Ser Asp Gly Phe Ala
705                 710                 715                 720

Asn Arg Asn Phe Met Gln Leu Ile His Asp Asp Ser Leu Thr Phe Lys
                725                 730                 735

Glu Asp Ile Gln Lys Ala Gln Val Ser Gly Gln Gly Asp Ser Leu His
            740                 745                 750

Glu His Ile Ala Asn Leu Ala Gly Ser Pro Ala Ile Lys Lys Gly Ile
        755                 760                 765

Leu Gln Thr Val Lys Val Val Asp Glu Leu Val Lys Val Met Gly Arg
    770                 775                 780

His Lys Pro Glu Asn Ile Val Ile Glu Met Ala Arg Glu Asn Gln Thr
785                 790                 795                 800

Thr Gln Lys Gly Gln Lys Asn Ser Arg Glu Arg Met Lys Arg Ile Glu
                805                 810                 815

Glu Gly Ile Lys Glu Leu Gly Ser Gln Ile Leu Lys Glu His Pro Val
            820                 825                 830

Glu Asn Thr Gln Leu Gln Asn Glu Lys Leu Tyr Leu Tyr Tyr Leu Gln
        835                 840                 845

Asn Gly Arg Asp Met Tyr Val Asp Gln Glu Leu Asp Ile Asn Arg Leu
    850                 855                 860

Ser Asp Tyr Asp Val Asp His Ile Val Pro Gln Ser Phe Leu Lys Asp
865                 870                 875                 880

Asp Ser Ile Asp Asn Lys Val Leu Thr Arg Ser Asp Lys Asn Arg Gly
                885                 890                 895

Lys Ser Asp Asn Val Pro Ser Glu Glu Val Val Lys Lys Met Lys Asn
            900                 905                 910

Tyr Trp Arg Gln Leu Leu Asn Ala Lys Leu Ile Thr Gln Arg Lys Phe
        915                 920                 925

Asp Asn Leu Thr Lys Ala Glu Arg Gly Gly Leu Ser Glu Leu Asp Lys
    930                 935                 940

Ala Gly Phe Ile Lys Arg Gln Leu Val Glu Thr Arg Gln Ile Thr Lys
945                 950                 955                 960

His Val Ala Gln Ile Leu Asp Ser Arg Met Asn Thr Lys Tyr Asp Glu
                965                 970                 975

Asn Asp Lys Leu Ile Arg Glu Val Lys Val Ile Thr Leu Lys Ser Lys
            980                 985                 990

Leu Val Ser Asp Phe Arg Lys Asp Phe Gln Phe Tyr Lys Val Arg Glu
        995                 1000                1005

Ile Asn Asn Tyr His His Ala His Asp Ala Tyr Leu Asn Ala Val Val
    1010                1015                1020

Gly Thr Ala Leu Ile Lys Lys Tyr Pro Lys Leu Glu Ser Glu Phe Val
1025                1030                1035                1040

Tyr Gly Asp Tyr Lys Val Tyr Asp Val Arg Lys Met Ile Ala Lys Ser
                1045                1050                1055

Glu Gln Glu Ile Gly Lys Ala Thr Ala Lys Tyr Phe Phe Tyr Ser Asn
            1060                1065                1070

Ile Met Asn Phe Phe Lys Thr Glu Ile Thr Leu Ala Asn Gly Glu Ile
        1075                1080                1085

Arg Lys Arg Pro Leu Ile Glu Thr Asn Gly Glu Thr Gly Glu Ile Val
    1090                1095                1100

Trp Asp Lys Gly Arg Asp Phe Ala Thr Val Arg Lys Val Leu Ser Met
1105                1110                1115                1120

Pro Gln Val Asn Ile Val Lys Lys Thr Glu Val Gln Thr Gly Gly Phe
                1125                1130                1135

Ser Lys Glu Ser Ile Leu Pro Lys Arg Asn Ser Asp Lys Leu Ile Ala
            1140                1145                1150

Arg Lys Lys Asp Trp Asp Pro Lys Lys Tyr Gly Gly Phe Asp Ser Pro
        1155                1160                1165

Thr Val Ala Tyr Ser Val Leu Val Val Ala Lys Val Glu Lys Gly Lys
    1170                1175                1180

Ser Lys Lys Leu Lys Ser Val Lys Glu Leu Leu Gly Ile Thr Ile Met
1185                1190                1195                1200

Glu Arg Ser Ser Phe Glu Lys Asn Pro Ile Asp Phe Leu Glu Ala Lys
                1205                1210                1215

Gly Tyr Lys Glu Val Lys Lys Asp Leu Ile Ile Lys Leu Pro Lys Tyr
            1220                1225                1230

Ser Leu Phe Glu Leu Glu Asn Gly Arg Lys Arg Met Leu Ala Ser Ala
        1235                1240                1245

Gly Glu Leu Gln Lys Gly Asn Glu Leu Ala Leu Pro Ser Lys Tyr Val
    1250                1255                1260

Asn Phe Leu Tyr Leu Ala Ser His Tyr Glu Lys Leu Lys Gly Ser Pro
1265                1270                1275                1280

Glu Asp Asn Glu Gln Lys Gln Leu Phe Val Glu Gln His Lys His Tyr
                1285                1290                1295

Leu Asp Glu Ile Ile Glu Gln Ile Ser Glu Phe Ser Lys Arg Val Ile
            1300                1305                1310

Leu Ala Asp Ala Asn Leu Asp Lys Val Leu Ser Ala Tyr Asn Lys His
        1315                1320                1325

Arg Asp Lys Pro Ile Arg Glu Gln Ala Glu Asn Ile Ile His Leu Phe
    1330                1335                1340

Thr Leu Thr Asn Leu Gly Ala Pro Ala Ala Phe Lys Tyr Phe Asp Thr
1345                1350                1355                1360

Thr Ile Asp Arg Lys Arg Tyr Thr Ser Thr Lys Glu Val Leu Asp Ala
                1365                1370                1375

Thr Leu Ile His Gln Ser Ile Thr Gly Leu Tyr Glu Thr Arg Ile Asp
            1380                1385                1390

Leu Ser Gln Leu Gly Gly Asp Ser Arg Ala Asp Pro Lys Lys Lys Arg
        1395                1400                1405

Lys Val Gly Ser Gly Arg Lys Lys Arg Arg Gln Arg Arg Arg Pro Gln
    1410                1415                1420


<210> 8
<211> 1410
<212> AA (amino acid)
<213> artificial
<400> SEQUENCE 8 (SPCas9D10ATAT):
Met His His His His His His His Gly Ser Pro Lys Lys Lys Arg Lys
1               5                   10                  15

Val Met Asp Lys Lys Tyr Ser Ile Gly Leu Asp Ile Gly Thr Asn Ser
            20                  25                  30

Val Gly Trp Ala Val Ile Thr Asp Glu Tyr Lys Val Pro Ser Lys Lys
        35                  40                  45

Phe Lys Val Leu Gly Asn Thr Asp Arg His Ser Ile Lys Lys Asn Leu
    50                  55                  60

Ile Gly Ala Leu Leu Phe Asp Ser Gly Glu Thr Ala Glu Ala Thr Arg
65                  70                  75                  80

Leu Lys Arg Thr Ala Arg Arg Arg Tyr Thr Arg Arg Lys Asn Arg Ile
                85                  90                  95

Cys Tyr Leu Gln Glu Ile Phe Ser Asn Glu Met Ala Lys Val Asp Asp
            100                 105                 110

Ser Phe Phe His Arg Leu Glu Glu Ser Phe Leu Val Glu Glu Asp Lys
        115                 120                 125

Lys His Glu Arg His Pro Ile Phe Gly Asn Ile Val Asp Glu Val Ala
    130                 135                 140

Tyr His Glu Lys Tyr Pro Thr Ile Tyr His Leu Arg Lys Lys Leu Val
145                 150                 155                 160

Asp Ser Thr Asp Lys Ala Asp Leu Arg Leu Ile Tyr Leu Ala Leu Ala
                165                 170                 175

His Met Ile Lys Phe Arg Gly His Phe Leu Ile Glu Gly Asp Leu Asn
            180                 185                 190

Pro Asp Asn Ser Asp Val Asp Lys Leu Phe Ile Gln Leu Val Gln Thr
        195                 200                 205

Tyr Asn Gln Leu Phe Glu Glu Asn Pro Ile Asn Ala Ser Gly Val Asp
    210                 215                 220

Ala Lys Ala Ile Leu Ser Ala Arg Leu Ser Lys Ser Arg Arg Leu Glu
225                 230                 235                 240

Asn Leu Ile Ala Gln Leu Pro Gly Glu Lys Lys Asn Gly Leu Phe Gly
                245                 250                 255

Asn Leu Ile Ala Leu Ser Leu Gly Leu Thr Pro Asn Phe Lys Ser Asn
            260                 265                 270

Phe Asp Leu Ala Glu Asp Ala Lys Leu Gln Leu Ser Lys Asp Thr Tyr
        275                 280                 285

Asp Asp Asp Leu Asp Asn Leu Leu Ala Gln Ile Gly Asp Gln Tyr Ala
    290                 295                 300

Asp Leu Phe Leu Ala Ala Lys Asn Leu Ser Asp Ala Ile Leu Leu Ser
305                 310                 315                 320

Asp Ile Leu Arg Val Asn Thr Glu Ile Thr Lys Ala Pro Leu Ser Ala
                325                 330                 335

Ser Met Ile Lys Arg Tyr Asp Glu His His Gln Asp Leu Thr Leu Leu
            340                 345                 350

Lys Ala Leu Val Arg Gln Gln Leu Pro Glu Lys Tyr Lys Glu Ile Phe
        355                 360                 365

Phe Asp Gln Ser Lys Asn Gly Tyr Ala Gly Tyr Ile Asp Gly Gly Ala
    370                 375                 380

Ser Gln Glu Glu Phe Tyr Lys Phe Ile Lys Pro Ile Leu Glu Lys Met
385                 390                 395                 400

Asp Gly Thr Glu Glu Leu Leu Val Lys Leu Asn Arg Glu Asp Leu Leu
                405                 410                 415

Arg Lys Gln Arg Thr Phe Asp Asn Gly Ser Ile Pro His Gln Ile His
            420                 425                 430

Leu Gly Glu Leu His Ala Ile Leu Arg Arg Gln Glu Asp Phe Tyr Pro
        435                 440                 445

Phe Leu Lys Asp Asn Arg Glu Lys Ile Glu Lys Ile Leu Thr Phe Arg
    450                 455                 460

Ile Pro Tyr Tyr Val Gly Pro Leu Ala Arg Gly Asn Ser Arg Phe Ala
465                 470                 475                 480

Trp Met Thr Arg Lys Ser Glu Glu Thr Ile Thr Pro Trp Asn Phe Glu
                485                 490                 495

Glu Val Val Asp Lys Gly Ala Ser Ala Gln Ser Phe Ile Glu Arg Met
            500                 505                 510

Thr Asn Phe Asp Lys Asn Leu Pro Asn Glu Lys Val Leu Pro Lys His
        515                 520                 525

Ser Leu Leu Tyr Glu Tyr Phe Thr Val Tyr Asn Glu Leu Thr Lys Val
    530                 535                 540

Lys Tyr Val Thr Glu Gly Met Arg Lys Pro Ala Phe Leu Ser Gly Glu
545                 550                 555                 560

Gln Lys Lys Ala Ile Val Asp Leu Leu Phe Lys Thr Asn Arg Lys Val
                565                 570                 575

Thr Val Lys Gln Leu Lys Glu Asp Tyr Phe Lys Lys Ile Glu Cys Phe
            580                 585                 590

Asp Ser Val Glu Ile Ser Gly Val Glu Asp Arg Phe Asn Ala Ser Leu
        595                 600                 605

Gly Thr Tyr His Asp Leu Leu Lys Ile Ile Lys Asp Lys Asp Phe Leu
    610                 615                 620

Asp Asn Glu Glu Asn Glu Asp Ile Leu Glu Asp Ile Val Leu Thr Leu
625                 630                 635                 640

Thr Leu Phe Glu Asp Arg Glu Met Ile Glu Glu Arg Leu Lys Thr Tyr
                645                 650                 655

Ala His Leu Phe Asp Asp Lys Val Met Lys Gln Leu Lys Arg Arg Arg
            660                 665                 670

Tyr Thr Gly Trp Gly Arg Leu Ser Arg Lys Leu Ile Asn Gly Ile Arg
        675                 680                 685

Asp Lys Gln Ser Gly Lys Thr Ile Leu Asp Phe Leu Lys Ser Asp Gly
    690                 695                 700

Phe Ala Asn Arg Asn Phe Met Gln Leu Ile His Asp Asp Ser Leu Thr
705                 710                 715                 720

Phe Lys Glu Asp Ile Gln Lys Ala Gln Val Ser Gly Gln Gly Asp Ser
                725                 730                 735

Leu His Glu His Ile Ala Asn Leu Ala Gly Ser Pro Ala Ile Lys Lys
            740                 745                 750

Gly Ile Leu Gln Thr Val Lys Val Val Asp Glu Leu Val Lys Val Met
        755                 760                 765

Gly Arg His Lys Pro Glu Asn Ile Val Ile Glu Met Ala Arg Glu Asn
    770                 775                 780

Gln Thr Thr Gln Lys Gly Gln Lys Asn Ser Arg Glu Arg Met Lys Arg
785                 790                 795                 800

Ile Glu Glu Gly Ile Lys Glu Leu Gly Ser Gln Ile Leu Lys Glu His
                805                 810                 815

Pro Val Glu Asn Thr Gln Leu Gln Asn Glu Lys Leu Tyr Leu Tyr Tyr
            820                 825                 830

Leu Gln Asn Gly Arg Asp Met Tyr Val Asp Gln Glu Leu Asp Ile Asn
        835                 840                 845

Arg Leu Ser Asp Tyr Asp Val Asp His Ile Val Pro Gln Ser Phe Leu
    850                 855                 860

Lys Asp Asp Ser Ile Asp Asn Lys Val Leu Thr Arg Ser Asp Lys Asn
865                 870                 875                 880

Arg Gly Lys Ser Asp Asn Val Pro Ser Glu Glu Val Val Lys Lys Met
                885                 890                 895

Lys Asn Tyr Trp Arg Gln Leu Leu Asn Ala Lys Leu Ile Thr Gln Arg
            900                 905                 910

Lys Phe Asp Asn Leu Thr Lys Ala Glu Arg Gly Gly Leu Ser Glu Leu
        915                 920                 925

Asp Lys Ala Gly Phe Ile Lys Arg Gln Leu Val Glu Thr Arg Gln Ile
    930                 935                 940

Thr Lys His Val Ala Gln Ile Leu Asp Ser Arg Met Asn Thr Lys Tyr
945                 950                 955                 960

Asp Glu Asn Asp Lys Leu Ile Arg Glu Val Lys Val Ile Thr Leu Lys
                965                 970                 975

Ser Lys Leu Val Ser Asp Phe Arg Lys Asp Phe Gln Phe Tyr Lys Val
            980                 985                 990

Arg Glu Ile Asn Asn Tyr His His Ala His Asp Ala Tyr Leu Asn Ala
        995                 1000                1005

Val Val Gly Thr Ala Leu Ile Lys Lys Tyr Pro Lys Leu Glu Ser Glu
    1010                1015                1020

Phe Val Tyr Gly Asp Tyr Lys Val Tyr Asp Val Arg Lys Met Ile Ala
1025                1030                1035                1040

Lys Ser Glu Gln Glu Ile Gly Lys Ala Thr Ala Lys Tyr Phe Phe Tyr
                1045                1050                1055

Ser Asn Ile Met Asn Phe Phe Lys Thr Glu Ile Thr Leu Ala Asn Gly
            1060                1065                1070

Glu Ile Arg Lys Arg Pro Leu Ile Glu Thr Asn Gly Glu Thr Gly Glu
        1075                1080                1085

Ile Val Trp Asp Lys Gly Arg Asp Phe Ala Thr Val Arg Lys Val Leu
    1090                1095                1100

Ser Met Pro Gln Val Asn Ile Val Lys Lys Thr Glu Val Gln Thr Gly
1105                1110                1115                1120

Gly Phe Ser Lys Glu Ser Ile Leu Pro Lys Arg Asn Ser Asp Lys Leu
                1125                1130                1135

Ile Ala Arg Lys Lys Asp Trp Asp Pro Lys Lys Tyr Gly Gly Phe Asp
            1140                1145                1150

Ser Pro Thr Val Ala Tyr Ser Val Leu Val Val Ala Lys Val Glu Lys
        1155                1160                1165

Gly Lys Ser Lys Lys Leu Lys Ser Val Lys Glu Leu Leu Gly Ile Thr
    1170                1175                1180

Ile Met Glu Arg Ser Ser Phe Glu Lys Asn Pro Ile Asp Phe Leu Glu
1185                1190                1195                1200

Ala Lys Gly Tyr Lys Glu Val Lys Lys Asp Leu Ile Ile Lys Leu Pro
                1205                1210                1215

Lys Tyr Ser Leu Phe Glu Leu Glu Asn Gly Arg Lys Arg Met Leu Ala
            1220                1225                1230

Ser Ala Gly Glu Leu Gln Lys Gly Asn Glu Leu Ala Leu Pro Ser Lys
        1235                1240                1245

Tyr Val Asn Phe Leu Tyr Leu Ala Ser His Tyr Glu Lys Leu Lys Gly
    1250                1255                1260

Ser Pro Glu Asp Asn Glu Gln Lys Gln Leu Phe Val Glu Gln His Lys
1265                1270                1275                1280

His Tyr Leu Asp Glu Ile Ile Glu Gln Ile Ser Glu Phe Ser Lys Arg
                1285                1290                1295

Val Ile Leu Ala Asp Ala Asn Leu Asp Lys Val Leu Ser Ala Tyr Asn
            1300                1305                1310

Lys His Arg Asp Lys Pro Ile Arg Glu Gln Ala Glu Asn Ile Ile His
        1315                1320                1325

Leu Phe Thr Leu Thr Asn Leu Gly Ala Pro Ala Ala Phe Lys Tyr Phe
    1330                1335                1340

Asp Thr Thr Ile Asp Arg Lys Arg Tyr Thr Ser Thr Lys Glu Val Leu
1345                1350                1355                1360

Asp Ala Thr Leu Ile His Gln Ser Ile Thr Gly Leu Tyr Glu Thr Arg
                1365                1370                1375

Ile Asp Leu Ser Gln Leu Gly Gly Asp Ser Arg Ala Asp Pro Lys Lys
            1380                1385                1390

Lys Arg Lys Val Gly Ser Gly Arg Lys Lys Arg Arg Gln Arg Arg Arg
        1395                1400                1405

Pro Gln
    1410

<210> 9
<211> 4191
<212> DNA
<213> artificial
<400> SEQUENCE 9 (SPCas9):

atg cat cat cac cac cac cat cac ggc agc ccc aag aag aag agg aag        48
Met His His His His His His His Gly Ser Pro Lys Lys Lys Arg Lys
  1               5                  10                  15

gtg atg gac aag aag tac tcc att ggg ctc gat atc ggc aca aac agc        96
Val Met Asp Lys Lys Tyr Ser Ile Gly Leu Asp Ile Gly Thr Asn Ser
                20                  25                  30

gtc ggc tgg gcc gtc att acg gac gag tac aag gtg ccg agc aaa aaa       144
Val Gly Trp Ala Val Ile Thr Asp Glu Tyr Lys Val Pro Ser Lys Lys
            35                  40                  45

ttc aaa gtt ctg ggc aat acc gat cgc cac agc ata aag aag aac ctc 192
Phe Lys Val Leu Gly Asn Thr Asp Arg His Ser Ile Lys Lys Asn Leu
    50                  55                  60

att ggc gcc ctc ctg ttc gac tcc ggg gag acg gcc gaa gcc acg cgg 240
Ile Gly Ala Leu Leu Phe Asp Ser Gly Glu Thr Ala Glu Ala Thr Arg
65                  70                  75                  80

ctc aaa aga aca gca cgg cgc aga tat acc cgc aga aag aat cgg atc 288
Leu Lys Arg Thr Ala Arg Arg Arg Tyr Thr Arg Arg Lys Asn Arg Ile
                85                  90                  95

tgc tac ctg cag gag atc ttt agt aat gag atg gct aag gtg gat gac 336
Cys Tyr Leu Gln Glu Ile Phe Ser Asn Glu Met Ala Lys Val Asp Asp
            100                 105                 110

tct ttc ttc cat agg ctg gag gag tcc ttt ttg gtg gag gag gat aaa 384
Ser Phe Phe His Arg Leu Glu Glu Ser Phe Leu Val Glu Glu Asp Lys
        115                 120                 125

aag cac gag cgc cac cca atc ttt ggc aat atc gtg gac gag gtg gcg 432
Lys His Glu Arg His Pro Ile Phe Gly Asn Ile Val Asp Glu Val Ala
    130                 135                 140

tac cat gaa aag tac cca acc ata tat cat ctg agg aag aag ctt gta 480
Tyr His Glu Lys Tyr Pro Thr Ile Tyr His Leu Arg Lys Lys Leu Val
145                 150                 155                 160

gac agt act gat aag gct gac ttg cgg ttg atc tat ctc gcg ctg gcg 528
Asp Ser Thr Asp Lys Ala Asp Leu Arg Leu Ile Tyr Leu Ala Leu Ala
                165                 170                 175

cat atg atc aaa ttt cgg gga cac ttc ctc atc gag ggg gac ctg aac 576
His Met Ile Lys Phe Arg Gly His Phe Leu Ile Glu Gly Asp Leu Asn
            180                 185                 190

cca gac aac agc gat gtc gac aaa ctc ttt atc caa ctg gtt cag act 624
Pro Asp Asn Ser Asp Val Asp Lys Leu Phe Ile Gln Leu Val Gln Thr
        195                 200                 205

tac aat cag ctt ttc gaa gag aac ccg atc aac gca tcc gga gtt gac 672
Tyr Asn Gln Leu Phe Glu Glu Asn Pro Ile Asn Ala Ser Gly Val Asp
    210                 215                 220

gcc aaa gca atc ctg agc gct agg ctg tcc aaa tcc cgg cgg ctc gaa 720
Ala Lys Ala Ile Leu Ser Ala Arg Leu Ser Lys Ser Arg Arg Leu Glu
225                 230                 235                 240

aac ctc atc gca cag ctc cct ggg gag aag aag aac ggc ctg ttt ggt 768
Asn Leu Ile Ala Gln Leu Pro Gly Glu Lys Lys Asn Gly Leu Phe Gly
                245                 250                 255

aat ctt atc gcc ctg tca ctc ggg ctg acc ccc aac ttt aaa tct aac 816
Asn Leu Ile Ala Leu Ser Leu Gly Leu Thr Pro Asn Phe Lys Ser Asn
            260                 265                 270

ttc gac ctg gcc gaa gat gcc aag ctt caa ctg agc aaa gac acc tac 864
Phe Asp Leu Ala Glu Asp Ala Lys Leu Gln Leu Ser Lys Asp Thr Tyr
        275                 280                 285

gat gat gat ctc gac aat ctg ctg gcc cag atc ggc gac cag tac gca 912
Asp Asp Asp Leu Asp Asn Leu Leu Ala Gln Ile Gly Asp Gln Tyr Ala

290                 295                 300

gac ctt ttt ttg gcg gca aag aac ctg tca gac gcc att ctg ctg agt          960
Asp Leu Phe Leu Ala Ala Lys Asn Leu Ser Asp Ala Ile Leu Leu Ser
305                 310                 315                 320

gat att ctg cga gtg aac acg gag atc acc aaa gct ccg ctg agc gct          1008
Asp Ile Leu Arg Val Asn Thr Glu Ile Thr Lys Ala Pro Leu Ser Ala
                325                 330                 335

agt atg atc aag cgc tat gat gag cac cac caa gac ttg act ttg ctg          1056
Ser Met Ile Lys Arg Tyr Asp Glu His His Gln Asp Leu Thr Leu Leu
            340                 345                 350

aag gcc ctt gtc aga cag caa ctg cct gag aag tac aag gaa att ttc          1104
Lys Ala Leu Val Arg Gln Gln Leu Pro Glu Lys Tyr Lys Glu Ile Phe
        355                 360                 365

ttc gat cag tct aaa aat ggc tac gcc gga tac att gac ggc gga gca          1152
Phe Asp Gln Ser Lys Asn Gly Tyr Ala Gly Tyr Ile Asp Gly Gly Ala
    370                 375                 380

agc cag gag gaa ttt tac aaa ttt att aag ccc atc ttg gaa aaa atg          1200
Ser Gln Glu Glu Phe Tyr Lys Phe Ile Lys Pro Ile Leu Glu Lys Met
385                 390                 395                 400

gac ggc acc gag gag ctg ctg gta aag ctt aac aga gaa gat ctg ttg          1248
Asp Gly Thr Glu Glu Leu Leu Val Lys Leu Asn Arg Glu Asp Leu Leu
                405                 410                 415

cgc aaa cag cgc act ttc gac aat gga agc atc ccc cac cag att cac          1296
Arg Lys Gln Arg Thr Phe Asp Asn Gly Ser Ile Pro His Gln Ile His
            420                 425                 430

ctg ggc gaa ctg cac gct atc ctc agg cgg caa gag gat ttc tac ccc          1344
Leu Gly Glu Leu His Ala Ile Leu Arg Arg Gln Glu Asp Phe Tyr Pro
        435                 440                 445

ttt ttg aaa gat aac agg gaa aag att gag aaa atc ctc aca ttt cgg          1392
Phe Leu Lys Asp Asn Arg Glu Lys Ile Glu Lys Ile Leu Thr Phe Arg
    450                 455                 460

ata ccc tac tat gta ggc ccc ctc gcc cgg gga aat tcc aga ttc gcg          1440
Ile Pro Tyr Tyr Val Gly Pro Leu Ala Arg Gly Asn Ser Arg Phe Ala
465                 470                 475                 480

tgg atg act cgc aaa tca gaa gag acc atc act ccc tgg aac ttc gag          1488
Trp Met Thr Arg Lys Ser Glu Glu Thr Ile Thr Pro Trp Asn Phe Glu
                485                 490                 495

gaa gtc gtg gat aag ggg gcc tct gcc cag tcc ttc atc gaa agg atg          1536
Glu Val Val Asp Lys Gly Ala Ser Ala Gln Ser Phe Ile Glu Arg Met
            500                 505                 510

act aac ttt gat aaa aat ctg cct aac gaa aag gtg ctt cct aaa cac          1584
Thr Asn Phe Asp Lys Asn Leu Pro Asn Glu Lys Val Leu Pro Lys His
        515                 520                 525

tct ctg ctg tac gag tac ttc aca gtt tat aac gag ctc acc aag gtc          1632
Ser Leu Leu Tyr Glu Tyr Phe Thr Val Tyr Asn Glu Leu Thr Lys Val
    530                 535                 540

aaa tac gtc aca gaa ggg atg aga aag cca gca ttc ctg tct gga gag 1680
Lys Tyr Val Thr Glu Gly Met Arg Lys Pro Ala Phe Leu Ser Gly Glu
545                 550                 555                 560

cag aag aaa gct atc gtg gac ctc ctc ttc aag acg aac cgg aaa gtt 1728
Gln Lys Lys Ala Ile Val Asp Leu Leu Phe Lys Thr Asn Arg Lys Val
                565                 570                 575

acc gtg aaa cag ctc aaa gaa gac tat ttc aaa aag att gaa tgt ttc 1776
Thr Val Lys Gln Leu Lys Glu Asp Tyr Phe Lys Lys Ile Glu Cys Phe
            580                 585                 590

gac tct gtt gaa atc agc gga gtg gag gat cgc ttc aac gca tcc ctg 1824
Asp Ser Val Glu Ile Ser Gly Val Glu Asp Arg Phe Asn Ala Ser Leu
        595                 600                 605

gga acg tat cac gat ctc ctg aaa atc att aaa gac aag gac ttc ctg 1872
Gly Thr Tyr His Asp Leu Leu Lys Ile Ile Lys Asp Lys Asp Phe Leu
    610                 615                 620

gac aat gag gag aac gag gac att ctt gag gac att gtc ctc acc ctt 1920
Asp Asn Glu Glu Asn Glu Asp Ile Leu Glu Asp Ile Val Leu Thr Leu
625                 630                 635                 640

acg ttg ttt gaa gat agg gag atg att gaa gaa cgc ttg aaa act tac 1968
Thr Leu Phe Glu Asp Arg Glu Met Ile Glu Glu Arg Leu Lys Thr Tyr
                645                 650                 655

gct cat ctc ttc gac gac aaa gtc atg aaa cag ctc aag agg cgc cga 2016
Ala His Leu Phe Asp Asp Lys Val Met Lys Gln Leu Lys Arg Arg Arg
            660                 665                 670

tat aca gga tgg ggg cgg ctg tca aga aaa ctg atc aat ggg atc cga 2064
Tyr Thr Gly Trp Gly Arg Leu Ser Arg Lys Leu Ile Asn Gly Ile Arg
        675                 680                 685

gac aag cag agt gga aag aca atc ctg gat ttt ctt aag tcc gat gga 2112
Asp Lys Gln Ser Gly Lys Thr Ile Leu Asp Phe Leu Lys Ser Asp Gly
    690                 695                 700

ttt gcc aac cgg aac ttc atg cag ttg atc cat gat gac tct ctc acc 2160
Phe Ala Asn Arg Asn Phe Met Gln Leu Ile His Asp Asp Ser Leu Thr
705                 710                 715                 720

ttt aag gag gac atc cag aaa gca caa gtt tct ggc cag ggg gac agt 2208
Phe Lys Glu Asp Ile Gln Lys Ala Gln Val Ser Gly Gln Gly Asp Ser
                725                 730                 735

ctt cac gag cac atc gct aat ctt gca ggt agc cca gct atc aaa aag 2256
Leu His Glu His Ile Ala Asn Leu Ala Gly Ser Pro Ala Ile Lys Lys
            740                 745                 750

gga ata ctg cag acc gtt aag gtc gtg gat gaa ctc gtc aaa gta atg 2304
Gly Ile Leu Gln Thr Val Lys Val Val Asp Glu Leu Val Lys Val Met
        755                 760                 765

gga agg cat aag ccc gag aat atc gtt atc gag atg gcc cga gag aac 2352
Gly Arg His Lys Pro Glu Asn Ile Val Ile Glu Met Ala Arg Glu Asn
    770                 775                 780

caa act acc cag aag gga cag aag aac agt agg gaa agg atg aag agg 2400
Gln Thr Thr Gln Lys Gly Gln Lys Asn Ser Arg Glu Arg Met Lys Arg

785                 790                 795                 800

att gaa gag ggt ata aaa gaa ctg ggg tcc caa atc ctt aag gaa cac          2448
Ile Glu Glu Gly Ile Lys Glu Leu Gly Ser Gln Ile Leu Lys Glu His
                805                 810                 815

cca gtt gaa aac acc cag ctt cag aat gag aag ctc tac ctg tac tac          2496
Pro Val Glu Asn Thr Gln Leu Gln Asn Glu Lys Leu Tyr Leu Tyr Tyr
            820                 825                 830

ctg cag aac ggc agg gac atg tac gtg gat cag gaa ctg gac atc aat          2544
Leu Gln Asn Gly Arg Asp Met Tyr Val Asp Gln Glu Leu Asp Ile Asn
        835                 840                 845

cgg ctc tcc gac tac gac gtg gat cat atc gtg ccc cag tct ttt ctc          2592
Arg Leu Ser Asp Tyr Asp Val Asp His Ile Val Pro Gln Ser Phe Leu
    850                 855                 860

aaa gat gat tct att gat aat aaa gtg ttg aca aga tcc gat aaa aat          2640
Lys Asp Asp Ser Ile Asp Asn Lys Val Leu Thr Arg Ser Asp Lys Asn
865                 870                 875                 880

aga ggg aag agt gat aac gtc ccc tca gaa gaa gtt gtc aag aaa atg          2688
Arg Gly Lys Ser Asp Asn Val Pro Ser Glu Glu Val Val Lys Lys Met
                885                 890                 895

aaa aat tat tgg cgg cag ctg ctg aac gcc aaa ctg atc aca caa cgg          2736
Lys Asn Tyr Trp Arg Gln Leu Leu Asn Ala Lys Leu Ile Thr Gln Arg
            900                 905                 910

aag ttc gat aat ctg act aag gct gaa cga ggt ggc ctg tct gag ttg          2784
Lys Phe Asp Asn Leu Thr Lys Ala Glu Arg Gly Gly Leu Ser Glu Leu
        915                 920                 925

gat aaa gcc ggc ttc atc aaa agg cag ctt gtt gag aca cgc cag atc          2832
Asp Lys Ala Gly Phe Ile Lys Arg Gln Leu Val Glu Thr Arg Gln Ile
    930                 935                 940

acc aag cac gtg gcc caa att ctc gat tca cgc atg aac acc aag tac          2880
Thr Lys His Val Ala Gln Ile Leu Asp Ser Arg Met Asn Thr Lys Tyr
945                 950                 955                 960

gat gaa aat gac aaa ctg att cga gag gtg aaa gtt att act ctg aag          2928
Asp Glu Asn Asp Lys Leu Ile Arg Glu Val Lys Val Ile Thr Leu Lys
                965                 970                 975

tct aag ctg gtc tca gat ttc aga aag gac ttt cag ttt tat aag gtg          2976
Ser Lys Leu Val Ser Asp Phe Arg Lys Asp Phe Gln Phe Tyr Lys Val
            980                 985                 990

aga gag atc aac aat tac cac cat gcg cat gat gcc tac ctg aat gca          3024
Arg Glu Ile Asn Asn Tyr His His Ala His Asp Ala Tyr Leu Asn Ala
        995                 1000                1005

gtg gta ggc act gca ctt atc aaa aaa tat ccc aag ctt gaa tct gaa          3072
Val Val Gly Thr Ala Leu Ile Lys Lys Tyr Pro Lys Leu Glu Ser Glu
    1010                1015                1020

ttt gtt tac gga gac tat aaa gtg tac gat gtt agg aaa atg atc gca          3120
Phe Val Tyr Gly Asp Tyr Lys Val Tyr Asp Val Arg Lys Met Ile Ala
1025                1030                1035                1040

aag tct gag cag gaa ata ggc aag gcc acc gct aag tac ttc ttt tac 3168
Lys Ser Glu Gln Glu Ile Gly Lys Ala Thr Ala Lys Tyr Phe Phe Tyr
                1045                1050                1055

agc aat att atg aat ttt ttc aag acc gag att aca ctg gcc aat gga 3216
Ser Asn Ile Met Asn Phe Phe Lys Thr Glu Ile Thr Leu Ala Asn Gly
            1060                1065                1070

gag att cgg aag cga cca ctt atc gaa aca aac gga gaa aca gga gaa 3264
Glu Ile Arg Lys Arg Pro Leu Ile Glu Thr Asn Gly Glu Thr Gly Glu
        1075                1080                1085

atc gtg tgg gac aag ggt agg gat ttc gcg aca gtc cgg aag gtc ctg 3312
Ile Val Trp Asp Lys Gly Arg Asp Phe Ala Thr Val Arg Lys Val Leu
    1090                1095                1100

tcc atg ccg cag gtg aac atc gtt aaa aag acc gaa gta cag acc gga 3360
Ser Met Pro Gln Val Asn Ile Val Lys Lys Thr Glu Val Gln Thr Gly
1105                1110                1115                1120

ggc ttc tcc aag gaa agt atc ctc ccg aaa agg aac agc gac aag ctg 3408
Gly Phe Ser Lys Glu Ser Ile Leu Pro Lys Arg Asn Ser Asp Lys Leu
                1125                1130                1135

atc gca cgc aaa aaa gat tgg gac ccc aag aaa tac ggc gga ttc gat 3456
Ile Ala Arg Lys Lys Asp Trp Asp Pro Lys Lys Tyr Gly Gly Phe Asp
            1140                1145                1150

tct cct aca gtc gct tac agt gta ctg gtt gtg gcc aaa gtg gag aaa 3504
Ser Pro Thr Val Ala Tyr Ser Val Leu Val Val Ala Lys Val Glu Lys
        1155                1160                1165

ggg aag tct aaa aaa ctc aaa agc gtc aag gaa ctg ctg ggc atc aca 3552
Gly Lys Ser Lys Lys Leu Lys Ser Val Lys Glu Leu Leu Gly Ile Thr
    1170                1175                1180

atc atg gag cga tca agc ttc gaa aaa aac ccc atc gac ttt ctc gag 3600
Ile Met Glu Arg Ser Ser Phe Glu Lys Asn Pro Ile Asp Phe Leu Glu
1185                1190                1195                1200

gcg aaa gga tat aaa gag gtc aaa aaa gac ctc atc att aag ctt ccc 3648
Ala Lys Gly Tyr Lys Glu Val Lys Lys Asp Leu Ile Ile Lys Leu Pro
                1205                1210                1215

aag tac tct ctc ttt gag ctt gaa aac ggc cgg aaa cga atg ctc gct 3696
Lys Tyr Ser Leu Phe Glu Leu Glu Asn Gly Arg Lys Arg Met Leu Ala
            1220                1225                1230

agt gcg ggc gag ctg cag aaa ggt aac gag ctg gca ctg ccc tct aaa 3744
Ser Ala Gly Glu Leu Gln Lys Gly Asn Glu Leu Ala Leu Pro Ser Lys
        1235                1240                1245

tac gtt aat ttc ttg tat ctg gcc agc cac tat gaa aag ctc aaa ggg 3792
Tyr Val Asn Phe Leu Tyr Leu Ala Ser His Tyr Glu Lys Leu Lys Gly
    1250                1255                1260

tct ccc gaa gat aat gag cag aag cag ctg ttc gtg gaa caa cac aaa 3840
Ser Pro Glu Asp Asn Glu Gln Lys Gln Leu Phe Val Glu Gln His Lys
1265                1270                1275                1280

cac tac ctt gat gag atc atc gag caa ata agc gaa ttc tcc aaa aga 3888
His Tyr Leu Asp Glu Ile Ile Glu Gln Ile Ser Glu Phe Ser Lys Arg

1285                1290                1295

gtg atc ctc gcc gac gct aac ctc gat aag gtg ctt tct gct tac aat        3936
Val Ile Leu Ala Asp Ala Asn Leu Asp Lys Val Leu Ser Ala Tyr Asn
            1300                1305                1310

aag cac agg gat aag ccc atc agg gag cag gca gaa aac att atc cac        3984
Lys His Arg Asp Lys Pro Ile Arg Glu Gln Ala Glu Asn Ile Ile His
        1315                1320                1325

ttg ttt act ctg acc aac ttg ggc gcg cct gca gcc ttc aag tac ttc        4032
Leu Phe Thr Leu Thr Asn Leu Gly Ala Pro Ala Ala Phe Lys Tyr Phe
    1330                1335                1340

gac acc acc ata gac aga aag cgg tac acc tct aca aag gag gtc ctg        4080
Asp Thr Thr Ile Asp Arg Lys Arg Tyr Thr Ser Thr Lys Glu Val Leu
1345                1350                1355                1360

gac gcc aca ctg att cat cag tca att acg ggg ctc tat gaa aca aga        4128
Asp Ala Thr Leu Ile His Gln Ser Ile Thr Gly Leu Tyr Glu Thr Arg
                1365                1370                1375

atc gac ctc tct cag ctc ggt gga gac agc agg gct gac ccc aag aag        4176
Ile Asp Leu Ser Gln Leu Gly Gly Asp Ser Arg Ala Asp Pro Lys Lys
            1380                1385                1390

aag agg aag gtg tga    4191
Lys Arg Lys Val
        1395

<210> 10
<211> 4191
<212> DNA
<213> artificial
<400> SEQUENCE 10 (SPCas9D10A):
atg cat cat cac cac cac cat cac ggc agc ccc aag aag aag agg aag        48
Met His His His His His His His Gly Ser Pro Lys Lys Lys Arg Lys
1               5                   10                  15

gtg atg gac aag aag tac tcc att ggg ctc gct atc ggc aca aac agc        96
Val Met Asp Lys Lys Tyr Ser Ile Gly Leu Ala Ile Gly Thr Asn Ser
            20                  25                  30

gtc ggc tgg gcc gtc att acg gac gag tac aag gtg ccg agc aaa aaa        144
Val Gly Trp Ala Val Ile Thr Asp Glu Tyr Lys Val Pro Ser Lys Lys
        35                  40                  45

ttc aaa gtt ctg ggc aat acc gat cgc cac agc ata aag aag aac ctc        192
Phe Lys Val Leu Gly Asn Thr Asp Arg His Ser Ile Lys Lys Asn Leu
    50                  55                  60

att ggc gcc ctc ctg ttc gac tcc ggg gag acg gcc gaa gcc acg cgg        240
Ile Gly Ala Leu Leu Phe Asp Ser Gly Glu Thr Ala Glu Ala Thr Arg
65                  70                  75                  80

ctc aaa aga aca gca cgg cgc aga tat acc cgc aga aag aat cgg atc        288
Leu Lys Arg Thr Ala Arg Arg Arg Tyr Thr Arg Arg Lys Asn Arg Ile
                85                  90                  95

tgc tac ctg cag gag atc ttt agt aat gag atg gct aag gtg gat gac        336
Cys Tyr Leu Gln Glu Ile Phe Ser Asn Glu Met Ala Lys Val Asp Asp
            100                 105                 110

tct ttc ttc cat agg ctg gag gag tcc ttt ttg gtg gag gag gat aaa 384
Ser Phe Phe His Arg Leu Glu Glu Ser Phe Leu Val Glu Glu Asp Lys
        115                 120                 125

aag cac gag cgc cac cca atc ttt ggc aat atc gtg gac gag gtg gcg 432
Lys His Glu Arg His Pro Ile Phe Gly Asn Ile Val Asp Glu Val Ala
    130                 135                 140

tac cat gaa aag tac cca acc ata tat cat ctg agg aag aag ctt gta 480
Tyr His Glu Lys Tyr Pro Thr Ile Tyr His Leu Arg Lys Lys Leu Val
145                 150                 155                 160

gac agt act gat aag gct gac ttg cgg ttg atc tat ctc gcg ctg gcg 528
Asp Ser Thr Asp Lys Ala Asp Leu Arg Leu Ile Tyr Leu Ala Leu Ala
                165                 170                 175

cat atg atc aaa ttt cgg gga cac ttc ctc atc gag ggg gac ctg aac 576
His Met Ile Lys Phe Arg Gly His Phe Leu Ile Glu Gly Asp Leu Asn
            180                 185                 190

cca gac aac agc gat gtc gac aaa ctc ttt atc caa ctg gtt cag act 624
Pro Asp Asn Ser Asp Val Asp Lys Leu Phe Ile Gln Leu Val Gln Thr
        195                 200                 205

tac aat cag ctt ttc gaa gag aac ccg atc aac gca tcc gga gtt gac 672
Tyr Asn Gln Leu Phe Glu Glu Asn Pro Ile Asn Ala Ser Gly Val Asp
    210                 215                 220

gcc aaa gca atc ctg agc gct agg ctg tcc aaa tcc cgg cgg ctc gaa 720
Ala Lys Ala Ile Leu Ser Ala Arg Leu Ser Lys Ser Arg Arg Leu Glu
225                 230                 235                 240

aac ctc atc gca cag ctc cct ggg gag aag aag aac ggc ctg ttt ggt 768
Asn Leu Ile Ala Gln Leu Pro Gly Glu Lys Lys Asn Gly Leu Phe Gly
                245                 250                 255

aat ctt atc gcc ctg tca ctc ggg ctg acc ccc aac ttt aaa tct aac 816
Asn Leu Ile Ala Leu Ser Leu Gly Leu Thr Pro Asn Phe Lys Ser Asn
            260                 265                 270

ttc gac ctg gcc gaa gat gcc aag ctt caa ctg agc aaa gac acc tac 864
Phe Asp Leu Ala Glu Asp Ala Lys Leu Gln Leu Ser Lys Asp Thr Tyr
        275                 280                 285

gat gat gat ctc gac aat ctg ctg gcc cag atc ggc gac cag tac gca 912
Asp Asp Asp Leu Asp Asn Leu Leu Ala Gln Ile Gly Asp Gln Tyr Ala
    290                 295                 300

gac ctt ttt ttg gcg gca aag aac ctg tca gac gcc att ctg ctg agt 960
Asp Leu Phe Leu Ala Ala Lys Asn Leu Ser Asp Ala Ile Leu Leu Ser
305                 310                 315                 320

gat att ctg cga gtg aac acg gag atc acc aaa gct ccg ctg agc gct 1008
Asp Ile Leu Arg Val Asn Thr Glu Ile Thr Lys Ala Pro Leu Ser Ala
                325                 330                 335

agt atg atc aag cgc tat gat gag cac cac caa gac ttg act ttg ctg 1056
Ser Met Ile Lys Arg Tyr Asp Glu His His Gln Asp Leu Thr Leu Leu
            340                 345                 350

aag gcc ctt gtc aga cag caa ctg cct gag aag tac aag gaa att ttc 1104
Lys Ala Leu Val Arg Gln Gln Leu Pro Glu Lys Tyr Lys Glu Ile Phe
        355                 360                 365

ttc gat cag tct aaa aat ggc tac gcc gga tac att gac ggc gga gca 1152
Phe Asp Gln Ser Lys Asn Gly Tyr Ala Gly Tyr Ile Asp Gly Gly Ala
    370                 375                 380

agc cag gag gaa ttt tac aaa ttt att aag ccc atc ttg gaa aaa atg 1200
Ser Gln Glu Glu Phe Tyr Lys Phe Ile Lys Pro Ile Leu Glu Lys Met
385                 390                 395                 400

gac ggc acc gag gag ctg ctg gta aag ctt aac aga gaa gat ctg ttg 1248
Asp Gly Thr Glu Glu Leu Leu Val Lys Leu Asn Arg Glu Asp Leu Leu
                405                 410                 415

cgc aaa cag cgc act ttc gac aat gga agc atc ccc cac cag att cac 1296
Arg Lys Gln Arg Thr Phe Asp Asn Gly Ser Ile Pro His Gln Ile His
            420                 425                 430

ctg ggc gaa ctg cac gct atc ctc agg cgg caa gag gat ttc tac ccc 1344
Leu Gly Glu Leu His Ala Ile Leu Arg Arg Gln Glu Asp Phe Tyr Pro
        435                 440                 445

ttt ttg aaa gat aac agg gaa aag att gag aaa atc ctc aca ttt cgg 1392
Phe Leu Lys Asp Asn Arg Glu Lys Ile Glu Lys Ile Leu Thr Phe Arg
    450                 455                 460

ata ccc tac tat gta ggc ccc ctc gcc cgg gga aat tcc aga ttc gcg 1440
Ile Pro Tyr Tyr Val Gly Pro Leu Ala Arg Gly Asn Ser Arg Phe Ala
465                 470                 475                 480

tgg atg act cgc aaa tca gaa gag acc atc act ccc tgg aac ttc gag 1488
Trp Met Thr Arg Lys Ser Glu Glu Thr Ile Thr Pro Trp Asn Phe Glu
                485                 490                 495

gaa gtc gtg gat aag ggg gcc tct gcc cag tcc ttc atc gaa agg atg 1536
Glu Val Val Asp Lys Gly Ala Ser Ala Gln Ser Phe Ile Glu Arg Met
            500                 505                 510

act aac ttt gat aaa aat ctg cct aac gaa aag gtg ctt cct aaa cac 1584
Thr Asn Phe Asp Lys Asn Leu Pro Asn Glu Lys Val Leu Pro Lys His
        515                 520                 525

tct ctg ctg tac gag tac ttc aca gtt tat aac gag ctc acc aag gtc 1632
Ser Leu Leu Tyr Glu Tyr Phe Thr Val Tyr Asn Glu Leu Thr Lys Val
    530                 535                 540

aaa tac gtc aca gaa ggg atg aga aag cca gca ttc ctg tct gga gag 1680
Lys Tyr Val Thr Glu Gly Met Arg Lys Pro Ala Phe Leu Ser Gly Glu
545                 550                 555                 560

cag aag aaa gct atc gtg gac ctc ctc ttc aag acg aac cgg aaa gtt 1728
Gln Lys Lys Ala Ile Val Asp Leu Leu Phe Lys Thr Asn Arg Lys Val
                565                 570                 575

acc gtg aaa cag ctc aaa gaa gac tat ttc aaa aag att gaa tgt ttc 1776
Thr Val Lys Gln Leu Lys Glu Asp Tyr Phe Lys Lys Ile Glu Cys Phe
            580                 585                 590

gac tct gtt gaa atc agc gga gtg gag gat cgc ttc aac gca tcc ctg 1824

Asp Ser Val Glu Ile Ser Gly Val Glu Asp Arg Phe Asn Ala Ser Leu
        595                 600                 605

gga acg tat cac gat ctc ctg aaa atc att aaa gac aag gac ttc ctg        1872
Gly Thr Tyr His Asp Leu Leu Lys Ile Ile Lys Asp Lys Asp Phe Leu
    610                 615                 620

gac aat gag gag aac gag gac att ctt gag gac att gtc ctc acc ctt        1920
Asp Asn Glu Glu Asn Glu Asp Ile Leu Glu Asp Ile Val Leu Thr Leu
625                 630                 635                 640

acg ttg ttt gaa gat agg gag atg att gaa gaa cgc ttg aaa act tac        1968
Thr Leu Phe Glu Asp Arg Glu Met Ile Glu Glu Arg Leu Lys Thr Tyr
                645                 650                 655

gct cat ctc ttc gac gac aaa gtc atg aaa cag ctc aag agg cgc cga        2016
Ala His Leu Phe Asp Asp Lys Val Met Lys Gln Leu Lys Arg Arg Arg
            660                 665                 670

tat aca gga tgg ggg cgg ctg tca aga aaa ctg atc aat ggg atc cga        2064
Tyr Thr Gly Trp Gly Arg Leu Ser Arg Lys Leu Ile Asn Gly Ile Arg
        675                 680                 685

gac aag cag agt gga aag aca atc ctg gat ttt ctt aag tcc gat gga        2112
Asp Lys Gln Ser Gly Lys Thr Ile Leu Asp Phe Leu Lys Ser Asp Gly
    690                 695                 700

ttt gcc aac agg aac ttc atg cag ttg atc cat gat gac tct ctc acc        2160
Phe Ala Asn Arg Asn Phe Met Gln Leu Ile His Asp Asp Ser Leu Thr
705                 710                 715                 720

ttt aag gag gac atc cag aaa gca caa gtt tct ggc cag ggg gac agt        2208
Phe Lys Glu Asp Ile Gln Lys Ala Gln Val Ser Gly Gln Gly Asp Ser
                725                 730                 735

ctt cac gag cac atc gct aat ctt gca ggt agc cca gct atc aaa aag        2256
Leu His Glu His Ile Ala Asn Leu Ala Gly Ser Pro Ala Ile Lys Lys
            740                 745                 750

gga ata ctg cag acc gtt aag gtc gtg gat gaa ctc gtc aaa gta atg        2304
Gly Ile Leu Gln Thr Val Lys Val Val Asp Glu Leu Val Lys Val Met
        755                 760                 765

gga agg cat aag ccc gag aat atc gtt atc gag atg gcc cga gag aac        2352
Gly Arg His Lys Pro Glu Asn Ile Val Ile Glu Met Ala Arg Glu Asn
    770                 775                 780

caa act acc cag aag gga cag aag aac agt agg gaa agg atg aag agg        2400
Gln Thr Thr Gln Lys Gly Gln Lys Asn Ser Arg Glu Arg Met Lys Arg
785                 790                 795                 800

att gaa gag ggt ata aaa gaa ctg ggg tcc caa atc ctt aag gaa cac        2448
Ile Glu Glu Gly Ile Lys Glu Leu Gly Ser Gln Ile Leu Lys Glu His
                805                 810                 815

cca gtt gaa aac acc cag ctt cag aat gag aag ctc tac ctg tac tac        2496
Pro Val Glu Asn Thr Gln Leu Gln Asn Glu Lys Leu Tyr Leu Tyr Tyr
            820                 825                 830

ctg cag aac ggc agg gac atg tac gtg gat cag gaa ctg gac atc aat        2544
Leu Gln Asn Gly Arg Asp Met Tyr Val Asp Gln Glu Leu Asp Ile Asn
        835                 840                 845

cgg ctc tcc gac tac gac gtg gat cat atc gtg ccc cag tct ttt ctc 2592
Arg Leu Ser Asp Tyr Asp Val Asp His Ile Val Pro Gln Ser Phe Leu
    850                 855                 860

aaa gat gat tct att gat aat aaa gtg ttg aca aga tcc gat aaa aat 2640
Lys Asp Asp Ser Ile Asp Asn Lys Val Leu Thr Arg Ser Asp Lys Asn
865                 870                 875                 880

aga ggg aag agt gat aac gtc ccc tca gaa gaa gtt gtc aag aaa atg 2688
Arg Gly Lys Ser Asp Asn Val Pro Ser Glu Glu Val Val Lys Lys Met
                885                 890                 895

aaa aat tat tgg cgg cag ctg ctg aac gcc aaa ctg atc aca caa cgg 2736
Lys Asn Tyr Trp Arg Gln Leu Leu Asn Ala Lys Leu Ile Thr Gln Arg
            900                 905                 910

aag ttc gat aat ctg act aag gct gaa cga ggt ggc ctg tct gag ttg 2784
Lys Phe Asp Asn Leu Thr Lys Ala Glu Arg Gly Gly Leu Ser Glu Leu
        915                 920                 925

gat aaa gcc ggc ttc atc aaa agg cag ctt gtt gag aca cgc cag atc 2832
Asp Lys Ala Gly Phe Ile Lys Arg Gln Leu Val Glu Thr Arg Gln Ile
    930                 935                 940

acc aag cac gtg gcc caa att ctc gat tca cgc atg aac acc aag tac 2880
Thr Lys His Val Ala Gln Ile Leu Asp Ser Arg Met Asn Thr Lys Tyr
945                 950                 955                 960

gat gaa aat gac aaa ctg att cga gag gtg aaa gtt att act ctg aag 2928
Asp Glu Asn Asp Lys Leu Ile Arg Glu Val Lys Val Ile Thr Leu Lys
                965                 970                 975

tct aag ctg gtc tca gat ttc aga aag gac ttt cag ttt tat aag gtg 2976
Ser Lys Leu Val Ser Asp Phe Arg Lys Asp Phe Gln Phe Tyr Lys Val
            980                 985                 990

aga gag atc aac aat tac cac cat gcg cat gat gcc tac ctg aat gca 3024
Arg Glu Ile Asn Asn Tyr His His Ala His Asp Ala Tyr Leu Asn Ala
        995                 1000                1005

gtg gta ggc act gca ctt atc aaa aaa tat ccc aag ctt gaa tct gaa 3072
Val Val Gly Thr Ala Leu Ile Lys Lys Tyr Pro Lys Leu Glu Ser Glu
    1010                1015                1020

ttt gtt tac gga gac tat aaa gtg tac gat gtt agg aaa atg atc gca 3120
Phe Val Tyr Gly Asp Tyr Lys Val Tyr Asp Val Arg Lys Met Ile Ala
1025                1030                1035                1040

aag tct gag cag gaa ata ggc aag gcc acc gct aag tac ttc ttt tac 3168
Lys Ser Glu Gln Glu Ile Gly Lys Ala Thr Ala Lys Tyr Phe Phe Tyr
                1045                1050                1055

agc aat att atg aat ttt ttc aag acc gag att aca ctg gcc aat gga 3216
Ser Asn Ile Met Asn Phe Phe Lys Thr Glu Ile Thr Leu Ala Asn Gly
            1060                1065                1070

gag att cgg aag cga cca ctt atc gaa aca aac gga gaa aca gga gaa 3264
Glu Ile Arg Lys Arg Pro Leu Ile Glu Thr Asn Gly Glu Thr Gly Glu
        1075                1080                1085

atc gtg tgg gac aag ggt agg gat ttc gcg aca gtc cgg aag gtc ctg 3312

Ile Val Trp Asp Lys Gly Arg Asp Phe Ala Thr Val Arg Lys Val Leu
    1090                1095                1100

tcc atg ccg cag gtg aac atc gtt aaa aag acc gaa gta cag acc gga      3360
Ser Met Pro Gln Val Asn Ile Val Lys Lys Thr Glu Val Gln Thr Gly
1105                1110                1115                1120

ggc ttc tcc aag gaa agt atc ctc ccg aaa agg aac agc gac aag ctg      3408
Gly Phe Ser Lys Glu Ser Ile Leu Pro Lys Arg Asn Ser Asp Lys Leu
                1125                1130                1135

atc gca cgc aaa aaa gat tgg gac ccc aag aaa tac ggc gga ttc gat      3456
Ile Ala Arg Lys Lys Asp Trp Asp Pro Lys Lys Tyr Gly Gly Phe Asp
            1140                1145                1150

tct cct aca gtc gct tac agt gta ctg gtt gtg gcc aaa gtg gag aaa      3504
Ser Pro Thr Val Ala Tyr Ser Val Leu Val Val Ala Lys Val Glu Lys
        1155                1160                1165

ggg aag tct aaa aaa ctc aaa agc gtc aag gaa ctg ctg ggc atc aca      3552
Gly Lys Ser Lys Lys Leu Lys Ser Val Lys Glu Leu Leu Gly Ile Thr
    1170                1175                1180

atc atg gag cga tca agc ttc gaa aaa aac ccc atc gac ttt ctc gag      3600
Ile Met Glu Arg Ser Ser Phe Glu Lys Asn Pro Ile Asp Phe Leu Glu
1185                1190                1195                1200

gcg aaa gga tat aaa gag gtc aaa aaa gac ctc atc att aag ctt ccc      3648
Ala Lys Gly Tyr Lys Glu Val Lys Lys Asp Leu Ile Ile Lys Leu Pro
                1205                1210                1215

aag tac tct ctc ttt gag ctt gaa aac ggc cgg aaa cga atg ctc gct      3696
Lys Tyr Ser Leu Phe Glu Leu Glu Asn Gly Arg Lys Arg Met Leu Ala
            1220                1225                1230

agt gcg ggc gag ctg cag aaa ggt aac gag ctg gca ctg ccc tct aaa      3744
Ser Ala Gly Glu Leu Gln Lys Gly Asn Glu Leu Ala Leu Pro Ser Lys
        1235                1240                1245

tac gtt aat ttc ttg tat ctg gcc agc cac tat gaa aag ctc aaa ggg      3792
Tyr Val Asn Phe Leu Tyr Leu Ala Ser His Tyr Glu Lys Leu Lys Gly
    1250                1255                1260

tct ccc gaa gat aat gag cag aag cag ctg ttc gtg gaa caa cac aaa      3840
Ser Pro Glu Asp Asn Glu Gln Lys Gln Leu Phe Val Glu Gln His Lys
1265                1270                1275                1280

cac tac ctt gat gag atc atc gag caa ata agc gaa ttc tcc aaa aga      3888
His Tyr Leu Asp Glu Ile Ile Glu Gln Ile Ser Glu Phe Ser Lys Arg
                1285                1290                1295

gtg atc ctc gcc gac gct aac ctc gat aag gtg ctt tct gct tac aat      3936
Val Ile Leu Ala Asp Ala Asn Leu Asp Lys Val Leu Ser Ala Tyr Asn
            1300                1305                1310

aag cac agg gat aag ccc atc agg gag cag gca gaa aac att atc cac      3984
Lys His Arg Asp Lys Pro Ile Arg Glu Gln Ala Glu Asn Ile Ile His
        1315                1320                1325

ttg ttt act ctg acc aac ttg ggc gcg cct gca gcc ttc aag tac ttc      4032
Leu Phe Thr Leu Thr Asn Leu Gly Ala Pro Ala Ala Phe Lys Tyr Phe
    1330                1335                1340

gac acc acc ata gac aga aag cgg tac acc tct aca aag gag gtc ctg 4080
Asp Thr Thr Ile Asp Arg Lys Arg Tyr Thr Ser Thr Lys Glu Val Leu
1345                1350                1355                1360

gac gcc aca ctg att cat cag tca att acg ggg ctc tat gaa aca aga 4128
Asp Ala Thr Leu Ile His Gln Ser Ile Thr Gly Leu Tyr Glu Thr Arg
                1365                1370                1375

atc gac ctc tct cag ctc ggt gga gac agc agg gct gac ccc aag aag 4176
Ile Asp Leu Ser Gln Leu Gly Gly Asp Ser Arg Ala Asp Pro Lys Lys
            1380                1385                1390

aag agg aag gtg tga 4191
Lys Arg Lys Val
        1395

<210> 11
<211> 3468
<212> DNA
<213> artificial
<400> SEQUENCE 11 (STCas9):
atg ctc gag cat cac cac cac cat cac ggc agc ccc aag aag aag agg 48
Met Leu Glu His His His His His His Gly Ser Pro Lys Lys Lys Arg
1               5                   10                  15

aag gtg ggc cgc gga gcc acc atg agc gac ctg gtg ctg ggc ctg gac 96
Lys Val Gly Arg Gly Ala Thr Met Ser Asp Leu Val Leu Gly Leu Asp
            20                  25                  30

atc ggc atc ggc agc gtg ggc gtg ggc atc ctg aac aag gtg acc ggc 144
Ile Gly Ile Gly Ser Val Gly Val Gly Ile Leu Asn Lys Val Thr Gly
        35                  40                  45

gag atc atc cac aag aac agt cgc atc ttc cct gct gct cag gct gag 192
Glu Ile Ile His Lys Asn Ser Arg Ile Phe Pro Ala Ala Gln Ala Glu
    50                  55                  60

aac aac ctg gtg cgc cgc acc aac cgc cag ggt cgc cgg ctt gct cgc 240
Asn Asn Leu Val Arg Arg Thr Asn Arg Gln Gly Arg Arg Leu Ala Arg
65                  70                  75                  80

cgc aag aag cac cgg cgc gtg cgc ctg aac cgc ctg ttc gag gag agc 288
Arg Lys Lys His Arg Arg Val Arg Leu Asn Arg Leu Phe Glu Glu Ser
                85                  90                  95

ggc ctg atc acc gac ttc acc aag atc agc atc aac ctg aac ccc tac 336
Gly Leu Ile Thr Asp Phe Thr Lys Ile Ser Ile Asn Leu Asn Pro Tyr
            100                 105                 110

cag ctg cgc gtg aag ggc ctg acc gac gag ctg agc aac gag gag ctg 384
Gln Leu Arg Val Lys Gly Leu Thr Asp Glu Leu Ser Asn Glu Glu Leu
        115                 120                 125

ttc atc gcc ctg aag aac atg gtg aag cac cgc ggc atc agc tac ctg 432
Phe Ile Ala Leu Lys Asn Met Val Lys His Arg Gly Ile Ser Tyr Leu
    130                 135                 140

gac gac gcc agc gac gac ggc aac agc agc gtg ggc gac tac gcc cag 480
Asp Asp Ala Ser Asp Asp Gly Asn Ser Ser Val Gly Asp Tyr Ala Gln
145                 150                 155                 160

atc gtg aag gag aac agc aag cag ctg gag acc aag acc ccc ggc cag 528
Ile Val Lys Glu Asn Ser Lys Gln Leu Glu Thr Lys Thr Pro Gly Gln
                165                 170                 175

atc cag ctg gag cgc tac cag acc tac ggc cag ctg cgc ggc gac ttc 576
Ile Gln Leu Glu Arg Tyr Gln Thr Tyr Gly Gln Leu Arg Gly Asp Phe
            180                 185                 190

acc gtg gag aag gac ggc aag aag cac cgc ctg atc aac gtg ttc ccc 624
Thr Val Glu Lys Asp Gly Lys Lys His Arg Leu Ile Asn Val Phe Pro
        195                 200                 205

acc agc gcc tac cgc agc gag gcc ctg cgc atc ctg cag acc cag cag 672
Thr Ser Ala Tyr Arg Ser Glu Ala Leu Arg Ile Leu Gln Thr Gln Gln
    210                 215                 220

gag ttc aac ccc cag atc acc gac gag ttc atc aac cgc tac ctg gag 720
Glu Phe Asn Pro Gln Ile Thr Asp Glu Phe Ile Asn Arg Tyr Leu Glu
225                 230                 235                 240

atc ctg acc ggc aag cgc aag tac tac cac ggc ccc ggc aac gag aag 768
Ile Leu Thr Gly Lys Arg Lys Tyr Tyr His Gly Pro Gly Asn Glu Lys
                245                 250                 255

agc cgc acc gac tac ggc cgc tac cgc acc agc ggc gag acc ctg gac 816
Ser Arg Thr Asp Tyr Gly Arg Tyr Arg Thr Ser Gly Glu Thr Leu Asp
            260                 265                 270

aac atc ttc ggc atc ctg atc ggc aag tgc acc ttc tac ccc gac gag 864
Asn Ile Phe Gly Ile Leu Ile Gly Lys Cys Thr Phe Tyr Pro Asp Glu
        275                 280                 285

ttc cgc gcc gcc aag gcc agc tac acc gcc cag gag ttc aac ctg ctg 912
Phe Arg Ala Ala Lys Ala Ser Tyr Thr Ala Gln Glu Phe Asn Leu Leu
    290                 295                 300

aac gac ctg aac aac ctg acc gtg ccc acc gag acc aag aag ctg agc 960
Asn Asp Leu Asn Asn Leu Thr Val Pro Thr Glu Thr Lys Lys Leu Ser
305                 310                 315                 320

aag gag cag aag aac cag atc atc aac tac gtg aag aac gag aag gcc 1008
Lys Glu Gln Lys Asn Gln Ile Ile Asn Tyr Val Lys Asn Glu Lys Ala
                325                 330                 335

atg ggc ccc gcc aag ctg ttc aag tac atc gcc aag ctg ctg agc tgc 1056
Met Gly Pro Ala Lys Leu Phe Lys Tyr Ile Ala Lys Leu Leu Ser Cys
            340                 345                 350

gac gtg gcc gac atc aag ggc tac cgc atc gac aag agc ggc aag gcc 1104
Asp Val Ala Asp Ile Lys Gly Tyr Arg Ile Asp Lys Ser Gly Lys Ala
        355                 360                 365

gag atc cac acc ttc gag gcc tac cgc aag atg aag acc ctg gag acc 1152
Glu Ile His Thr Phe Glu Ala Tyr Arg Lys Met Lys Thr Leu Glu Thr
    370                 375                 380

ctg gac atc gag cag atg gac cgc gag acc ctg gac aag ctg gcc tac 1200
Leu Asp Ile Glu Gln Met Asp Arg Glu Thr Leu Asp Lys Leu Ala Tyr
385                 390                 395                 400

gtg ctg acc ctg aac acc gag cgc gag ggc atc cag gag gcc ctg gag 1248

Val Leu Thr Leu Asn Thr Glu Arg Glu Gly Ile Gln Glu Ala Leu Glu
                405                 410                 415

cac gag ttc gcc gac ggc agc ttc agc cag aag cag gtg gac gag ctg      1296
His Glu Phe Ala Asp Gly Ser Phe Ser Gln Lys Gln Val Asp Glu Leu
            420                 425                 430

gtg cag ttc cgc aag gcc aac agc agc atc ttc ggc aag ggc tgg cac      1344
Val Gln Phe Arg Lys Ala Asn Ser Ser Ile Phe Gly Lys Gly Trp His
        435                 440                 445

aac ttc agc gtg aag ctg atg atg gag ctg atc ccc gag ctg tac gag      1392
Asn Phe Ser Val Lys Leu Met Met Glu Leu Ile Pro Glu Leu Tyr Glu
    450                 455                 460

acc agc gag gag cag atg acc atc ctg acc cgc ctg ggc aag cag aag      1440
Thr Ser Glu Glu Gln Met Thr Ile Leu Thr Arg Leu Gly Lys Gln Lys
465                 470                 475                 480

acc acc agc agc agc aac aag acc aag tac atc gac gag aag ctg ctg      1488
Thr Thr Ser Ser Ser Asn Lys Thr Lys Tyr Ile Asp Glu Lys Leu Leu
                485                 490                 495

acc gag gag atc tac aac ccc gtg gtg gcc aag agc gtg cgc cag gcc      1536
Thr Glu Glu Ile Tyr Asn Pro Val Val Ala Lys Ser Val Arg Gln Ala
            500                 505                 510

atc aag atc gtg aac gcc gcc atc aag gag tac ggc gac ttc gac aac      1584
Ile Lys Ile Val Asn Ala Ala Ile Lys Glu Tyr Gly Asp Phe Asp Asn
        515                 520                 525

atc gtg atc gag atg gcc cgc gag acc aac gag gac gac gag aag aag      1632
Ile Val Ile Glu Met Ala Arg Glu Thr Asn Glu Asp Asp Glu Lys Lys
    530                 535                 540

gcc atc cag aag atc cag aag gcc aac aag gac gag aag gac gcc gcc      1680
Ala Ile Gln Lys Ile Gln Lys Ala Asn Lys Asp Glu Lys Asp Ala Ala
545                 550                 555                 560

atg ctg aag gcc gcc aac cag tac aac ggc aag gcc gag ctg ccc cac      1728
Met Leu Lys Ala Ala Asn Gln Tyr Asn Gly Lys Ala Glu Leu Pro His
                565                 570                 575

agc gtg ttc cac ggc cac aag cag ctg gcc acc aag atc cgc ctg tgg      1776
Ser Val Phe His Gly His Lys Gln Leu Ala Thr Lys Ile Arg Leu Trp
            580                 585                 590

cac cag cag ggc gag cgc tgc ctg tac acc ggc aag acc atc agc atc      1824
His Gln Gln Gly Glu Arg Cys Leu Tyr Thr Gly Lys Thr Ile Ser Ile
        595                 600                 605

cac gac ctg atc aac aac agc aac cag ttc gag gtg gac cac atc ctg      1872
His Asp Leu Ile Asn Asn Ser Asn Gln Phe Glu Val Asp His Ile Leu
    610                 615                 620

ccc ctg agc atc acc ttc gac gac agc ctg gcc aac aag gtg ctg gtg      1920
Pro Leu Ser Ile Thr Phe Asp Asp Ser Leu Ala Asn Lys Val Leu Val
625                 630                 635                 640

tac gcc acc gcc aac cag gag aag ggc cag cgc acc ccc tac cag gcc      1968
Tyr Ala Thr Ala Asn Gln Glu Lys Gly Gln Arg Thr Pro Tyr Gln Ala
                645                 650                 655

ctg gac agc atg gac gac gcc tgg agc ttc cgc gag ctg aag gcc ttc 2016
Leu Asp Ser Met Asp Asp Ala Trp Ser Phe Arg Glu Leu Lys Ala Phe
            660                 665                 670

gtg cgc gag agc aag acc ctg agc aac aag aag aag gag tac ctg ctg 2064
Val Arg Glu Ser Lys Thr Leu Ser Asn Lys Lys Lys Glu Tyr Leu Leu
        675                 680                 685

acc gag gag gac atc agc aag ttc gac gtg cgc aag aag ttc atc gag 2112
Thr Glu Glu Asp Ile Ser Lys Phe Asp Val Arg Lys Lys Phe Ile Glu
    690                 695                 700

cgc aac ctg gtg gac acc ctc tac gcc agc cgc gtg gtg ctg aac gcc 2160
Arg Asn Leu Val Asp Thr Leu Tyr Ala Ser Arg Val Val Leu Asn Ala
705                 710                 715                 720

ctg cag gag cac ttc cgc gcc cac aag atc gac acc aag gtg agc gtg 2208
Leu Gln Glu His Phe Arg Ala His Lys Ile Asp Thr Lys Val Ser Val
                725                 730                 735

gtg cgc ggc cag ttc acc agc cag ctg cgc cgc cac tgg ggc atc gag 2256
Val Arg Gly Gln Phe Thr Ser Gln Leu Arg Arg His Trp Gly Ile Glu
            740                 745                 750

aag acc cgc gac acc tac cac cac cac gcc gtg gac gcc ctg atc att 2304
Lys Thr Arg Asp Thr Tyr His His His Ala Val Asp Ala Leu Ile Ile
        755                 760                 765

gcg gct tct agc cag ctg aac ctg tgg aag aag cag aag aac acc ctg 2352
Ala Ala Ser Ser Gln Leu Asn Leu Trp Lys Lys Gln Lys Asn Thr Leu
    770                 775                 780

gtg agc tac agc gag gac cag ctg ctg gac atc gag acc ggc gag ctg 2400
Val Ser Tyr Ser Glu Asp Gln Leu Leu Asp Ile Glu Thr Gly Glu Leu
785                 790                 795                 800

atc agc gac gac gag tac aag gag agc gtg ttc aag gcc ccc tac cag 2448
Ile Ser Asp Asp Glu Tyr Lys Glu Ser Val Phe Lys Ala Pro Tyr Gln
                805                 810                 815

cac ttc gtg gac acc ctg aag agc aag gag ttc gag gac agc atc ctg 2496
His Phe Val Asp Thr Leu Lys Ser Lys Glu Phe Glu Asp Ser Ile Leu
            820                 825                 830

ttc agc tac cag gtg gac agc aag ttc aac cgc aag atc agc gac gcc 2544
Phe Ser Tyr Gln Val Asp Ser Lys Phe Asn Arg Lys Ile Ser Asp Ala
        835                 840                 845

acc atc tac gcc acc cgc cag gcc aag gtg ggc aag gac aag gcc gac 2592
Thr Ile Tyr Ala Thr Arg Gln Ala Lys Val Gly Lys Asp Lys Ala Asp
    850                 855                 860

gag acc tac gtg ctg ggc aag atc aag gac atc tac acc cag gac ggc 2640
Glu Thr Tyr Val Leu Gly Lys Ile Lys Asp Ile Tyr Thr Gln Asp Gly
865                 870                 875                 880

tac gac gcc ttc atg aag atc tac aag aag gac aag agc aag ttc ctg 2688
Tyr Asp Ala Phe Met Lys Ile Tyr Lys Lys Asp Lys Ser Lys Phe Leu
                885                 890                 895

atg tac cgc cac gac ccc cag acc ttc gag aag gtg atc gag ccc atc 2736

Met Tyr Arg His Asp Pro Gln Thr Phe Glu Lys Val Ile Glu Pro Ile
            900                 905                 910

ctg gag aac tac ccc aac aag cag atc aac gat aaa ggc aag gag gtg 2784
Leu Glu Asn Tyr Pro Asn Lys Gln Ile Asn Asp Lys Gly Lys Glu Val
        915                 920                 925

ccc tgc aac ccc ttc ctg aag tac aag gag gag cac ggc tac atc cgc 2832
Pro Cys Asn Pro Phe Leu Lys Tyr Lys Glu Glu His Gly Tyr Ile Arg
    930                 935                 940

aag tac agc aag aag ggc aac ggc ccc gag atc aag agc ctg aag tac 2880
Lys Tyr Ser Lys Lys Gly Asn Gly Pro Glu Ile Lys Ser Leu Lys Tyr
945                 950                 955                 960

tac gac agc aag ctg ggc aac cac atc gac atc acc ccc aag gac agc 2928
Tyr Asp Ser Lys Leu Gly Asn His Ile Asp Ile Thr Pro Lys Asp Ser
                965                 970                 975

aac aac aag gtg gtg ctg cag agc gtg agc ccc tgg cgc gcc gac gtg 2976
Asn Asn Lys Val Val Leu Gln Ser Val Ser Pro Trp Arg Ala Asp Val
            980                 985                 990

tac ttc aac aag acc acc ggc aag tac gag atc ctg ggc ctg aag tac 3024
Tyr Phe Asn Lys Thr Thr Gly Lys Tyr Glu Ile Leu Gly Leu Lys Tyr
        995                 1000                1005

gcc gac ctg cag ttt gat aag ggc acc ggc acc tac aag atc agc cag 3072
Ala Asp Leu Gln Phe Asp Lys Gly Thr Gly Thr Tyr Lys Ile Ser Gln
    1010                1015                1020

gag aag tac aac gac atc aag aag aag gag ggc gtg gac agc gac agc 3120
Glu Lys Tyr Asn Asp Ile Lys Lys Lys Glu Gly Val Asp Ser Asp Ser
1025                1030                1035                1040

gag ttc aag ttc acc ctg tac aag aac gac ctt ctg ctg gtg aag gac 3168
Glu Phe Lys Phe Thr Leu Tyr Lys Asn Asp Leu Leu Leu Val Lys Asp
                1045                1050                1055

acc gag acc aag gag caa cag ctg ttc cgc ttc ctg agc cgc acc atg 3216
Thr Glu Thr Lys Glu Gln Gln Leu Phe Arg Phe Leu Ser Arg Thr Met
            1060                1065                1070

ccc aag cag aag cac tac gtg gag ctg aag ccc tac gac aag cag aag 2364
Pro Lys Gln Lys His Tyr Val Glu Leu Lys Pro Tyr Asp Lys Gln Lys
        1075                1080                1085

ttc gag ggc ggc gag gcc ctg atc aag gtg ctg ggc aac gtg gcc aac 3312
Phe Glu Gly Gly Glu Ala Leu Ile Lys Val Leu Gly Asn Val Ala Asn
    1090                1095                1100

agc ggc cag tgc aag aag ggc ctg ggc aag agc aac atc agc atc tac 3360
Ser Gly Gln Cys Lys Lys Gly Leu Gly Lys Ser Asn Ile Ser Ile Tyr
1105                1110                1115                1120

aag gtg cgc acc gac gtg ctg ggc aac cag cac atc atc aag aac gag 3408
Lys Val Arg Thr Asp Val Leu Gly Asn Gln His Ile Ile Lys Asn Glu
                1125                1130                1135

ggc gac aag ccc aag ttg gac ttc agc agg gct gac ccc aag aag aag 3456
Gly Asp Lys Pro Lys Leu Asp Phe Ser Arg Ala Asp Pro Lys Lys Lys
            1140                1145                1150

agg aag gtg tga        3468
Arg Lys Val
        1155

<210> 12
<211> 4023
<212> DNA
<213> artificial
<400> SEQUENCE 12 (AsCpf1):
atg cat cat cac cac cac cat cac ggc agc ccc aag aag aag agg aag        48
Met His His His His His His His Gly Ser Pro Lys Lys Lys Arg Lys
1               5                   10                  15

gtg atg aca cag ttc gag ggc ttt acc aac ctg tat cag gtg agc aag        96
Val Met Thr Gln Phe Glu Gly Phe Thr Asn Leu Tyr Gln Val Ser Lys
            20                  25                  30

aca ctg cgg ttt gag ctg atc cca cag ggc aag acc ctg aag cac atc        144
Thr Leu Arg Phe Glu Leu Ile Pro Gln Gly Lys Thr Leu Lys His Ile
        35                  40                  45

cag gag cag ggc ttc atc gag gag gac aag gcc cgc aat gat cac tac        192
Gln Glu Gln Gly Phe Ile Glu Glu Asp Lys Ala Arg Asn Asp His Tyr
    50                  55                  60

aag gag ctg aag ccc atc atc gat cgg atc tac aag acc tat gcc gac        240
Lys Glu Leu Lys Pro Ile Ile Asp Arg Ile Tyr Lys Thr Tyr Ala Asp
65                  70                  75                  80

cag tgc ctg cag ctg gtg cag ctg gat tgg gag aac ctg agc gcc gcc        288
Gln Cys Leu Gln Leu Val Gln Leu Asp Trp Glu Asn Leu Ser Ala Ala
                85                  90                  95

atc gac tcc tat aga aag gag aaa acc gag gag aca agg aac gcc ctg        336
Ile Asp Ser Tyr Arg Lys Glu Lys Thr Glu Glu Thr Arg Asn Ala Leu
            100                 105                 110

atc gag gag cag gcc aca tat cgc aat gcc atc cac gac tac ttc atc        384
Ile Glu Glu Gln Ala Thr Tyr Arg Asn Ala Ile His Asp Tyr Phe Ile
        115                 120                 125

ggc cgg aca gac aac ctg acc gat gcc atc aat aag aga cac gcc gag        432
Gly Arg Thr Asp Asn Leu Thr Asp Ala Ile Asn Lys Arg His Ala Glu
    130                 135                 140

atc tac aag ggc ctg ttc aag gcc gag ctg ttt aat ggc aag gtg ctg        480
Ile Tyr Lys Gly Leu Phe Lys Ala Glu Leu Phe Asn Gly Lys Val Leu
145                 150                 155                 160

aag cag ctg ggc acc gtg acc aca acc gag cac gag aac gcc ctg ctg        528
Lys Gln Leu Gly Thr Val Thr Thr Thr Glu His Glu Asn Ala Leu Leu
                165                 170                 175

cgg agc ttc gac aag ttt aca acc tac ttc tcc ggc ttt tat gag aac        576
Arg Ser Phe Asp Lys Phe Thr Thr Tyr Phe Ser Gly Phe Tyr Glu Asn
            180                 185                 190

agg aag aac gtg ttc agc gcc gag gat atc agc aca gcc atc cca cac        624
Arg Lys Asn Val Phe Ser Ala Glu Asp Ile Ser Thr Ala Ile Pro His

195                 200                 205

cgc atc gtg cag gac aac ttc ccc aag ttt aag gag aat tgt cac atc          672
Arg Ile Val Gln Asp Asn Phe Pro Lys Phe Lys Glu Asn Cys His Ile
    210                 215                 220

ttc aca cgc ctg atc acc gcc gtg ccc agc ctg cgg gag cac ttt gag          720
Phe Thr Arg Leu Ile Thr Ala Val Pro Ser Leu Arg Glu His Phe Glu
225                 230                 235                 240

aac gtg aag aag gcc atc ggc atc ttc gtg agc acc tcc atc gag gag          768
Asn Val Lys Lys Ala Ile Gly Ile Phe Val Ser Thr Ser Ile Glu Glu
                245                 250                 255

gtg ttt tcc ttc cct ttt tat aac cag ctg ctg aca cag acc cag atc          816
Val Phe Ser Phe Pro Phe Tyr Asn Gln Leu Leu Thr Gln Thr Gln Ile
            260                 265                 270

gac ctg tat aac cag ctg ctg gga gga atc tct cgg gag gca ggc acc          864
Asp Leu Tyr Asn Gln Leu Leu Gly Gly Ile Ser Arg Glu Ala Gly Thr
        275                 280                 285

gag aag atc aag ggc ctg aac gag gtg ctg aat ctg gcc atc cag aag          912
Glu Lys Ile Lys Gly Leu Asn Glu Val Leu Asn Leu Ala Ile Gln Lys
    290                 295                 300

aat gat gag aca gcc cac atc atc gcc tcc ctg cca cac aga ttc atc          960
Asn Asp Glu Thr Ala His Ile Ile Ala Ser Leu Pro His Arg Phe Ile
305                 310                 315                 320

ccc ctg ttt aag cag atc ctg tcc gat agg aac acc ctg tct ttc atc          1008
Pro Leu Phe Lys Gln Ile Leu Ser Asp Arg Asn Thr Leu Ser Phe Ile
                325                 330                 335

ctg gag gag ttt aag agc gac gag gaa gtg atc cag tcc ttc tgc aag          1056
Leu Glu Glu Phe Lys Ser Asp Glu Glu Val Ile Gln Ser Phe Cys Lys
            340                 345                 350

tac aag aca ctg ctg aga aac gag aac gtg ctg gag aca gcc gag gcc          1104
Tyr Lys Thr Leu Leu Arg Asn Glu Asn Val Leu Glu Thr Ala Glu Ala
        355                 360                 365

ctg ttt aac gag ctg aac agc atc gac ctg aca cac atc ttc atc agc          1152
Leu Phe Asn Glu Leu Asn Ser Ile Asp Leu Thr His Ile Phe Ile Ser
    370                 375                 380

cac aag aag ctg gag aca atc agc agc gcc ctg tgc gac cac tgg gat          1200
His Lys Lys Leu Glu Thr Ile Ser Ser Ala Leu Cys Asp His Trp Asp
385                 390                 395                 400

aca ctg agg aat gcc ctg tat gag cgg aga atc tcc gag ctg aca ggc          1248
Thr Leu Arg Asn Ala Leu Tyr Glu Arg Arg Ile Ser Glu Leu Thr Gly
                405                 410                 415

aag atc acc aag tct gcc aag gag aag gtg cag cgc agc ctg aag cac          1296
Lys Ile Thr Lys Ser Ala Lys Glu Lys Val Gln Arg Ser Leu Lys His
            420                 425                 430

gag gat atc aac ctg cag gag atc atc tct gcc gca ggc aag gag ctg          1344
Glu Asp Ile Asn Leu Gln Glu Ile Ile Ser Ala Ala Gly Lys Glu Leu
        435                 440                 445

agc gag gcc ttc aag cag aaa acc agc gag atc ctg tcc cac gca cac          1392

Ser Glu Ala Phe Lys Gln Lys Thr Ser Glu Ile Leu Ser His Ala His
    450                 455                 460

gcc gcc ctg gat cag cca ctg cct aca acc ctg aag aag cag gag gag      1440
Ala Ala Leu Asp Gln Pro Leu Pro Thr Thr Leu Lys Lys Gln Glu Glu
465                 470                 475                 480

aag gag atc ctg aag tct cag ctg gac agc ctg ctg ggc ctg tac cac      1488
Lys Glu Ile Leu Lys Ser Gln Leu Asp Ser Leu Leu Gly Leu Tyr His
                485                 490                 495

ctg ctg gac tgg ttt gcc gtg gat gag tcc aac gag gtg gac ccc gag      1536
Leu Leu Asp Trp Phe Ala Val Asp Glu Ser Asn Glu Val Asp Pro Glu
            500                 505                 510

ttc tct gcc cgg ctg acc ggc atc aag ctg gag atg gag cct tct ctg      1584
Phe Ser Ala Arg Leu Thr Gly Ile Lys Leu Glu Met Glu Pro Ser Leu
        515                 520                 525

agc ttc tac aac aag gcc aga aat tat gcc acc aag aag ccc tac tcc      1632
Ser Phe Tyr Asn Lys Ala Arg Asn Tyr Ala Thr Lys Lys Pro Tyr Ser
    530                 535                 540

gtg gag aag ttc aag ctg aac ttt cag atg cct aca ctg gcc tct ggc      1680
Val Glu Lys Phe Lys Leu Asn Phe Gln Met Pro Thr Leu Ala Ser Gly
545                 550                 555                 560

tgg gac gtg aat aag gag aag aac aat ggc gcc atc ctg ttt gtg aag      1728
Trp Asp Val Asn Lys Glu Lys Asn Asn Gly Ala Ile Leu Phe Val Lys
                565                 570                 575

aac ggc ctg tac tat ctg ggc atc atg cca aag cag aag ggc agg tat      1776
Asn Gly Leu Tyr Tyr Leu Gly Ile Met Pro Lys Gln Lys Gly Arg Tyr
            580                 585                 590

aag gcc ctg agc ttc gag ccc aca gag aaa acc agc gag ggc ttt gat      1824
Lys Ala Leu Ser Phe Glu Pro Thr Glu Lys Thr Ser Glu Gly Phe Asp
        595                 600                 605

aag atg tac tat gac tac ttc cct gat gcc gcc aag atg atc cca aag      1872
Lys Met Tyr Tyr Asp Tyr Phe Pro Asp Ala Ala Lys Met Ile Pro Lys
    610                 615                 620

tgc agc acc cag ctg aag gcc gtg aca gcc cac ttt cag acc cac aca      1920
Cys Ser Thr Gln Leu Lys Ala Val Thr Ala His Phe Gln Thr His Thr
625                 630                 635                 640

acc ccc atc ctg ctg tcc aac aat ttc atc gag cct ctg gag atc aca      1968
Thr Pro Ile Leu Leu Ser Asn Asn Phe Ile Glu Pro Leu Glu Ile Thr
                645                 650                 655

aag gag atc tac gac ctg aac aat cct gag aag gag cca aag aag ttt      2016
Lys Glu Ile Tyr Asp Leu Asn Asn Pro Glu Lys Glu Pro Lys Lys Phe
            660                 665                 670

cag aca gcc tac gcc aag aaa acc ggc gac cag aag ggc tac aga gag      2064
Gln Thr Ala Tyr Ala Lys Lys Thr Gly Asp Gln Lys Gly Tyr Arg Glu
        675                 680                 685

gcc ctg tgc aag tgg atc gac ttc aca agg gat ttt ctg tcc aag tat      2112
Ala Leu Cys Lys Trp Ile Asp Phe Thr Arg Asp Phe Leu Ser Lys Tyr

690                 695                 700

acc aag aca acc tct atc gat ctg tct agc ctg cgg cca tcc tct cag          2160
Thr Lys Thr Thr Ser Ile Asp Leu Ser Ser Leu Arg Pro Ser Ser Gln
705                 710                 715                 720

tat aag gac ctg ggc gag tac tat gcc gag ctg aat ccc ctg ctg tac          2208
Tyr Lys Asp Leu Gly Glu Tyr Tyr Ala Glu Leu Asn Pro Leu Leu Tyr
                725                 730                 735

cac atc agc ttc cag aga atc gcc gag aag gag atc atg gat gcc gtg          2256
His Ile Ser Phe Gln Arg Ile Ala Glu Lys Glu Ile Met Asp Ala Val
            740                 745                 750

gag aca ggc aag ctg tac ctg ttc cag atc tat aac aag gac ttt gcc          2304
Glu Thr Gly Lys Leu Tyr Leu Phe Gln Ile Tyr Asn Lys Asp Phe Ala
        755                 760                 765

aag ggc cac cac ggc aag cct aat ctg cac aca ctg tat tgg acc ggc          2352
Lys Gly His His Gly Lys Pro Asn Leu His Thr Leu Tyr Trp Thr Gly
    770                 775                 780

ctg ttt tct cca gag aac ctg gcc aag aca agc atc aag ctg aat ggc          2400
Leu Phe Ser Pro Glu Asn Leu Ala Lys Thr Ser Ile Lys Leu Asn Gly
785                 790                 795                 800

cag gcc gag ctg ttc tac cgc cct aag tcc agg atg aag agg atg gca          2448
Gln Ala Glu Leu Phe Tyr Arg Pro Lys Ser Arg Met Lys Arg Met Ala
                805                 810                 815

cac cgg ctg gga gag aag atg ctg aac aag aag ctg aag gat cag aaa          2496
His Arg Leu Gly Glu Lys Met Leu Asn Lys Lys Leu Lys Asp Gln Lys
            820                 825                 830

acc cca atc ccc gac acc ctg tac cag gag ctg tac gac tat gtg aat          2544
Thr Pro Ile Pro Asp Thr Leu Tyr Gln Glu Leu Tyr Asp Tyr Val Asn
        835                 840                 845

cac aga ctg tcc cac gac ctg tct gat gag gcc agg gcc ctg ctg ccc          2592
His Arg Leu Ser His Asp Leu Ser Asp Glu Ala Arg Ala Leu Leu Pro
    850                 855                 860

aac gtg atc acc aag gag gtg tct cac gag atc atc aag gat agg cgc          2640
Asn Val Ile Thr Lys Glu Val Ser His Glu Ile Ile Lys Asp Arg Arg
865                 870                 875                 880

ttt acc agc gac aag ttc ttt ttc cac gtg cct atc aca ctg aac tat          2688
Phe Thr Ser Asp Lys Phe Phe Phe His Val Pro Ile Thr Leu Asn Tyr
                885                 890                 895

cag gcc gcc aat tcc cca tct aag ttc aac cag agg gtg aat gcc tac          2736
Gln Ala Ala Asn Ser Pro Ser Lys Phe Asn Gln Arg Val Asn Ala Tyr
            900                 905                 910

ctg aag gag cac ccc gag aca cct atc atc ggc atc gat cgg ggc gag          2784
Leu Lys Glu His Pro Glu Thr Pro Ile Ile Gly Ile Asp Arg Gly Glu
        915                 920                 925

aga aac ctg atc tat atc aca gtg atc gac tcc acc ggc aag atc ctg          2832
Arg Asn Leu Ile Tyr Ile Thr Val Ile Asp Ser Thr Gly Lys Ile Leu
    930                 935                 940

gag cag cgg agc ctg aac acc atc cag cag ttt gat tac cag aag aag          2880

Glu Gln Arg Ser Leu Asn Thr Ile Gln Gln Phe Asp Tyr Gln Lys Lys
945                 950                 955                 960

ctg gac aac agg gag aag gag agg gtg gca gca agg cag gcc tgg tct      2928
Leu Asp Asn Arg Glu Lys Glu Arg Val Ala Ala Arg Gln Ala Trp Ser
                965                 970                 975

gtg gtg ggc aca atc aag gat ctg aag cag ggc tat ctg agc cag gtc      2976
Val Val Gly Thr Ile Lys Asp Leu Lys Gln Gly Tyr Leu Ser Gln Val
            980                 985                 990

atc cac gag atc gtg gac ctg atg atc cac tac cag gcc gtg gtg gtg      3024
Ile His Glu Ile Val Asp Leu Met Ile His Tyr Gln Ala Val Val Val
        995                 1000                1005

ctg gag aac ctg aat ttc ggc ttt aag agc aag agg acc ggc atc gcc      3072
Leu Glu Asn Leu Asn Phe Gly Phe Lys Ser Lys Arg Thr Gly Ile Ala
    1010                1015                1020

gag aag gcc gtg tac cag cag ttc gag aag atg ctg atc gat aag ctg      3120
Glu Lys Ala Val Tyr Gln Gln Phe Glu Lys Met Leu Ile Asp Lys Leu
1025                1030                1035                1040

aat tgc ctg gtg ctg aag gac tat cca gca gag aaa gtg gga ggc gtg      3168
Asn Cys Leu Val Leu Lys Asp Tyr Pro Ala Glu Lys Val Gly Gly Val
                1045                1050                1055

ctg aac cca tac cag ctg aca gac cag ttc acc tcc ttt gcc aag atg      3216
Leu Asn Pro Tyr Gln Leu Thr Asp Gln Phe Thr Ser Phe Ala Lys Met
            1060                1065                1070

ggc acc cag tct ggc ttc ctg ttt tac gtg cct gcc cca tat aca tct      3264
Gly Thr Gln Ser Gly Phe Leu Phe Tyr Val Pro Ala Pro Tyr Thr Ser
        1075                1080                1085

aag atc gat ccc ctg acc ggc ttc gtg gac ccc ttc gtg tgg aaa acc      3312
Lys Ile Asp Pro Leu Thr Gly Phe Val Asp Pro Phe Val Trp Lys Thr
    1090                1095                1100

atc aag aat cac gag agc cgc aag cac ttc ctg gag ggc ttc gac ttt      3360
Ile Lys Asn His Glu Ser Arg Lys His Phe Leu Glu Gly Phe Asp Phe
1105                1110                1115                1120

ctg cac tac gac gtg aaa acc ggc gac ttc atc ctg cac ttt aag atg      3408
Leu His Tyr Asp Val Lys Thr Gly Asp Phe Ile Leu His Phe Lys Met
                1125                1130                1135

aac aga aat ctg tcc ttc cag agg ggc ctg ccc ggc ttt atg cct gca      3456
Asn Arg Asn Leu Ser Phe Gln Arg Gly Leu Pro Gly Phe Met Pro Ala
            1140                1145                1150

tgg gat atc gtg ttc gag aag aac gag aca cag ttt gac gcc aag ggc      3504
Trp Asp Ile Val Phe Glu Lys Asn Glu Thr Gln Phe Asp Ala Lys Gly
        1155                1160                1165

acc cct ttc atc gcc ggc aag aga atc gtg cca gtg atc gag aat cac      3552
Thr Pro Phe Ile Ala Gly Lys Arg Ile Val Pro Val Ile Glu Asn His
    1170                1175                1180

aga ttc acc ggc aga tac cgg gac ctg tat cct gcc aac gag ctg atc      3600
Arg Phe Thr Gly Arg Tyr Arg Asp Leu Tyr Pro Ala Asn Glu Leu Ile

1185                1190                1195                1200

gcc ctg ctg gag gag aag ggc atc gtg ttc agg gat ggc tcc aac atc        3648
Ala Leu Leu Glu Glu Lys Gly Ile Val Phe Arg Asp Gly Ser Asn Ile
                1205                1210                1215

ctg cca aag ctg ctg gag aat gac gat tct cac gcc atc gac acc atg        3696
Leu Pro Lys Leu Leu Glu Asn Asp Asp Ser His Ala Ile Asp Thr Met
            1220                1225                1230

gtg gcc ctg atc cgc agc gtg ctg cag atg cgg aac tcc aat gcc gcc        3744
Val Ala Leu Ile Arg Ser Val Leu Gln Met Arg Asn Ser Asn Ala Ala
        1235                1240                1245

aca ggc gag gac tat atc aac agc ccc gtg cgc gat ctg aat ggc gtg        3792
Thr Gly Glu Asp Tyr Ile Asn Ser Pro Val Arg Asp Leu Asn Gly Val
    1250                1255                1260

tgc ttc gac tcc cgg ttt cag aac cca gag tgg ccc atg gac gcc gat        3840
Cys Phe Asp Ser Arg Phe Gln Asn Pro Glu Trp Pro Met Asp Ala Asp
1265                1270                1275                1280

gcc aat ggc gcc tac cac atc gcc ctg aag ggc cag ctg ctg ctg aat        3888
Ala Asn Gly Ala Tyr His Ile Ala Leu Lys Gly Gln Leu Leu Leu Asn
                1285                1290                1295

cac ctg aag gag agc aag gat ctg aag ctg cag aac ggc atc tcc aat        3936
His Leu Lys Glu Ser Lys Asp Leu Lys Leu Gln Asn Gly Ile Ser Asn
            1300                1305                1310

cag gac tgg ctg gcc tac atc cag gag ctg cgc aac aaa agg ccg gcg        3984
Gln Asp Trp Leu Ala Tyr Ile Gln Glu Leu Arg Asn Lys Arg Pro Ala
        1315                1320                1325

gcc acg aaa aag gcc ggc cag gca aaa aag aaa aag tga    4023
Ala Thr Lys Lys Ala Gly Gln Ala Lys Lys Lys Lys
    1330                1335                1340

<210> 13
<211> 4233
<212> DNA
<213> artificial
<400> SEQUENCE 13 (TATSPCas9):

atg cat ggc cgc aaa aaa cgc cgc cag cgc cgc cgc ccg cag ggc agc        48
Met His Gly Arg Lys Lys Arg Arg Gln Arg Arg Arg Pro Gln Gly Ser
  1               5                  10                  15

cat cac cac cac cat cac ggc agc ccc aag aag aag agg aag gtg atg        96
His His His His His His Gly Ser Pro Lys Lys Lys Arg Lys Val Met
            20                  25                  30

gac aag aag tac tcc att ggg ctc gat atc ggc aca aac agc gtc ggc        144
Asp Lys Lys Tyr Ser Ile Gly Leu Asp Ile Gly Thr Asn Ser Val Gly
        35                  40                  45

tgg gcc gtc att acg gac gag tac aag gtg ccg agc aaa aaa ttc aaa        192
Trp Ala Val Ile Thr Asp Glu Tyr Lys Val Pro Ser Lys Lys Phe Lys
    50                  55                  60

gtt ctg ggc aat acc gat cgc cac agc ata aag aag aac ctc att ggc        240
Val Leu Gly Asn Thr Asp Arg His Ser Ile Lys Lys Asn Leu Ile Gly
65                  70                  75                  80

gcc ctc ctg ttc gac tcc ggg gag acg gcc gaa gcc acg cgg ctc aaa          288
Ala Leu Leu Phe Asp Ser Gly Glu Thr Ala Glu Ala Thr Arg Leu Lys
                    85                  90                  95

aga aca gca cgg cgc aga tat acc cgc aga aag aat cgg atc tgc tac          336
Arg Thr Ala Arg Arg Arg Tyr Thr Arg Arg Lys Asn Arg Ile Cys Tyr
            100                 105                 110

ctg cag gag atc ttt agt aat gag atg gct aag gtg gat gac tct ttc          384
Leu Gln Glu Ile Phe Ser Asn Glu Met Ala Lys Val Asp Asp Ser Phe
        115                 120                 125

ttc cat agg ctg gag gag tcc ttt ttg gtg gag gag gat aaa aag cac          432
Phe His Arg Leu Glu Glu Ser Phe Leu Val Glu Glu Asp Lys Lys His
    130                 135                 140

gag cgc cac cca atc ttt ggc aat atc gtg gac gag gtg gcg tac cat          480
Glu Arg His Pro Ile Phe Gly Asn Ile Val Asp Glu Val Ala Tyr His
145                 150                 155                 160

gaa aag tac cca acc ata tat cat ctg agg aag aag ctt gta gac agt          528
Glu Lys Tyr Pro Thr Ile Tyr His Leu Arg Lys Lys Leu Val Asp Ser
                165                 170                 175

act gat aag gct gac ttg cgg ttg atc tat ctc gcg ctg gcg cat atg          576
Thr Asp Lys Ala Asp Leu Arg Leu Ile Tyr Leu Ala Leu Ala His Met
            180                 185                 190

atc aaa ttt cgg gga cac ttc ctc atc gag ggg gac ctg aac cca gac          624
Ile Lys Phe Arg Gly His Phe Leu Ile Glu Gly Asp Leu Asn Pro Asp
        195                 200                 205

aac agc gat gtc gac aaa ctc ttt atc caa ctg gtt cag act tac aat          672
Asn Ser Asp Val Asp Lys Leu Phe Ile Gln Leu Val Gln Thr Tyr Asn
    210                 215                 220

cag ctt ttc gaa gag aac ccg atc aac gca tcc gga gtt gac gcc aaa          720
Gln Leu Phe Glu Glu Asn Pro Ile Asn Ala Ser Gly Val Asp Ala Lys
225                 230                 235                 240

gca atc ctg agc gct agg ctg tcc aaa tcc cgg cgg ctc gaa aac ctc          768
Ala Ile Leu Ser Ala Arg Leu Ser Lys Ser Arg Arg Leu Glu Asn Leu
                245                 250                 255

atc gca cag ctc cct ggg gag aag aag aac ggc ctg ttt ggt aat ctt          816
Ile Ala Gln Leu Pro Gly Glu Lys Lys Asn Gly Leu Phe Gly Asn Leu
            260                 265                 270

atc gcc ctg tca ctc ggg ctg acc ccc aac ttt aaa tct aac ttc gac          864
Ile Ala Leu Ser Leu Gly Leu Thr Pro Asn Phe Lys Ser Asn Phe Asp
        275                 280                 285

ctg gcc gaa gat gcc aag ctt caa ctg agc aaa gac acc tac gat gat          912
Leu Ala Glu Asp Ala Lys Leu Gln Leu Ser Lys Asp Thr Tyr Asp Asp
    290                 295                 300

gat ctc gac aat ctg ctg gcc cag atc ggc gac cag tac gca gac ctt          960
Asp Leu Asp Asn Leu Leu Ala Gln Ile Gly Asp Gln Tyr Ala Asp Leu
305                 310                 315                 320

ttt ttg gcg gca aag aac ctg tca gac gcc att ctg ctg agt gat att 1008
Phe Leu Ala Ala Lys Asn Leu Ser Asp Ala Ile Leu Leu Ser Asp Ile
                325                 330                 335

ctg cga gtg aac acg gag atc acc aaa gct ccg ctg agc gct agt atg 1056
Leu Arg Val Asn Thr Glu Ile Thr Lys Ala Pro Leu Ser Ala Ser Met
            340                 345                 350

atc aag cgc tat gat gag cac cac caa gac ttg act ttg ctg aag gcc 1104
Ile Lys Arg Tyr Asp Glu His His Gln Asp Leu Thr Leu Leu Lys Ala
        355                 360                 365

ctt gtc aga cag caa ctg cct gag aag tac aag gaa att ttc ttc gat 1152
Leu Val Arg Gln Gln Leu Pro Glu Lys Tyr Lys Glu Ile Phe Phe Asp
    370                 375                 380

cag tct aaa aat ggc tac gcc gga tac att gac ggc gga gca agc cag 1200
Gln Ser Lys Asn Gly Tyr Ala Gly Tyr Ile Asp Gly Gly Ala Ser Gln
385                 390                 395                 400

gag gaa ttt tac aaa ttt att aag ccc atc ttg gaa aaa atg gac ggc 1248
Glu Glu Phe Tyr Lys Phe Ile Lys Pro Ile Leu Glu Lys Met Asp Gly
                405                 410                 415

acc gag gag ctg ctg gta aag ctt aac aga gaa gat ctg ttg cgc aaa 1296
Thr Glu Glu Leu Leu Val Lys Leu Asn Arg Glu Asp Leu Leu Arg Lys
            420                 425                 430

cag cgc act ttc gac aat gga agc atc ccc cac cag att cac ctg ggc 1344
Gln Arg Thr Phe Asp Asn Gly Ser Ile Pro His Gln Ile His Leu Gly
        435                 440                 445

gaa ctg cac gct atc ctc agg cgg caa gag gat ttc tac ccc ttt ttg 1392
Glu Leu His Ala Ile Leu Arg Arg Gln Glu Asp Phe Tyr Pro Phe Leu
    450                 455                 460

aaa gat aac agg gaa aag att gag aaa atc ctc aca ttt cgg ata ccc 1440
Lys Asp Asn Arg Glu Lys Ile Glu Lys Ile Leu Thr Phe Arg Ile Pro
465                 470                 475                 480

tac tat gta ggc ccc ctc gcc cgg gga aat tcc aga ttc gcg tgg atg 1488
Tyr Tyr Val Gly Pro Leu Ala Arg Gly Asn Ser Arg Phe Ala Trp Met
                485                 490                 495

act cgc aaa tca gaa gag acc atc act ccc tgg aac ttc gag gaa gtc 1536
Thr Arg Lys Ser Glu Glu Thr Ile Thr Pro Trp Asn Phe Glu Glu Val
            500                 505                 510

gtg gat aag ggg gcc tct gcc cag tcc ttc atc gaa agg atg act aac 1584
Val Asp Lys Gly Ala Ser Ala Gln Ser Phe Ile Glu Arg Met Thr Asn
        515                 520                 525

ttt gat aaa aat ctg cct aac gaa aag gtg ctt cct aaa cac tct ctg 1632
Phe Asp Lys Asn Leu Pro Asn Glu Lys Val Leu Pro Lys His Ser Leu
    530                 535                 540

ctg tac gag tac ttc aca gtt tat aac gag ctc acc aag gtc aaa tac 1680
Leu Tyr Glu Tyr Phe Thr Val Tyr Asn Glu Leu Thr Lys Val Lys Tyr
545                 550                 555                 560

gtc aca gaa ggg atg aga aag cca gca ttc ctg tct gga gag cag aag 1728
Val Thr Glu Gly Met Arg Lys Pro Ala Phe Leu Ser Gly Glu Gln Lys
                565                 570                 575

aaa gct atc gtg gac ctc ctc ttc aag acg aac cgg aaa gtt acc gtg          1776
Lys Ala Ile Val Asp Leu Leu Phe Lys Thr Asn Arg Lys Val Thr Val
                580                 585                 590

aaa cag ctc aaa gaa gac tat ttc aaa aag att gaa tgt ttc gac tct          1824
Lys Gln Leu Lys Glu Asp Tyr Phe Lys Lys Ile Glu Cys Phe Asp Ser
        595                 600                 605

gtt gaa atc agc gga gtg gag gat cgc ttc aac gca tcc ctg gga acg          1872
Val Glu Ile Ser Gly Val Glu Asp Arg Phe Asn Ala Ser Leu Gly Thr
    610                 615                 620

tat cac gat ctc ctg aaa atc att aaa gac aag gac ttc ctg gac aat          1920
Tyr His Asp Leu Leu Lys Ile Ile Lys Asp Lys Asp Phe Leu Asp Asn
625                 630                 635                 640

gag gag aac gag gac att ctt gag gac att gtc ctc acc ctt acg ttg          1968
Glu Glu Asn Glu Asp Ile Leu Glu Asp Ile Val Leu Thr Leu Thr Leu
                645                 650                 655

ttt gaa gat agg gag atg att gaa gaa cgc ttg aaa act tac gct cat          2016
Phe Glu Asp Arg Glu Met Ile Glu Glu Arg Leu Lys Thr Tyr Ala His
            660                 665                 670

ctc ttc gac gac aaa gtc atg aaa cag ctc aag agg cgc cga tat aca          2064
Leu Phe Asp Asp Lys Val Met Lys Gln Leu Lys Arg Arg Arg Tyr Thr
        675                 680                 685

gga tgg ggg cgg ctg tca aga aaa ctg atc aat ggg atc cga gac aag          2112
Gly Trp Gly Arg Leu Ser Arg Lys Leu Ile Asn Gly Ile Arg Asp Lys
    690                 695                 700

cag agt gga aag aca atc ctg gat ttt ctt aag tcc gat gga ttt gcc          2160
Gln Ser Gly Lys Thr Ile Leu Asp Phe Leu Lys Ser Asp Gly Phe Ala
705                 710                 715                 720

aac cgg aac ttc atg cag ttg atc cat gat gac tct ctc acc ttt aag          2208
Asn Arg Asn Phe Met Gln Leu Ile His Asp Asp Ser Leu Thr Phe Lys
                725                 730                 735

gag gac atc cag aaa gca caa gtt tct ggc cag ggg gac agt ctt cac          2256
Glu Asp Ile Gln Lys Ala Gln Val Ser Gly Gln Gly Asp Ser Leu His
            740                 745                 750

gag cac atc gct aat ctt gca ggt agc cca gct atc aaa aag gga ata          2304
Glu His Ile Ala Asn Leu Ala Gly Ser Pro Ala Ile Lys Lys Gly Ile
        755                 760                 765

ctg cag acc gtt aag gtc gtg gat gaa ctc gtc aaa gta atg gga agg          2352
Leu Gln Thr Val Lys Val Val Asp Glu Leu Val Lys Val Met Gly Arg
    770                 775                 780

cat aag ccc gag aat atc gtt atc gag atg gcc cga gag aac caa act          2400
His Lys Pro Glu Asn Ile Val Ile Glu Met Ala Arg Glu Asn Gln Thr
785                 790                 795                 800

acc cag aag gga cag aag aac agt agg gaa agg atg aag agg att gaa          2448
Thr Gln Lys Gly Gln Lys Asn Ser Arg Glu Arg Met Lys Arg Ile Glu
                805                 810                 815

gag ggt ata aaa gaa ctg ggg tcc caa atc ctt aag gaa cac cca gtt 2496
Glu Gly Ile Lys Glu Leu Gly Ser Gln Ile Leu Lys Glu His Pro Val
            820                 825                 830

gaa aac acc cag ctt cag aat gag aag ctc tac ctg tac tac ctg cag 2544
Glu Asn Thr Gln Leu Gln Asn Glu Lys Leu Tyr Leu Tyr Tyr Leu Gln
        835                 840                 845

aac ggc agg gac atg tac gtg gat cag gaa ctg gac atc aat cgg ctc 2592
Asn Gly Arg Asp Met Tyr Val Asp Gln Glu Leu Asp Ile Asn Arg Leu
    850                 855                 860

tcc gac tac gac gtg gat cat atc gtg ccc cag tct ttt ctc aaa gat 2640
Ser Asp Tyr Asp Val Asp His Ile Val Pro Gln Ser Phe Leu Lys Asp
865                 870                 875                 880

gat tct att gat aat aaa gtg ttg aca aga tcc gat aaa aat aga ggg 2688
Asp Ser Ile Asp Asn Lys Val Leu Thr Arg Ser Asp Lys Asn Arg Gly
                885                 890                 895

aag agt gat aac gtc ccc tca gaa gaa gtt gtc aag aaa atg aaa aat 2736
Lys Ser Asp Asn Val Pro Ser Glu Glu Val Val Lys Lys Met Lys Asn
            900                 905                 910

tat tgg cgg cag ctg ctg aac gcc aaa ctg atc aca caa cgg aag ttc 2784
Tyr Trp Arg Gln Leu Leu Asn Ala Lys Leu Ile Thr Gln Arg Lys Phe
        915                 920                 925

gat aat ctg act aag gct gaa cga ggt ggc ctg tct gag ttg gat aaa 2832
Asp Asn Leu Thr Lys Ala Glu Arg Gly Gly Leu Ser Glu Leu Asp Lys
    930                 935                 940

gcc ggc ttc atc aaa agg cag ctt gtt gag aca cgc cag atc acc aag 2880
Ala Gly Phe Ile Lys Arg Gln Leu Val Glu Thr Arg Gln Ile Thr Lys
945                 950                 955                 960

cac gtg gcc caa att ctc gat tca cgc atg aac acc aag tac gat gaa 2928
His Val Ala Gln Ile Leu Asp Ser Arg Met Asn Thr Lys Tyr Asp Glu
                965                 970                 975

aat gac aaa ctg att cga gag gtg aaa gtt att act ctg aag tct aag 2976
Asn Asp Lys Leu Ile Arg Glu Val Lys Val Ile Thr Leu Lys Ser Lys
            980                 985                 990

ctg gtc tca gat ttc aga aag gac ttt cag ttt tat aag gtg aga gag 3024
Leu Val Ser Asp Phe Arg Lys Asp Phe Gln Phe Tyr Lys Val Arg Glu
        995                 1000                1005

atc aac aat tac cac cat gcg cat gat gcc tac ctg aat gca gtg gta 3072
Ile Asn Asn Tyr His His Ala His Asp Ala Tyr Leu Asn Ala Val Val
    1010                1015                1020

ggc act gca ctt atc aaa aaa tat ccc aag ctt gaa tct gaa ttt gtt 3120
Gly Thr Ala Leu Ile Lys Lys Tyr Pro Lys Leu Glu Ser Glu Phe Val
1025                1030                1035                1040

tac gga gac tat aaa gtg tac gat gtt agg aaa atg atc gca aag tct 3168
Tyr Gly Asp Tyr Lys Val Tyr Asp Val Arg Lys Met Ile Ala Lys Ser
                1045                1050                1055

gag cag gaa ata ggc aag gcc acc gct aag tac ttc ttt tac agc aat 3216
Glu Gln Glu Ile Gly Lys Ala Thr Ala Lys Tyr Phe Phe Tyr Ser Asn
            1060                1065                1070

att atg aat ttt ttc aag acc gag att aca ctg gcc aat gga gag att 3264
Ile Met Asn Phe Phe Lys Thr Glu Ile Thr Leu Ala Asn Gly Glu Ile
        1075                1080                1085

cgg aag cga cca ctt atc gaa aca aac gga gaa aca gga gaa atc gtg 3312
Arg Lys Arg Pro Leu Ile Glu Thr Asn Gly Glu Thr Gly Glu Ile Val
    1090                1095                1100

tgg gac aag ggt agg gat ttc gcg aca gtc cgg aag gtc ctg tcc atg 3360
Trp Asp Lys Gly Arg Asp Phe Ala Thr Val Arg Lys Val Leu Ser Met
1105                1110                1115                1120

ccg cag gtg aac atc gtt aaa aag acc gaa gta cag acc gga ggc ttc 3408
Pro Gln Val Asn Ile Val Lys Lys Thr Glu Val Gln Thr Gly Gly Phe
                1125                1130                1135

tcc aag gaa agt atc ctc ccg aaa agg aac agc gac aag ctg atc gca 3456
Ser Lys Glu Ser Ile Leu Pro Lys Arg Asn Ser Asp Lys Leu Ile Ala
            1140                1145                1150

cgc aaa aaa gat tgg gac ccc aag aaa tac ggc gga ttc gat tct cct 3504
Arg Lys Lys Asp Trp Asp Pro Lys Lys Tyr Gly Gly Phe Asp Ser Pro
        1155                1160                1165

aca gtc gct tac agt gta ctg gtt gtg gcc aaa gtg gag aaa ggg aag 2552
Thr Val Ala Tyr Ser Val Leu Val Val Ala Lys Val Glu Lys Gly Lys
    1170                1175                1180

tct aaa aaa ctc aaa agc gtc aag gaa ctg ctg ggc atc aca atc atg 3600
Ser Lys Lys Leu Lys Ser Val Lys Glu Leu Leu Gly Ile Thr Ile Met
1185                1190                1195                1200

gag cga tca agc ttc gaa aaa aac ccc atc gac ttt ctc gag gcg aaa 3648
Glu Arg Ser Ser Phe Glu Lys Asn Pro Ile Asp Phe Leu Glu Ala Lys
                1205                1210                1215

gga tat aaa gag gtc aaa aaa gac ctc atc att aag ctt ccc aag tac 3696
Gly Tyr Lys Glu Val Lys Lys Asp Leu Ile Ile Lys Leu Pro Lys Tyr
            1220                1225                1230

tct ctc ttt gag ctt gaa aac ggc cgg aaa cga atg ctc gct agt gcg 3744
Ser Leu Phe Glu Leu Glu Asn Gly Arg Lys Arg Met Leu Ala Ser Ala
        1235                1240                1245

ggc gag ctg cag aaa ggt aac gag ctg gca ctg ccc tct aaa tac gtt 3792
Gly Glu Leu Gln Lys Gly Asn Glu Leu Ala Leu Pro Ser Lys Tyr Val
    1250                1255                1260

aat ttc ttg tat ctg gcc agc cac tat gaa aag ctc aaa ggg tct ccc 3840
Asn Phe Leu Tyr Leu Ala Ser His Tyr Glu Lys Leu Lys Gly Ser Pro
1265                1270                1275                1280

gaa gat aat gag cag aag cag ctg ttc gtg gaa caa cac aaa cac tac 3888
Glu Asp Asn Glu Gln Lys Gln Leu Phe Val Glu Gln His Lys His Tyr
                1285                1290                1295

ctt gat gag atc atc gag caa ata agc gaa ttc tcc aaa aga gtg atc 3936
Leu Asp Glu Ile Ile Glu Gln Ile Ser Glu Phe Ser Lys Arg Val Ile
            1300                1305                1310

ctc gcc gac gct aac ctc gat aag gtg ctt tct gct tac aat aag cac 3984
Leu Ala Asp Ala Asn Leu Asp Lys Val Leu Ser Ala Tyr Asn Lys His
        1315                1320                1325

agg gat aag ccc atc agg gag cag gca gaa aac att atc cac ttg ttt 4032
Arg Asp Lys Pro Ile Arg Glu Gln Ala Glu Asn Ile Ile His Leu Phe
    1330                1335                1340

act ctg acc aac ttg ggc gcg cct gca gcc ttc aag tac ttc gac acc 4080
Thr Leu Thr Asn Leu Gly Ala Pro Ala Ala Phe Lys Tyr Phe Asp Thr
1345                1350                1355                1360

acc ata gac aga aag cgg tac acc tct aca aag gag gtc ctg gac gcc 4128
Thr Ile Asp Arg Lys Arg Tyr Thr Ser Thr Lys Glu Val Leu Asp Ala
                1365                1370                1375

aca ctg att cat cag tca att acg ggg ctc tat gaa aca aga atc gac 4176
Thr Leu Ile His Gln Ser Ile Thr Gly Leu Tyr Glu Thr Arg Ile Asp
            1380                1385                1390

ctc tct cag ctc ggt gga gac agc agg gct gac ccc aag aag aag agg 4224
Leu Ser Gln Leu Gly Gly Asp Ser Arg Ala Asp Pro Lys Lys Lys Arg
        1395                1400                1405

aag gtg tga 4233
Lys Val
    1410

<210> 14
<211> 4233
<212> DNA
<213> artificial
<400> SEQUENCE 14 (SPCas9TAT):
atg cat cat cac cac cac cat cac ggc agc ccc aag aag aag agg aag 48
Met His His His His His His His Gly Ser Pro Lys Lys Lys Arg Lys
1               5                   10                  15

gtg atg gac aag aag tac tcc att ggg ctc gat atc ggc aca aac agc 96
Val Met Asp Lys Lys Tyr Ser Ile Gly Leu Asp Ile Gly Thr Asn Ser
            20                  25                  30

gtc ggc tgg gcc gtc att acg gac gag tac aag gtg ccg agc aaa aaa 144
Val Gly Trp Ala Val Ile Thr Asp Glu Tyr Lys Val Pro Ser Lys Lys
        35                  40                  45

ttc aaa gtt ctg ggc aat acc gat cgc cac agc ata aag aag aac ctc 192
Phe Lys Val Leu Gly Asn Thr Asp Arg His Ser Ile Lys Lys Asn Leu
    50                  55                  60

att ggc gcc ctc ctg ttc gac tcc ggg gag acg gcc gaa gcc acg cgg 240
Ile Gly Ala Leu Leu Phe Asp Ser Gly Glu Thr Ala Glu Ala Thr Arg
65                  70                  75                  80

ctc aaa aga aca gca cgg cgc aga tat acc cgc aga aag aat cgg atc 288
Leu Lys Arg Thr Ala Arg Arg Arg Tyr Thr Arg Arg Lys Asn Arg Ile
                85                  90                  95

tgc tac ctg cag gag atc ttt agt aat gag atg gct aag gtg gat gac 336
Cys Tyr Leu Gln Glu Ile Phe Ser Asn Glu Met Ala Lys Val Asp Asp
            100                 105                 110

tct ttc ttc cat agg ctg gag gag tcc ttt ttg gtg gag gag gat aaa 384
Ser Phe Phe His Arg Leu Glu Glu Ser Phe Leu Val Glu Glu Asp Lys

        115                 120                 125

aag cac gag cgc cac cca atc ttt ggc aat atc gtg gac gag gtg gcg 432
Lys His Glu Arg His Pro Ile Phe Gly Asn Ile Val Asp Glu Val Ala
    130                 135                 140

tac cat gaa aag tac cca acc ata tat cat ctg agg aag aag ctt gta 480
Tyr His Glu Lys Tyr Pro Thr Ile Tyr His Leu Arg Lys Lys Leu Val
145                 150                 155                 160

gac agt act gat aag gct gac ttg cgg ttg atc tat ctc gcg ctg gcg 528
Asp Ser Thr Asp Lys Ala Asp Leu Arg Leu Ile Tyr Leu Ala Leu Ala
                165                 170                 175

cat atg atc aaa ttt cgg gga cac ttc ctc atc gag ggg gac ctg aac 576
His Met Ile Lys Phe Arg Gly His Phe Leu Ile Glu Gly Asp Leu Asn
            180                 185                 190

cca gac aac agc gat gtc gac aaa ctc ttt atc caa ctg gtt cag act 624
Pro Asp Asn Ser Asp Val Asp Lys Leu Phe Ile Gln Leu Val Gln Thr
        195                 200                 205

tac aat cag ctt ttc gaa gag aac ccg atc aac gca tcc gga gtt gac 672
Tyr Asn Gln Leu Phe Glu Glu Asn Pro Ile Asn Ala Ser Gly Val Asp
    210                 215                 220

gcc aaa gca atc ctg agc gct agg ctg tcc aaa tcc cgg cgg ctc gaa 720
Ala Lys Ala Ile Leu Ser Ala Arg Leu Ser Lys Ser Arg Arg Leu Glu
225                 230                 235                 240

aac ctc atc gca cag ctc cct ggg gag aag aag aac ggc ctg ttt ggt 768
Asn Leu Ile Ala Gln Leu Pro Gly Glu Lys Lys Asn Gly Leu Phe Gly
                245                 250                 255

aat ctt atc gcc ctg tca ctc ggg ctg acc ccc aac ttt aaa tct aac 816
Asn Leu Ile Ala Leu Ser Leu Gly Leu Thr Pro Asn Phe Lys Ser Asn
            260                 265                 270

ttc gac ctg gcc gaa gat gcc aag ctt caa ctg agc aaa gac acc tac 864
Phe Asp Leu Ala Glu Asp Ala Lys Leu Gln Leu Ser Lys Asp Thr Tyr
        275                 280                 285

gat gat gat ctc gac aat ctg ctg gcc cag atc ggc gac cag tac gca 912
Asp Asp Asp Leu Asp Asn Leu Leu Ala Gln Ile Gly Asp Gln Tyr Ala
    290                 295                 300

gac ctt ttt ttg gcg gca aag aac ctg tca gac gcc att ctg ctg agt 960
Asp Leu Phe Leu Ala Ala Lys Asn Leu Ser Asp Ala Ile Leu Leu Ser
305                 310                 315                 320

gat att ctg cga gtg aac acg gag atc acc aaa gct ccg ctg agc gct 1008
Asp Ile Leu Arg Val Asn Thr Glu Ile Thr Lys Ala Pro Leu Ser Ala
                325                 330                 335

agt atg atc aag cgc tat gat gag cac cac caa gac ttg act ttg ctg 1056
Ser Met Ile Lys Arg Tyr Asp Glu His His Gln Asp Leu Thr Leu Leu
            340                 345                 350

aag gcc ctt gtc aga cag caa ctg cct gag aag tac aag gaa att ttc 1104
Lys Ala Leu Val Arg Gln Gln Leu Pro Glu Lys Tyr Lys Glu Ile Phe
        355                 360                 365

ttc gat cag tct aaa aat ggc tac gcc gga tac att gac ggc gga gca 1152

Phe Asp Gln Ser Lys Asn Gly Tyr Ala Gly Tyr Ile Asp Gly Gly Ala
    370                 375                 380

agc cag gag gaa ttt tac aaa ttt att aag ccc atc ttg gaa aaa atg      1200
Ser Gln Glu Glu Phe Tyr Lys Phe Ile Lys Pro Ile Leu Glu Lys Met
385                 390                 395                 400

gac ggc acc gag gag ctg ctg gta aag ctt aac aga gaa gat ctg ttg      1248
Asp Gly Thr Glu Glu Leu Leu Val Lys Leu Asn Arg Glu Asp Leu Leu
                405                 410                 415

cgc aaa cag cgc act ttc gac aat gga agc atc ccc cac cag att cac      1296
Arg Lys Gln Arg Thr Phe Asp Asn Gly Ser Ile Pro His Gln Ile His
            420                 425                 430

ctg ggc gaa ctg cac gct atc ctc agg cgg caa gag gat ttc tac ccc      1344
Leu Gly Glu Leu His Ala Ile Leu Arg Arg Gln Glu Asp Phe Tyr Pro
        435                 440                 445

ttt ttg aaa gat aac agg gaa aag att gag aaa atc ctc aca ttt cgg      1392
Phe Leu Lys Asp Asn Arg Glu Lys Ile Glu Lys Ile Leu Thr Phe Arg
    450                 455                 460

ata ccc tac tat gta ggc ccc ctc gcc cgg gga aat tcc aga ttc gcg      1440
Ile Pro Tyr Tyr Val Gly Pro Leu Ala Arg Gly Asn Ser Arg Phe Ala
465                 470                 475                 480

tgg atg act cgc aaa tca gaa gag acc atc act ccc tgg aac ttc gag      1488
Trp Met Thr Arg Lys Ser Glu Glu Thr Ile Thr Pro Trp Asn Phe Glu
                485                 490                 495

gaa gtc gtg gat aag ggg gcc tct gcc cag tcc ttc atc gaa agg atg      1536
Glu Val Val Asp Lys Gly Ala Ser Ala Gln Ser Phe Ile Glu Arg Met
            500                 505                 510

act aac ttt gat aaa aat ctg cct aac gaa aag gtg ctt cct aaa cac      1584
Thr Asn Phe Asp Lys Asn Leu Pro Asn Glu Lys Val Leu Pro Lys His
        515                 520                 525

tct ctg ctg tac gag tac ttc aca gtt tat aac gag ctc acc aag gtc      1632
Ser Leu Leu Tyr Glu Tyr Phe Thr Val Tyr Asn Glu Leu Thr Lys Val
    530                 535                 540

aaa tac gtc aca gaa ggg atg aga aag cca gca ttc ctg tct gga gag      1680
Lys Tyr Val Thr Glu Gly Met Arg Lys Pro Ala Phe Leu Ser Gly Glu
545                 550                 555                 560

cag aag aaa gct atc gtg gac ctc ctc ttc aag acg aac cgg aaa gtt      1728
Gln Lys Lys Ala Ile Val Asp Leu Leu Phe Lys Thr Asn Arg Lys Val
                565                 570                 575

acc gtg aaa cag ctc aaa gaa gac tat ttc aaa aag att gaa tgt ttc      1776
Thr Val Lys Gln Leu Lys Glu Asp Tyr Phe Lys Lys Ile Glu Cys Phe
            580                 585                 590

gac tct gtt gaa atc agc gga gtg gag gat cgc ttc aac gca tcc ctg      1824
Asp Ser Val Glu Ile Ser Gly Val Glu Asp Arg Phe Asn Ala Ser Leu
        595                 600                 605

gga acg tat cac gat ctc ctg aaa atc att aaa gac aag gac ttc ctg      1872
Gly Thr Tyr His Asp Leu Leu Lys Ile Ile Lys Asp Lys Asp Phe Leu

610                 615                 620

gac aat gag gag aac gag gac att ctt gag gac att gtc ctc acc ctt          1920
Asp Asn Glu Glu Asn Glu Asp Ile Leu Glu Asp Ile Val Leu Thr Leu
625                 630                 635                 640

acg ttg ttt gaa gat agg gag atg att gaa gaa cgc ttg aaa act tac          1968
Thr Leu Phe Glu Asp Arg Glu Met Ile Glu Glu Arg Leu Lys Thr Tyr
                645                 650                 655

gct cat ctc ttc gac gac aaa gtc atg aaa cag ctc aag agg cgc cga          2016
Ala His Leu Phe Asp Asp Lys Val Met Lys Gln Leu Lys Arg Arg Arg
            660                 665                 670

tat aca gga tgg ggg cgg ctg tca aga aaa ctg atc aat ggg atc cga          2064
Tyr Thr Gly Trp Gly Arg Leu Ser Arg Lys Leu Ile Asn Gly Ile Arg
        675                 680                 685

gac aag cag agt gga aag aca atc ctg gat ttt ctt aag tcc gat gga          2112
Asp Lys Gln Ser Gly Lys Thr Ile Leu Asp Phe Leu Lys Ser Asp Gly
    690                 695                 700

ttt gcc aac cgg aac ttc atg cag ttg atc cat gat gac tct ctc acc          2160
Phe Ala Asn Arg Asn Phe Met Gln Leu Ile His Asp Asp Ser Leu Thr
705                 710                 715                 720

ttt aag gag gac atc cag aaa gca caa gtt tct ggc cag ggg gac agt          2208
Phe Lys Glu Asp Ile Gln Lys Ala Gln Val Ser Gly Gln Gly Asp Ser
                725                 730                 735

ctt cac gag cac atc gct aat ctt gca ggt agc cca gct atc aaa aag          2256
Leu His Glu His Ile Ala Asn Leu Ala Gly Ser Pro Ala Ile Lys Lys
            740                 745                 750

gga ata ctg cag acc gtt aag gtc gtg gat gaa ctc gtc aaa gta atg          2304
Gly Ile Leu Gln Thr Val Lys Val Val Asp Glu Leu Val Lys Val Met
        755                 760                 765

gga agg cat aag ccc gag aat atc gtt atc gag atg gcc cga gag aac          2352
Gly Arg His Lys Pro Glu Asn Ile Val Ile Glu Met Ala Arg Glu Asn
    770                 775                 780

caa act acc cag aag gga cag aag aac agt agg gaa agg atg aag agg          2400
Gln Thr Thr Gln Lys Gly Gln Lys Asn Ser Arg Glu Arg Met Lys Arg
785                 790                 795                 800

att gaa gag ggt ata aaa gaa ctg ggg tcc caa atc ctt aag gaa cac          2448
Ile Glu Glu Gly Ile Lys Glu Leu Gly Ser Gln Ile Leu Lys Glu His
                805                 810                 815

cca gtt gaa aac acc cag ctt cag aat gag aag ctc tac ctg tac tac          2496
Pro Val Glu Asn Thr Gln Leu Gln Asn Glu Lys Leu Tyr Leu Tyr Tyr
            820                 825                 830

ctg cag aac ggc agg gac atg tac gtg gat cag gaa ctg gac atc aat          2544
Leu Gln Asn Gly Arg Asp Met Tyr Val Asp Gln Glu Leu Asp Ile Asn
        835                 840                 845

cgg ctc tcc gac tac gac gtg gat cat atc gtg ccc cag tct ttt ctc          2592
Arg Leu Ser Asp Tyr Asp Val Asp His Ile Val Pro Gln Ser Phe Leu
    850                 855                 860

aaa gat gat tct att gat aat aaa gtg ttg aca aga tcc gat aaa aat          2640

Lys Asp Asp Ser Ile Asp Asn Lys Val Leu Thr Arg Ser Asp Lys Asn
865                 870                 875                 880

aga ggg aag agt gat aac gtc ccc tca gaa gaa gtt gtc aag aaa atg        2688
Arg Gly Lys Ser Asp Asn Val Pro Ser Glu Glu Val Val Lys Lys Met
                885                 890                 895

aaa aat tat tgg cgg cag ctg ctg aac gcc aaa ctg atc aca caa cgg        2736
Lys Asn Tyr Trp Arg Gln Leu Leu Asn Ala Lys Leu Ile Thr Gln Arg
            900                 905                 910

aag ttc gat aat ctg act aag gct gaa cga ggt ggc ctg tct gag ttg        2784
Lys Phe Asp Asn Leu Thr Lys Ala Glu Arg Gly Gly Leu Ser Glu Leu
        915                 920                 925

gat aaa gcc ggc ttc atc aaa agg cag ctt gtt gag aca cgc cag atc        2832
Asp Lys Ala Gly Phe Ile Lys Arg Gln Leu Val Glu Thr Arg Gln Ile
    930                 935                 940

acc aag cac gtg gcc caa att ctc gat tca cgc atg aac acc aag tac        2880
Thr Lys His Val Ala Gln Ile Leu Asp Ser Arg Met Asn Thr Lys Tyr
945                 950                 955                 960

gat gaa aat gac aaa ctg att cga gag gtg aaa gtt att act ctg aag        2928
Asp Glu Asn Asp Lys Leu Ile Arg Glu Val Lys Val Ile Thr Leu Lys
                965                 970                 975

tct aag ctg gtc tca gat ttc aga aag gac ttt cag ttt tat aag gtg        2976
Ser Lys Leu Val Ser Asp Phe Arg Lys Asp Phe Gln Phe Tyr Lys Val
            980                 985                 990

aga gag atc aac aat tac cac cat gcg cat gat gcc tac ctg aat gca        3024
Arg Glu Ile Asn Asn Tyr His His Ala His Asp Ala Tyr Leu Asn Ala
        995                 1000                1005

gtg gta ggc act gca ctt atc aaa aaa tat ccc aag ctt gaa tct gaa        3072
Val Val Gly Thr Ala Leu Ile Lys Lys Tyr Pro Lys Leu Glu Ser Glu
    1010                1015                1020

ttt gtt tac gga gac tat aaa gtg tac gat gtt agg aaa atg atc gca        3120
Phe Val Tyr Gly Asp Tyr Lys Val Tyr Asp Val Arg Lys Met Ile Ala
1025                1030                1035                1040

aag tct gag cag gaa ata ggc aag gcc acc gct aag tac ttc ttt tac        3168
Lys Ser Glu Gln Glu Ile Gly Lys Ala Thr Ala Lys Tyr Phe Phe Tyr
                1045                1050                1055

agc aat att atg aat ttt ttc aag acc gag att aca ctg gcc aat gga        3216
Ser Asn Ile Met Asn Phe Phe Lys Thr Glu Ile Thr Leu Ala Asn Gly
            1060                1065                1070

gag att cgg aag cga cca ctt atc gaa aca aac gga gaa aca gga gaa        3264
Glu Ile Arg Lys Arg Pro Leu Ile Glu Thr Asn Gly Glu Thr Gly Glu
        1075                1080                1085

atc gtg tgg gac aag ggt agg gat ttc gcg aca gtc cgg aag gtc ctg        3312
Ile Val Trp Asp Lys Gly Arg Asp Phe Ala Thr Val Arg Lys Val Leu
    1090                1095                1100

tcc atg ccg cag gtg aac atc gtt aaa aag acc gaa gta cag acc gga        3360
Ser Met Pro Gln Val Asn Ile Val Lys Lys Thr Glu Val Gln Thr Gly

1105                1110                1115                1120

ggc ttc tcc aag gaa agt atc ctc ccg aaa agg aac agc gac aag ctg        3408
Gly Phe Ser Lys Glu Ser Ile Leu Pro Lys Arg Asn Ser Asp Lys Leu
                1125                1130                1135

atc gca cgc aaa aaa gat tgg gac ccc aag aaa tac ggc gga ttc gat        3456
Ile Ala Arg Lys Lys Asp Trp Asp Pro Lys Lys Tyr Gly Gly Phe Asp
            1140                1145                1150

tct cct aca gtc gct tac agt gta ctg gtt gtg gcc aaa gtg gag aaa        3504
Ser Pro Thr Val Ala Tyr Ser Val Leu Val Val Ala Lys Val Glu Lys
        1155                1160                1165

ggg aag tct aaa aaa ctc aaa agc gtc aag gaa ctg ctg ggc atc aca        3552
Gly Lys Ser Lys Lys Leu Lys Ser Val Lys Glu Leu Leu Gly Ile Thr
    1170                1175                1180

atc atg gag cga tca agc ttc gaa aaa aac ccc atc gac ttt ctc gag        3600
Ile Met Glu Arg Ser Ser Phe Glu Lys Asn Pro Ile Asp Phe Leu Glu
1185                1190                1195                1200

gcg aaa gga tat aaa gag gtc aaa aaa gac ctc atc att aag ctt ccc        3648
Ala Lys Gly Tyr Lys Glu Val Lys Lys Asp Leu Ile Ile Lys Leu Pro
                1205                1210                1215

aag tac tct ctc ttt gag ctt gaa aac ggc cgg aaa cga atg ctc gct        3696
Lys Tyr Ser Leu Phe Glu Leu Glu Asn Gly Arg Lys Arg Met Leu Ala
            1220                1225                1230

agt gcg ggc gag ctg cag aaa ggt aac gag ctg gca ctg ccc tct aaa        3744
Ser Ala Gly Glu Leu Gln Lys Gly Asn Glu Leu Ala Leu Pro Ser Lys
        1235                1240                1245

tac gtt aat ttc ttg tat ctg gcc agc cac tat gaa aag ctc aaa ggg        3792
Tyr Val Asn Phe Leu Tyr Leu Ala Ser His Tyr Glu Lys Leu Lys Gly
    1250                1255                1260

tct ccc gaa gat aat gag cag aag cag ctg ttc gtg gaa caa cac aaa        3840
Ser Pro Glu Asp Asn Glu Gln Lys Gln Leu Phe Val Glu Gln His Lys
1265                1270                1275                1280

cac tac ctt gat gag atc atc gag caa ata agc gaa ttc tcc aaa aga        3888
His Tyr Leu Asp Glu Ile Ile Glu Gln Ile Ser Glu Phe Ser Lys Arg
                1285                1290                1295

gtg atc ctc gcc gac gct aac ctc gat aag gtg ctt tct gct tac aat        3936
Val Ile Leu Ala Asp Ala Asn Leu Asp Lys Val Leu Ser Ala Tyr Asn
            1300                1305                1310

aag cac agg gat aag ccc atc agg gag cag gca gaa aac att atc cac        3984
Lys His Arg Asp Lys Pro Ile Arg Glu Gln Ala Glu Asn Ile Ile His
        1315                1320                1325

ttg ttt act ctg acc aac ttg ggc gcg cct gca gcc ttc aag tac ttc        4032
Leu Phe Thr Leu Thr Asn Leu Gly Ala Pro Ala Ala Phe Lys Tyr Phe
    1330                1335                1340

gac acc acc ata gac aga aag cgg tac acc tct aca aag gag gtc ctg        4080
Asp Thr Thr Ile Asp Arg Lys Arg Tyr Thr Ser Thr Lys Glu Val Leu
1345                1350                1355                1360

gac gcc aca ctg att cat cag tca att acg ggg ctc tat gaa aca aga        4128

Asp Ala Thr Leu Ile His Gln Ser Ile Thr Gly Leu Tyr Glu Thr Arg
                1365                1370                1375

atc gac ctc tct cag ctc ggt gga gac agc agg gct gac ccc aag aag 4176
Ile Asp Leu Ser Gln Leu Gly Gly Asp Ser Arg Ala Asp Pro Lys Lys
            1380                1385                1390

aag agg aag gtg ggc agt ggc cgc aaa aaa cgc cgc cag cgc cgc cgc 4224
Lys Arg Lys Val Gly Ser Gly Arg Lys Lys Arg Arg Gln Arg Arg Arg
        1395                1400                1405

ccg cag tga 4233
Pro Gln
    1410

<210> 15
<211> 4275
<212> DNA
<213> artificial
<400> SEQUENCE 15 (TATSPCas9TAT):
atg cat ggc cgc aaa aaa cgc cgc cag cgc cgc cgc ccg cag ggc agc 48
Met His Gly Arg Lys Lys Arg Arg Gln Arg Arg Arg Pro Gln Gly Ser
1               5                   10                  15

cat cac cac cac cat cac ggc agc ccc aag aag aag agg aag gtg atg 96
His His His His His His Gly Ser Pro Lys Lys Lys Arg Lys Val Met
            20                  25                  30

gac aag aag tac tcc att ggg ctc gat atc ggc aca aac agc gtc ggc 144
Asp Lys Lys Tyr Ser Ile Gly Leu Asp Ile Gly Thr Asn Ser Val Gly
        35                  40                  45

tgg gcc gtc att acg gac gag tac aag gtg ccg agc aaa aaa ttc aaa 192
Trp Ala Val Ile Thr Asp Glu Tyr Lys Val Pro Ser Lys Lys Phe Lys
    50                  55                  60

gtt ctg ggc aat acc gat cgc cac agc ata aag aag aac ctc att ggc 240
Val Leu Gly Asn Thr Asp Arg His Ser Ile Lys Lys Asn Leu Ile Gly
65                  70                  75                  80

gcc ctc ctg ttc gac tcc ggg gag acg gcc gaa gcc acg cgg ctc aaa 288
Ala Leu Leu Phe Asp Ser Gly Glu Thr Ala Glu Ala Thr Arg Leu Lys
                85                  90                  95

aga aca gca cgg cgc aga tat acc cgc aga aag aat cgg atc tgc tac 336
Arg Thr Ala Arg Arg Arg Tyr Thr Arg Arg Lys Asn Arg Ile Cys Tyr
            100                 105                 110

ctg cag gag atc ttt agt aat gag atg gct aag gtg gat gac tct ttc 384
Leu Gln Glu Ile Phe Ser Asn Glu Met Ala Lys Val Asp Asp Ser Phe
        115                 120                 125

ttc cat agg ctg gag gag tcc ttt ttg gtg gag gag gat aaa aag cac 432
Phe His Arg Leu Glu Glu Ser Phe Leu Val Glu Glu Asp Lys Lys His
    130                 135                 140

gag cgc cac cca atc ttt ggc aat atc gtg gac gag gtg gcg tac cat 480
Glu Arg His Pro Ile Phe Gly Asn Ile Val Asp Glu Val Ala Tyr His
145                 150                 155                 160

gaa aag tac cca acc ata tat cat ctg agg aag aag ctt gta gac agt 528
Glu Lys Tyr Pro Thr Ile Tyr His Leu Arg Lys Lys Leu Val Asp Ser
                165                 170                 175

act gat aag gct gac ttg cgg ttg atc tat ctc gcg ctg gcg cat atg 576
Thr Asp Lys Ala Asp Leu Arg Leu Ile Tyr Leu Ala Leu Ala His Met
            180                 185                 190

atc aaa ttt cgg gga cac ttc ctc atc gag ggg gac ctg aac cca gac 624
Ile Lys Phe Arg Gly His Phe Leu Ile Glu Gly Asp Leu Asn Pro Asp
        195                 200                 205

aac agc gat gtc gac aaa ctc ttt atc caa ctg gtt cag act tac aat 672
Asn Ser Asp Val Asp Lys Leu Phe Ile Gln Leu Val Gln Thr Tyr Asn
    210                 215                 220

cag ctt ttc gaa gag aac ccg atc aac gca tcc gga gtt gac gcc aaa 720
Gln Leu Phe Glu Glu Asn Pro Ile Asn Ala Ser Gly Val Asp Ala Lys
225                 230                 235                 240

gca atc ctg agc gct agg ctg tcc aaa tcc cgg cgg ctc gaa aac ctc 768
Ala Ile Leu Ser Ala Arg Leu Ser Lys Ser Arg Arg Leu Glu Asn Leu
                245                 250                 255

atc gca cag ctc cct ggg gag aag aag aac ggc ctg ttt ggt aat ctt 816
Ile Ala Gln Leu Pro Gly Glu Lys Lys Asn Gly Leu Phe Gly Asn Leu
            260                 265                 270

atc gcc ctg tca ctc ggg ctg acc ccc aac ttt aaa tct aac ttc gac 864
Ile Ala Leu Ser Leu Gly Leu Thr Pro Asn Phe Lys Ser Asn Phe Asp
        275                 280                 285

ctg gcc gaa gat gcc aag ctt caa ctg agc aaa gac acc tac gat gat 912
Leu Ala Glu Asp Ala Lys Leu Gln Leu Ser Lys Asp Thr Tyr Asp Asp
    290                 295                 300

gat ctc gac aat ctg ctg gcc cag atc ggc gac cag tac gca gac ctt 960
Asp Leu Asp Asn Leu Leu Ala Gln Ile Gly Asp Gln Tyr Ala Asp Leu
305                 310                 315                 320

ttt ttg gcg gca aag aac ctg tca gac gcc att ctg ctg agt gat att 1008
Phe Leu Ala Ala Lys Asn Leu Ser Asp Ala Ile Leu Leu Ser Asp Ile
                325                 330                 335

ctg cga gtg aac acg gag atc acc aaa gct ccg ctg agc gct agt atg 1056
Leu Arg Val Asn Thr Glu Ile Thr Lys Ala Pro Leu Ser Ala Ser Met
            340                 345                 350

atc aag cgc tat gat gag cac cac caa gac ttg act ttg ctg aag gcc 1104
Ile Lys Arg Tyr Asp Glu His His Gln Asp Leu Thr Leu Leu Lys Ala
        355                 360                 365

ctt gtc aga cag caa ctg cct gag aag tac aag gaa att ttc ttc gat 1152
Leu Val Arg Gln Gln Leu Pro Glu Lys Tyr Lys Glu Ile Phe Phe Asp
    370                 375                 380

cag tct aaa aat ggc tac gcc gga tac att gac ggc gga gca agc cag 1200
Gln Ser Lys Asn Gly Tyr Ala Gly Tyr Ile Asp Gly Gly Ala Ser Gln
385                 390                 395                 400

gag gaa ttt tac aaa ttt att aag ccc atc ttg gaa aaa atg gac ggc 1248
Glu Glu Phe Tyr Lys Phe Ile Lys Pro Ile Leu Glu Lys Met Asp Gly
                405                 410                 415

acc gag gag ctg ctg gta aag ctt aac aga gaa gat ctg ttg cgc aaa 1296
Thr Glu Glu Leu Leu Val Lys Leu Asn Arg Glu Asp Leu Leu Arg Lys
            420                 425                 430

cag cgc act ttc gac aat gga agc atc ccc cac cag att cac ctg ggc 1344
Gln Arg Thr Phe Asp Asn Gly Ser Ile Pro His Gln Ile His Leu Gly
        435                 440                 445

gaa ctg cac gct atc ctc agg cgg caa gag gat ttc tac ccc ttt ttg 1392
Glu Leu His Ala Ile Leu Arg Arg Gln Glu Asp Phe Tyr Pro Phe Leu
    450                 455                 460

aaa gat aac agg gaa aag att gag aaa atc ctc aca ttt cgg ata ccc 1440
Lys Asp Asn Arg Glu Lys Ile Glu Lys Ile Leu Thr Phe Arg Ile Pro
465                 470                 475                 480

tac tat gta ggc ccc ctc gcc cgg gga aat tcc aga ttc gcg tgg atg 1488
Tyr Tyr Val Gly Pro Leu Ala Arg Gly Asn Ser Arg Phe Ala Trp Met
                485                 490                 495

act cgc aaa tca gaa gag acc atc act ccc tgg aac ttc gag gaa gtc 1536
Thr Arg Lys Ser Glu Glu Thr Ile Thr Pro Trp Asn Phe Glu Glu Val
            500                 505                 510

gtg gat aag ggg gcc tct gcc cag tcc ttc atc gaa agg atg act aac 1584
Val Asp Lys Gly Ala Ser Ala Gln Ser Phe Ile Glu Arg Met Thr Asn
        515                 520                 525

ttt gat aaa aat ctg cct aac gaa aag gtg ctt cct aaa cac tct ctg 1632
Phe Asp Lys Asn Leu Pro Asn Glu Lys Val Leu Pro Lys His Ser Leu
    530                 535                 540

ctg tac gag tac ttc aca gtt tat aac gag ctc acc aag gtc aaa tac 1680
Leu Tyr Glu Tyr Phe Thr Val Tyr Asn Glu Leu Thr Lys Val Lys Tyr
545                 550                 555                 560

gtc aca gaa ggg atg aga aag cca gca ttc ctg tct gga gag cag aag 1728
Val Thr Glu Gly Met Arg Lys Pro Ala Phe Leu Ser Gly Glu Gln Lys
                565                 570                 575

aaa gct atc gtg gac ctc ctc ttc aag acg aac cgg aaa gtt acc gtg 1776
Lys Ala Ile Val Asp Leu Leu Phe Lys Thr Asn Arg Lys Val Thr Val
            580                 585                 590

aaa cag ctc aaa gaa gac tat ttc aaa aag att gaa tgt ttc gac tct 1824
Lys Gln Leu Lys Glu Asp Tyr Phe Lys Lys Ile Glu Cys Phe Asp Ser
        595                 600                 605

gtt gaa atc agc gga gtg gag gat cgc ttc aac gca tcc ctg gga acg 1872
Val Glu Ile Ser Gly Val Glu Asp Arg Phe Asn Ala Ser Leu Gly Thr
    610                 615                 620

tat cac gat ctc ctg aaa atc att aaa gac aag gac ttc ctg gac aat 1920
Tyr His Asp Leu Leu Lys Ile Ile Lys Asp Lys Asp Phe Leu Asp Asn
625                 630                 635                 640

gag gag aac gag gac att ctt gag gac att gtc ctc acc ctt acg ttg 1968
Glu Glu Asn Glu Asp Ile Leu Glu Asp Ile Val Leu Thr Leu Thr Leu
                645                 650                 655

ttt gaa gat agg gag atg att gaa gaa cgc ttg aaa act tac gct cat 2016
Phe Glu Asp Arg Glu Met Ile Glu Glu Arg Leu Lys Thr Tyr Ala His
            660                 665                 670

ctc ttc gac gac aaa gtc atg aaa cag ctc aag agg cgc cga tat aca 2064
Leu Phe Asp Asp Lys Val Met Lys Gln Leu Lys Arg Arg Arg Tyr Thr
        675                 680                 685

gga tgg ggg cgg ctg tca aga aaa ctg atc aat ggg atc cga gac aag 2112
Gly Trp Gly Arg Leu Ser Arg Lys Leu Ile Asn Gly Ile Arg Asp Lys
    690                 695                 700

cag agt gga aag aca atc ctg gat ttt ctt aag tcc gat gga ttt gcc 2160
Gln Ser Gly Lys Thr Ile Leu Asp Phe Leu Lys Ser Asp Gly Phe Ala
705                 710                 715                 720

aac cgg aac ttc atg cag ttg atc cat gat gac tct ctc acc ttt aag 2208
Asn Arg Asn Phe Met Gln Leu Ile His Asp Asp Ser Leu Thr Phe Lys
                725                 730                 735

gag gac atc cag aaa gca caa gtt tct ggc cag ggg gac agt ctt cac 2256
Glu Asp Ile Gln Lys Ala Gln Val Ser Gly Gln Gly Asp Ser Leu His
            740                 745                 750

gag cac atc gct aat ctt gca ggt agc cca gct atc aaa aag gga ata 2304
Glu His Ile Ala Asn Leu Ala Gly Ser Pro Ala Ile Lys Lys Gly Ile
        755                 760                 765

ctg cag acc gtt aag gtc gtg gat gaa ctc gtc aaa gta atg gga agg 2352
Leu Gln Thr Val Lys Val Val Asp Glu Leu Val Lys Val Met Gly Arg
    770                 775                 780

cat aag ccc gag aat atc gtt atc gag atg gcc cga gag aac caa act 2400
His Lys Pro Glu Asn Ile Val Ile Glu Met Ala Arg Glu Asn Gln Thr
785                 790                 795                 800

acc cag aag gga cag aag aac agt agg gaa agg atg aag agg att gaa 2448
Thr Gln Lys Gly Gln Lys Asn Ser Arg Glu Arg Met Lys Arg Ile Glu
                805                 810                 815

gag ggt ata aaa gaa ctg ggg tcc caa atc ctt aag gaa cac cca gtt 2496
Glu Gly Ile Lys Glu Leu Gly Ser Gln Ile Leu Lys Glu His Pro Val
            820                 825                 830

gaa aac acc cag ctt cag aat gag aag ctc tac ctg tac tac ctg cag 2544
Glu Asn Thr Gln Leu Gln Asn Glu Lys Leu Tyr Leu Tyr Tyr Leu Gln
        835                 840                 845

aac ggc agg gac atg tac gtg gat cag gaa ctg gac atc aat cgg ctc 2592
Asn Gly Arg Asp Met Tyr Val Asp Gln Glu Leu Asp Ile Asn Arg Leu
    850                 855                 860

tcc gac tac gac gtg gat cat atc gtg ccc cag tct ttt ctc aaa gat 2640
Ser Asp Tyr Asp Val Asp His Ile Val Pro Gln Ser Phe Leu Lys Asp
865                 870                 875                 880

gat tct att gat aat aaa gtg ttg aca aga tcc gat aaa aat aga ggg 2689
Asp Ser Ile Asp Asn Lys Val Leu Thr Arg Ser Asp Lys Asn Arg Gly
                885                 890                 895

aag agt gat aac gtc ccc tca gaa gaa gtt gtc aag aaa atg aaa aat 2736
Lys Ser Asp Asn Val Pro Ser Glu Glu Val Val Lys Lys Met Lys Asn
            900                 905                 910

tat tgg cgg cag ctg ctg aac gcc aaa ctg atc aca caa cgg aag ttc 2784
Tyr Trp Arg Gln Leu Leu Asn Ala Lys Leu Ile Thr Gln Arg Lys Phe
        915                 920                 925

gat aat ctg act aag gct gaa cga ggt ggc ctg tct gag ttg gat aaa 2832
Asp Asn Leu Thr Lys Ala Glu Arg Gly Gly Leu Ser Glu Leu Asp Lys
    930                 935                 940

gcc ggc ttc atc aaa agg cag ctt gtt gag aca cgc cag atc acc aag 2880
Ala Gly Phe Ile Lys Arg Gln Leu Val Glu Thr Arg Gln Ile Thr Lys
945                 950                 955                 960

cac gtg gcc caa att ctc gat tca cgc atg aac acc aag tac gat gaa 2928
His Val Ala Gln Ile Leu Asp Ser Arg Met Asn Thr Lys Tyr Asp Glu
                965                 970                 975

aat gac aaa ctg att cga gag gtg aaa gtt att act ctg aag tct aag 2976
Asn Asp Lys Leu Ile Arg Glu Val Lys Val Ile Thr Leu Lys Ser Lys
            980                 985                 990

ctg gtc tca gat ttc aga aag gac ttt cag ttt tat aag gtg aga gag 3024
Leu Val Ser Asp Phe Arg Lys Asp Phe Gln Phe Tyr Lys Val Arg Glu
        995                 1000                1005

atc aac aat tac cac cat gcg cat gat gcc tac ctg aat gca gtg gta 3072
Ile Asn Asn Tyr His His Ala His Asp Ala Tyr Leu Asn Ala Val Val
    1010                1015                1020

ggc act gca ctt atc aaa aaa tat ccc aag ctt gaa tct gaa ttt gtt 3120
Gly Thr Ala Leu Ile Lys Lys Tyr Pro Lys Leu Glu Ser Glu Phe Val
1025                1030                1035                1040

tac gga gac tat aaa gtg tac gat gtt agg aaa atg atc gca aag tct 3168
Tyr Gly Asp Tyr Lys Val Tyr Asp Val Arg Lys Met Ile Ala Lys Ser
                1045                1050                1055

gag cag gaa ata ggc aag gcc acc gct aag tac ttc ttt tac agc aat 3216
Glu Gln Glu Ile Gly Lys Ala Thr Ala Lys Tyr Phe Phe Tyr Ser Asn
            1060                1065                1070

att atg aat ttt ttc aag acc gag att aca ctg gcc aat gga gag att 3264
Ile Met Asn Phe Phe Lys Thr Glu Ile Thr Leu Ala Asn Gly Glu Ile
        1075                1080                1085

cgg aag cga cca ctt atc gaa aca aac gga gaa aca gga gaa atc gtg 3312
Arg Lys Arg Pro Leu Ile Glu Thr Asn Gly Glu Thr Gly Glu Ile Val
    1090                1095                1100

tgg gac aag ggt agg gat ttc gcg aca gtc cgg aag gtc ctg tcc atg 3360
Trp Asp Lys Gly Arg Asp Phe Ala Thr Val Arg Lys Val Leu Ser Met
1105                1110                1115                1120

ccg cag gtg aac atc gtt aaa aag acc gaa gta cag acc gga ggc ttc 3408
Pro Gln Val Asn Ile Val Lys Lys Thr Glu Val Gln Thr Gly Gly Phe
                1125                1130                1135

tcc aag gaa agt atc ctc ccg aaa agg aac agc gac aag ctg atc gca 3456
Ser Lys Glu Ser Ile Leu Pro Lys Arg Asn Ser Asp Lys Leu Ile Ala
            1140                1145                1150

cgc aaa aaa gat tgg gac ccc aag aaa tac ggc gga ttc gat tct cct 3504
Arg Lys Lys Asp Trp Asp Pro Lys Lys Tyr Gly Gly Phe Asp Ser Pro
        1155                1160                1165

aca gtc gct tac agt gta ctg gtt gtg gcc aaa gtg gag aaa ggg aag 3552
Thr Val Ala Tyr Ser Val Leu Val Val Ala Lys Val Glu Lys Gly Lys
    1170                1175                1180

tct aaa aaa ctc aaa agc gtc aag gaa ctg ctg ggc atc aca atc atg 3600
Ser Lys Lys Leu Lys Ser Val Lys Glu Leu Leu Gly Ile Thr Ile Met
1185                1190                1195                1200

gag cga tca agc ttc gaa aaa aac ccc atc gac ttt ctc gag gcg aaa 3648
Glu Arg Ser Ser Phe Glu Lys Asn Pro Ile Asp Phe Leu Glu Ala Lys
                1205                1210                1215

gga tat aaa gag gtc aaa aaa gac ctc atc att aag ctt ccc aag tac 3696
Gly Tyr Lys Glu Val Lys Lys Asp Leu Ile Ile Lys Leu Pro Lys Tyr
            1220                1225                1230

tct ctc ttt gag ctt gaa aac ggc cgg aaa cga atg ctc gct agt gcg 3744
Ser Leu Phe Glu Leu Glu Asn Gly Arg Lys Arg Met Leu Ala Ser Ala
        1235                1240                1245

ggc gag ctg cag aaa ggt aac gag ctg gca ctg ccc tct aaa tac gtt 3792
Gly Glu Leu Gln Lys Gly Asn Glu Leu Ala Leu Pro Ser Lys Tyr Val
    1250                1255                1260

aat ttc ttg tat ctg gcc agc cac tat gaa aag ctc aaa ggg tct ccc 3840
Asn Phe Leu Tyr Leu Ala Ser His Tyr Glu Lys Leu Lys Gly Ser Pro
1265                1270                1275                1280

gaa gat aat gag cag aag cag ctg ttc gtg gaa caa cac aaa cac tac 3888
Glu Asp Asn Glu Gln Lys Gln Leu Phe Val Glu Gln His Lys His Tyr
                1285                1290                1295

ctt gat gag atc atc gag caa ata agc gaa ttc tcc aaa aga gtg atc 3936
Leu Asp Glu Ile Ile Glu Gln Ile Ser Glu Phe Ser Lys Arg Val Ile
            1300                1305                1310

ctc gcc gac gct aac ctc gat aag gtg ctt tct gct tac aat aag cac 3984
Leu Ala Asp Ala Asn Leu Asp Lys Val Leu Ser Ala Tyr Asn Lys His
        1315                1320                1325

agg gat aag ccc atc agg gag cag gca gaa aac att atc cac ttg ttt 4032
Arg Asp Lys Pro Ile Arg Glu Gln Ala Glu Asn Ile Ile His Leu Phe
    1330                1335                1340

act ctg acc aac ttg ggc gcg cct gca gcc ttc aag tac ttc gac acc 4080
Thr Leu Thr Asn Leu Gly Ala Pro Ala Ala Phe Lys Tyr Phe Asp Thr
1345                1350                1355                1360

acc ata gac aga aag cgg tac acc tct aca aag gag gtc ctg gac gcc 4128
Thr Ile Asp Arg Lys Arg Tyr Thr Ser Thr Lys Glu Val Leu Asp Ala
                1365                1370                1375

aca ctg att cat cag tca att acg ggg ctc tat gaa aca aga atc gac 4176
Thr Leu Ile His Gln Ser Ile Thr Gly Leu Tyr Glu Thr Arg Ile Asp
            1380                1385                1390

ctc tct cag ctc ggt gga gac agc agg gct gac ccc aag aag aag agg 4224
Leu Ser Gln Leu Gly Gly Asp Ser Arg Ala Asp Pro Lys Lys Lys Arg
        1395                1400                1405

aag gtg ggc agt ggc cgc aaa aaa cgc cgc cag cgc cgc cgc ccg cag     4272
Lys Val Gly Ser Gly Arg Lys Lys Arg Arg Gln Arg Arg Arg Pro Gln
   1410                1415                1420

tga    4275

<210> 16
<211> 4233
<212> DNA
<213> artificial
<400> SEQUENCE 16 (SPCas9D10ATAT):
atg cat cat cac cac cac cat cac ggc agc ccc aag aag aag agg aag     48
Met His His His His His His His Gly Ser Pro Lys Lys Lys Arg Lys
1               5                   10                  15

gtg atg gac aag aag tac tcc att ggg ctc gat atc ggc aca aac agc     96
Val Met Asp Lys Lys Tyr Ser Ile Gly Leu Asp Ile Gly Thr Asn Ser
            20                  25                  30

gtc ggc tgg gcc gtc att acg gac gag tac aag gtg ccg agc aaa aaa     144
Val Gly Trp Ala Val Ile Thr Asp Glu Tyr Lys Val Pro Ser Lys Lys
        35                  40                  45

ttc aaa gtt ctg ggc aat acc gat cgc cac agc ata aag aag aac ctc     192
Phe Lys Val Leu Gly Asn Thr Asp Arg His Ser Ile Lys Lys Asn Leu
    50                  55                  60

att ggc gcc ctc ctg ttc gac tcc ggg gag acg gcc gaa gcc acg cgg     240
Ile Gly Ala Leu Leu Phe Asp Ser Gly Glu Thr Ala Glu Ala Thr Arg
65                  70                  75                  80

ctc aaa aga aca gca cgg cgc aga tat acc cgc aga aag aat cgg atc     288
Leu Lys Arg Thr Ala Arg Arg Arg Tyr Thr Arg Arg Lys Asn Arg Ile
                85                  90                  95

tgc tac ctg cag gag atc ttt agt aat gag atg gct aag gtg gat gac     336
Cys Tyr Leu Gln Glu Ile Phe Ser Asn Glu Met Ala Lys Val Asp Asp
            100                 105                 110

tct ttc ttc cat agg ctg gag gag tcc ttt ttg gtg gag gag gat aaa     384
Ser Phe Phe His Arg Leu Glu Glu Ser Phe Leu Val Glu Glu Asp Lys
        115                 120                 125

aag cac gag cgc cac cca atc ttt ggc aat atc gtg gac gag gtg gcg     432
Lys His Glu Arg His Pro Ile Phe Gly Asn Ile Val Asp Glu Val Ala
    130                 135                 140

tac cat gaa aag tac cca acc ata tat cat ctg agg aag aag ctt gta     480
Tyr His Glu Lys Tyr Pro Thr Ile Tyr His Leu Arg Lys Lys Leu Val
145                 150                 155                 160

gac agt act gat aag gct gac ttg cgg ttg atc tat ctc gcg ctg gcg     528
Asp Ser Thr Asp Lys Ala Asp Leu Arg Leu Ile Tyr Leu Ala Leu Ala
                165                 170                 175

cat atg atc aaa ttt cgg gga cac ttc ctc atc gag ggg gac ctg aac     576
His Met Ile Lys Phe Arg Gly His Phe Leu Ile Glu Gly Asp Leu Asn
            180                 185                 190

cca gac aac agc gat gtc gac aaa ctc ttt atc caa ctg gtt cag act     624
Pro Asp Asn Ser Asp Val Asp Lys Leu Phe Ile Gln Leu Val Gln Thr

195                 200                 205

tac aat cag ctt ttc gaa gag aac ccg atc aac gca tcc gga gtt gac         672
Tyr Asn Gln Leu Phe Glu Glu Asn Pro Ile Asn Ala Ser Gly Val Asp
    210                 215                 220

gcc aaa gca atc ctg agc gct agg ctg tcc aaa tcc cgg cgg ctc gaa         720
Ala Lys Ala Ile Leu Ser Ala Arg Leu Ser Lys Ser Arg Arg Leu Glu
225                 230                 235                 240

aac ctc atc gca cag ctc cct ggg gag aag aag aac ggc ctg ttt ggt         768
Asn Leu Ile Ala Gln Leu Pro Gly Glu Lys Lys Asn Gly Leu Phe Gly
                245                 250                 255

aat ctt atc gcc ctg tca ctc ggg ctg acc ccc aac ttt aaa tct aac         816
Asn Leu Ile Ala Leu Ser Leu Gly Leu Thr Pro Asn Phe Lys Ser Asn
            260                 265                 270

ttc gac ctg gcc gaa gat gcc aag ctt caa ctg agc aaa gac acc tac         864
Phe Asp Leu Ala Glu Asp Ala Lys Leu Gln Leu Ser Lys Asp Thr Tyr
        275                 280                 285

gat gat gat ctc gac aat ctg ctg gcc cag atc ggc gac cag tac gca         912
Asp Asp Asp Leu Asp Asn Leu Leu Ala Gln Ile Gly Asp Gln Tyr Ala
    290                 295                 300

gac ctt ttt ttg gcg gca aag aac ctg tca gac gcc att ctg ctg agt         960
Asp Leu Phe Leu Ala Ala Lys Asn Leu Ser Asp Ala Ile Leu Leu Ser
305                 310                 315                 320

gat att ctg cga gtg aac acg gag atc acc aaa gct ccg ctg agc gct        1008
Asp Ile Leu Arg Val Asn Thr Glu Ile Thr Lys Ala Pro Leu Ser Ala
                325                 330                 335

agt atg atc aag cgc tat gat gag cac cac caa gac ttg act ttg ctg        1056
Ser Met Ile Lys Arg Tyr Asp Glu His His Gln Asp Leu Thr Leu Leu
            340                 345                 350

aag gcc ctt gtc aga cag caa ctg cct gag aag tac aag gaa att ttc        1104
Lys Ala Leu Val Arg Gln Gln Leu Pro Glu Lys Tyr Lys Glu Ile Phe
        355                 360                 365

ttc gat cag tct aaa aat ggc tac gcc gga tac att gac ggc gga gca        1152
Phe Asp Gln Ser Lys Asn Gly Tyr Ala Gly Tyr Ile Asp Gly Gly Ala
    370                 375                 380

agc cag gag gaa ttt tac aaa ttt att aag ccc atc ttg gaa aaa atg        1200
Ser Gln Glu Glu Phe Tyr Lys Phe Ile Lys Pro Ile Leu Glu Lys Met
385                 390                 395                 400

gac ggc acc gag gag ctg ctg gta aag ctt aac aga gaa gat ctg ttg        1248
Asp Gly Thr Glu Glu Leu Leu Val Lys Leu Asn Arg Glu Asp Leu Leu
                405                 410                 415

cgc aaa cag cgc act ttc gac aat gga agc atc ccc cac cag att cac        1296
Arg Lys Gln Arg Thr Phe Asp Asn Gly Ser Ile Pro His Gln Ile His
            420                 425                 430

ctg ggc gaa ctg cac gct atc ctc agg cgg caa gag gat ttc tac ccc        1344
Leu Gly Glu Leu His Ala Ile Leu Arg Arg Gln Glu Asp Phe Tyr Pro
        435                 440                 445

ttt ttg aaa gat aac agg gaa aag att gag aaa atc ctc aca ttt cgg        1392

Phe Leu Lys Asp Asn Arg Glu Lys Ile Glu Lys Ile Leu Thr Phe Arg
    450                 455                 460

ata ccc tac tat gta ggc ccc ctc gcc cgg gga aat tcc aga ttc gcg      1440
Ile Pro Tyr Tyr Val Gly Pro Leu Ala Arg Gly Asn Ser Arg Phe Ala
465                 470                 475                 480

tgg atg act cgc aaa tca gaa gag acc atc act ccc tgg aac ttc gag      1488
Trp Met Thr Arg Lys Ser Glu Glu Thr Ile Thr Pro Trp Asn Phe Glu
                485                 490                 495

gaa gtc gtg gat aag ggg gcc tct gcc cag tcc ttc atc gaa agg atg      1536
Glu Val Val Asp Lys Gly Ala Ser Ala Gln Ser Phe Ile Glu Arg Met
            500                 505                 510

act aac ttt gat aaa aat ctg cct aac gaa aag gtg ctt cct aaa cac      1584
Thr Asn Phe Asp Lys Asn Leu Pro Asn Glu Lys Val Leu Pro Lys His
        515                 520                 525

tct ctg ctg tac gag tac ttc aca gtt tat aac gag ctc acc aag gtc      1632
Ser Leu Leu Tyr Glu Tyr Phe Thr Val Tyr Asn Glu Leu Thr Lys Val
    530                 535                 540

aaa tac gtc aca gaa ggg atg aga aag cca gca ttc ctg tct gga gag      1680
Lys Tyr Val Thr Glu Gly Met Arg Lys Pro Ala Phe Leu Ser Gly Glu
545                 550                 555                 560

cag aag aaa gct atc gtg gac ctc ctc ttc aag acg aac cgg aaa gtt      1728
Gln Lys Lys Ala Ile Val Asp Leu Leu Phe Lys Thr Asn Arg Lys Val
                565                 570                 575

acc gtg aaa cag ctc aaa gaa gac tat ttc aaa aag att gaa tgt ttc      1776
Thr Val Lys Gln Leu Lys Glu Asp Tyr Phe Lys Lys Ile Glu Cys Phe
            580                 585                 590

gac tct gtt gaa atc agc gga gtg gag gat cgc ttc aac gca tcc ctg      1824
Asp Ser Val Glu Ile Ser Gly Val Glu Asp Arg Phe Asn Ala Ser Leu
        595                 600                 605

gga acg tat cac gat ctc ctg aaa atc att aaa gac aag gac ttc ctg      1872
Gly Thr Tyr His Asp Leu Leu Lys Ile Ile Lys Asp Lys Asp Phe Leu
    610                 615                 620

gac aat gag gag aac gag gac att ctt gag gac att gtc ctc acc ctt      1920
Asp Asn Glu Glu Asn Glu Asp Ile Leu Glu Asp Ile Val Leu Thr Leu
625                 630                 635                 640

acg ttg ttt gaa gat agg gag atg att gaa gaa cgc ttg aaa act tac      1968
Thr Leu Phe Glu Asp Arg Glu Met Ile Glu Glu Arg Leu Lys Thr Tyr
                645                 650                 655

gct cat ctc ttc gac gac aaa gtc atg aaa cag ctc aag agg cgc cga      2016
Ala His Leu Phe Asp Asp Lys Val Met Lys Gln Leu Lys Arg Arg Arg
            660                 665                 670

tat aca gga tgg ggg cgg ctg tca aga aaa ctg atc aat ggg atc cga      2064
Tyr Thr Gly Trp Gly Arg Leu Ser Arg Lys Leu Ile Asn Gly Ile Arg
        675                 680                 685

gac aag cag agt gga aag aca atc ctg gat ttt ctt aag tcc gat gga      2112
Asp Lys Gln Ser Gly Lys Thr Ile Leu Asp Phe Leu Lys Ser Asp Gly

690                 695                 700

ttt gcc aac cgg aac ttc atg cag ttg atc cat gat gac tct ctc acc         2160
Phe Ala Asn Arg Asn Phe Met Gln Leu Ile His Asp Asp Ser Leu Thr
705                 710                 715                 720

ttt aag gag gac atc cag aaa gca caa gtt tct ggc cag ggg gac agt         2208
Phe Lys Glu Asp Ile Gln Lys Ala Gln Val Ser Gly Gln Gly Asp Ser
                725                 730                 735

ctt cac gag cac atc gct aat ctt gca ggt agc cca gct atc aaa aag         2256
Leu His Glu His Ile Ala Asn Leu Ala Gly Ser Pro Ala Ile Lys Lys
            740                 745                 750

gga ata ctg cag acc gtt aag gtc gtg gat gaa ctc gtc aaa gta atg         2304
Gly Ile Leu Gln Thr Val Lys Val Val Asp Glu Leu Val Lys Val Met
        755                 760                 765

gga agg cat aag ccc gag aat atc gtt atc gag atg gcc cga gag aac         2352
Gly Arg His Lys Pro Glu Asn Ile Val Ile Glu Met Ala Arg Glu Asn
    770                 775                 780

caa act acc cag aag gga cag aag aac agt agg gaa agg atg aag agg         2401
Gln Thr Thr Gln Lys Gly Gln Lys Asn Ser Arg Glu Arg Met Lys Arg
785                 790                 795                 800

att gaa gag ggt ata aaa gaa ctg ggg tcc caa atc ctt aag gaa cac         2448
Ile Glu Glu Gly Ile Lys Glu Leu Gly Ser Gln Ile Leu Lys Glu His
                805                 810                 815

cca gtt gaa aac acc cag ctt cag aat gag aag ctc tac ctg tac tac         2496
Pro Val Glu Asn Thr Gln Leu Gln Asn Glu Lys Leu Tyr Leu Tyr Tyr
            820                 825                 830

ctg cag aac ggc agg gac atg tac gtg gat cag gaa ctg gac atc aat         2544
Leu Gln Asn Gly Arg Asp Met Tyr Val Asp Gln Glu Leu Asp Ile Asn
        835                 840                 845

cgg ctc tcc gac tac gac gtg gat cat atc gtg ccc cag tct ttt ctc         2592
Arg Leu Ser Asp Tyr Asp Val Asp His Ile Val Pro Gln Ser Phe Leu
    850                 855                 860

aaa gat gat tct att gat aat aaa gtg ttg aca aga tcc gat aaa aat         2640
Lys Asp Asp Ser Ile Asp Asn Lys Val Leu Thr Arg Ser Asp Lys Asn
865                 870                 875                 880

aga ggg aag agt gat aac gtc ccc tca gaa gaa gtt gtc aag aaa atg         2688
Arg Gly Lys Ser Asp Asn Val Pro Ser Glu Glu Val Val Lys Lys Met
                885                 890                 895

aaa aat tat tgg cgg cag ctg ctg aac gcc aaa ctg atc aca caa cgg         2736
Lys Asn Tyr Trp Arg Gln Leu Leu Asn Ala Lys Leu Ile Thr Gln Arg
            900                 905                 910

aag ttc gat aat ctg act aag gct gaa cga ggt ggc ctg tct gag ttg         2784
Lys Phe Asp Asn Leu Thr Lys Ala Glu Arg Gly Gly Leu Ser Glu Leu
        915                 920                 925

gat aaa gcc ggc ttc atc aaa agg cag ctt gtt gag aca cgc cag atc         2832
Asp Lys Ala Gly Phe Ile Lys Arg Gln Leu Val Glu Thr Arg Gln Ile
    930                 935                 940

acc aag cac gtg gcc caa att ctc gat tca cgc atg aac acc aag tac         2880

Thr Lys His Val Ala Gln Ile Leu Asp Ser Arg Met Asn Thr Lys Tyr
945                 950                 955                 960

gat gaa aat gac aaa ctg att cga gag gtg aaa gtt att act ctg aag        2928
Asp Glu Asn Asp Lys Leu Ile Arg Glu Val Lys Val Ile Thr Leu Lys
                965                 970                 975

tct aag ctg gtc tca gat ttc aga aag gac ttt cag ttt tat aag gtg        2976
Ser Lys Leu Val Ser Asp Phe Arg Lys Asp Phe Gln Phe Tyr Lys Val
            980                 985                 990

aga gag atc aac aat tac cac cat gcg cat gat gcc tac ctg aat gca        3024
Arg Glu Ile Asn Asn Tyr His His Ala His Asp Ala Tyr Leu Asn Ala
        995                 1000                1005

gtg gta ggc act gca ctt atc aaa aaa tat ccc aag ctt gaa tct gaa        3072
Val Val Gly Thr Ala Leu Ile Lys Lys Tyr Pro Lys Leu Glu Ser Glu
    1010                1015                1020

ttt gtt tac gga gac tat aaa gtg tac gat gtt agg aaa atg atc gca        3120
Phe Val Tyr Gly Asp Tyr Lys Val Tyr Asp Val Arg Lys Met Ile Ala
1025                1030                1035                1040

aag tct gag cag gaa ata ggc aag gcc acc gct aag tac ttc ttt tac        3168
Lys Ser Glu Gln Glu Ile Gly Lys Ala Thr Ala Lys Tyr Phe Phe Tyr
                1045                1050                1055

agc aat att atg aat ttt ttc aag acc gag att aca ctg gcc aat gga        3216
Ser Asn Ile Met Asn Phe Phe Lys Thr Glu Ile Thr Leu Ala Asn Gly
            1060                1065                1070

gag att cgg aag cga cca ctt atc gaa aca aac gga gaa aca gga gaa        3264
Glu Ile Arg Lys Arg Pro Leu Ile Glu Thr Asn Gly Glu Thr Gly Glu
        1075                1080                1085

atc gtg tgg gac aag ggt agg gat ttc gcg aca gtc cgg aag gtc ctg        3312
Ile Val Trp Asp Lys Gly Arg Asp Phe Ala Thr Val Arg Lys Val Leu
    1090                1095                1100

tcc atg ccg cag gtg aac atc gtt aaa aag acc gaa gta cag acc gga        3360
Ser Met Pro Gln Val Asn Ile Val Lys Lys Thr Glu Val Gln Thr Gly
1105                1110                1115                1120

ggc ttc tcc aag gaa agt atc ctc ccg aaa agg aac agc gac aag ctg        3408
Gly Phe Ser Lys Glu Ser Ile Leu Pro Lys Arg Asn Ser Asp Lys Leu
                1125                1130                1135

atc gca cgc aaa aaa gat tgg gac ccc aag aaa tac ggc gga ttc gat        3456
Ile Ala Arg Lys Lys Asp Trp Asp Pro Lys Lys Tyr Gly Gly Phe Asp
            1140                1145                1150

tct cct aca gtc gct tac agt gta ctg gtt gtg gcc aaa gtg gag aaa        3504
Ser Pro Thr Val Ala Tyr Ser Val Leu Val Val Ala Lys Val Glu Lys
        1155                1160                1165

ggg aag tct aaa aaa ctc aaa agc gtc aag gaa ctg ctg ggc atc aca        3552
Gly Lys Ser Lys Lys Leu Lys Ser Val Lys Glu Leu Leu Gly Ile Thr
    1170                1175                1180

atc atg gag cga tca agc ttc gaa aaa aac ccc atc gac ttt ctc gag        3600
Ile Met Glu Arg Ser Ser Phe Glu Lys Asn Pro Ile Asp Phe Leu Glu

1185                1190                1195                1200

gcg aaa gga tat aaa gag gtc aaa aaa gac ctc atc att aag ctt ccc      3648
Ala Lys Gly Tyr Lys Glu Val Lys Lys Asp Leu Ile Ile Lys Leu Pro
                1205                1210                1215

aag tac tct ctc ttt gag ctt gaa aac ggc cgg aaa cga atg ctc gct      3696
Lys Tyr Ser Leu Phe Glu Leu Glu Asn Gly Arg Lys Arg Met Leu Ala
            1220                1225                1230

agt gcg ggc gag ctg cag aaa ggt aac gag ctg gca ctg ccc tct aaa      3744
Ser Ala Gly Glu Leu Gln Lys Gly Asn Glu Leu Ala Leu Pro Ser Lys
        1235                1240                1245

tac gtt aat ttc ttg tat ctg gcc agc cac tat gaa aag ctc aaa ggg      3792
Tyr Val Asn Phe Leu Tyr Leu Ala Ser His Tyr Glu Lys Leu Lys Gly
    1250                1255                1260

tct ccc gaa gat aat gag cag aag cag ctg ttc gtg gaa caa cac aaa      3840
Ser Pro Glu Asp Asn Glu Gln Lys Gln Leu Phe Val Glu Gln His Lys
1265                1270                1275                1280

cac tac ctt gat gag atc atc gag caa ata agc gaa ttc tcc aaa aga      3888
His Tyr Leu Asp Glu Ile Ile Glu Gln Ile Ser Glu Phe Ser Lys Arg
                1285                1290                1295

gtg atc ctc gcc gac gct aac ctc gat aag gtg ctt tct gct tac aat      3936
Val Ile Leu Ala Asp Ala Asn Leu Asp Lys Val Leu Ser Ala Tyr Asn
            1300                1305                1310

aag cac agg gat aag ccc atc agg gag cag gca gaa aac att atc cac      3984
Lys His Arg Asp Lys Pro Ile Arg Glu Gln Ala Glu Asn Ile Ile His
        1315                1320                1325

ttg ttt act ctg acc aac ttg ggc gcg cct gca gcc ttc aag tac ttc      4032
Leu Phe Thr Leu Thr Asn Leu Gly Ala Pro Ala Ala Phe Lys Tyr Phe
    1330                1335                1340

gac acc acc ata gac aga aag cgg tac acc tct aca aag gag gtc ctg      4080
Asp Thr Thr Ile Asp Arg Lys Arg Tyr Thr Ser Thr Lys Glu Val Leu
1345                1350                1355                1360

gac gcc aca ctg att cat cag tca att acg ggg ctc tat gaa aca aga      4128
Asp Ala Thr Leu Ile His Gln Ser Ile Thr Gly Leu Tyr Glu Thr Arg
                1365                1370                1375

atc gac ctc tct cag ctc ggt gga gac agc agg gct gac ccc aag aag      4176
Ile Asp Leu Ser Gln Leu Gly Gly Asp Ser Arg Ala Asp Pro Lys Lys
            1380                1385                1390

aag agg aag gtg ggc agt ggc cgc aaa aaa cgc cgc cag cgc cgc cgc      4224
Lys Arg Lys Val Gly Ser Gly Arg Lys Lys Arg Arg Gln Arg Arg Arg
        1395                1400                1405

ccg cag tga    4233
Pro Gln
    1410

â\200\203

## Claims

1. Method for preparation of recombinant nuclease of the Cas family of the CRISPR/Cas system involving the transformation of competent E. coli cells with a new expression plasmid encoding the target protein, with further cultivation of transformed producing cells under conditions ensuring the expression of the specified recombinant nuclease in the Luria-Bertani (LB) broth with the addition of ampicillin and the use of isopropyl-β-D-thiogalactoside (IPTG) as an inducer of the expression of the target protein, isolation of the target protein from the soluble fraction and two-stage chromatographic purification, **characterized** as follows:
as the producing cells the competent cells of Rosetta-gami B (DE3) E. coli are used;
MgSO₄ and glucose are added to the culture medium and the producing cells are grown until the starting culture is obtained then the culture is diluted to an optical density of OD 600≈0.3 and the glucose content in this medium is reduced by at least two times;
the producing cells are then cultured to achieve an optical density of OD 600≈1.5-1.9, IPTG is added and incubated at 22°C for the effective expression of the target protein;
after the centrifugation at the end of cultivation, cells are resuspended in a buffer for lysis based on Na₂HPO₄/NaH₂PO₄ and destroyed by ultrasonic disintegration followed by centrifugation;
to the fraction of soluble cytoplasm containing the target protein obtained after centrifugation NaCl and imidazole are added and the first stage of chromatographic purification is carried out by metal-affinity chromatography, while the elution of the target protein is carried out with a buffer containing Na₂HPO₄/NaH₂PO₄, NaCl and imidazole;
at the second stage of chromatographic purification the target protein in the specified buffer is diluted at least 20 times with a buffer with Tris-HCl, while the conductivity of the solution does not exceed 5 mS/cm and is subjected to the cation exchange chromatography followed by elution of the target protein with a buffer containing Tris-HCl and NaCl where DTT and EDTA are added to the specified buffer to stabilize the target protein;
additional purification of recombinant nuclease of the Cas family from endotoxins of producing cells is carried out by:
addition after the chromatography to the specified target protein solution in the buffer the detergent Triton X-114 for removing bacterial endotoxin due to transition of Triton X-114 from the water-insoluble fraction to the water-soluble one and back with the formation of micelles and subsequent separation of water-soluble and water-insoluble fractions by centrifugation,
then Triton X-114 is added again to the water-soluble fraction obtained after centrifugation with a subsequent transition of the water-insoluble fraction to the water-soluble one and back, with the formation of micelles and separation by centrifugation,
in this case, for removal of bacterial endotoxins from recombinant nucleases preparations of Cas family of CRISPR/Cas system to a level that allows applying the specified preparation in vivo, stage with the addition of Triton X-114 is repeated at least 6 times;
after the final centrifugation, at the end of the additional purification stage an aqueous phase is selected including a purified recombinant nuclease of the Cas family and
if necessary, lyophilization is carried out with the addition of auxiliary substances for lyophilization.

2. The method of the claim 1 different by adding 2 mM MgSO₄ and 2% of glucose with 100 mkg/ml of ampicillin into the culture medium of LB and then diluted with 100 mkg/ml ampicillin added with 2 mM MgSO₄ and 1% of glucose to obtain the starting culture.

3. The method of the claim 1 different by growing producing cells at 37°C for 16-20 hours after dilution they are cultivated at 37°C until they get the optical density of OD₆₀₀≈1,5-1,9, and after the addition of IPTG they are incubated at 22°C for 20-24 hours to obtain the starting culture.

4. The method of the claim 1 different by the resuspension of the cells in the buffer for the lysis containing 20 mM Na₂HPO₄/NaH₂PO₄ with pH 6,6-8,0.

5. The method of the claim 1 different by carrying out the centrifugation at the end of cultivation at 6,000 g for 10 minutes and/or centrifugation to separate the fraction of soluble cytoplasm is carried out at 10,000 g for one hour and/or centrifugation at the stage of additional purification from endotoxins is carried out at 10,000 g for 10 minutes at 23°C.

6. The method of the claim 1 different by adding NaCl at the first stage of chromatographic purification to the specified fraction of soluble cytoplasm to a final concentration of 500 mM and imidazole to a final concentration of 10 mM, the elution of the target protein is carried out with a buffer with pH 6,6-8,0, containing 20 mM Na₂HPO₄/NaH₂PO₄, 500 mM NaCl and 250 mM of imidazole.

7. The method of the claim 1 different by dilution of the target protein at the second stage of chromatographic purification in the specified buffer for at least 20 times with a buffer with a pH of 8.2 containing 20 mM Tris-HCl with the solution conductivity of 3-5 mS/cm, the target protein elution is carried out with the buffer with pH 8,2, containing 20 mM Tris-HCl and 600-1000 mM NaCl.

8. The method of the claim 1 different by using Chelating Sepharose FF chromatography resin at the first stage of chromatographic purification and/or at the second stage of the chromatographic purification SP Sepharose FF chromatography resin is used.

9. The method of the claim 1 different by adding the buffer to stabilize the target protein with up to 2 mM DTT and 0,2 mM EDTA.

10. The method of the claim 1 different by carrying out additional purification of the recombinant nuclease of the Cas family from endotoxins by performing the following steps:
adding a detergent Triton X-114 up to 1% to the solution of the target protein in the buffer obtained after the chromatographic purification steps;
placing the resulting solution on ice until absolute transparency of the solution is achieved, followed by holding at 37 °C until absolute opacity of the solution is achieved in order to enrich the solution with micelles;
subsequent separation of the aqueous and micellar fractions of the solution by centrifugation;
adding Triton X-114 detergent up to 1% to the upper aqueous phase obtained after centrifugation and repeating the treatment until preparation of a recombinant nuclease of the Cas family with a bacterial endotoxin content reduced to a level that allows the use of the specified preparation in vivo.

11. The method of the claim 10 different by placing the solution on ice and / or kept at 37 ° C for at least 15 minutes.

12. The method of the claim 1 different by repeating the stages with addition of Triton X-114 at the stage of additional purification for 6 - 9 times.

13. The method of the claim 1 different by obtaining the preparation of the Cas family recombinant nuclease with 0,3-1,5 EU/ml contained in buffer solution containing Tris-HCl, NaCl, DTT 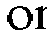 EDTA, preferable with 20 mM Tris-HCl, 600-1000 mM NaCl, 2 mM DTT, 0,2 mM EDTA at pH 8,2.

14. The method of the claim 1 different by obtaining lyophilized preparation of recombinant nuclease of the Cas family by adding lyophilization excipients selected from of glycerol or of trehalose, preferably of glycerol at a concentration of 10-50% or of trehalose with a concentration of 50-500 mM to the buffer solution containing the recombinant nuclease of Cas family with 0,3-1,5 EU/ml, Tris-HCl, NaCl, DTT and EDTA, preferably 20 mM Tris-HCl, 600-1000 mM NaCl, 2 mM DTT, 0,2 mM EDTA at pH 8,2, with subsequent lyophilization.

15. The method of any claims 1-14 different by recombinant nuclease of Cas family of CRISPR/Cas system being characterized with the amino acid sequence selected from SEQ ID NO: 1-8.

16. The method of any claims 1-14 different by coding the amino acid sequence of the recombinant nuclease of Cas family of CRISPR/Cas system with the nucleotide sequence selected from SEQ ID NO: 9-16.

17. Preparation of recombinant nucleases of Cas family and CRISPR/Cas system, substantially free from bacterial endotoxins for use in CRISPR/Cas system, which was an aqueous phase comprising purified recombinant nuclease of Cas family obtained after the final centrifugation at the completion of the additional purification phase or freeze-dried in accordance with the method of claims 1-16.

18. Preparation under the claim 17, where the preparation substantially free from bacterial endotoxins of the recombinant nuclease of Cas family of CRISPR/Cas system contains not less than 1,5 EU/ml of bacterial endotoxins, preferably of 0,3 UE/ml.

19. Preparation under any of the claims 17-18, which is a freeze-dried preparation.

20. Kit to be used in the CRISPR/Cas system including preparation of the recombinant nuclease of the Cas family of the CRISPR/Cas system in accordance with any of the claims 17-19, optionally a target-specific guide RNA and instructions for use.

21. Kit under the claim 20 different by including preparation of the recombinant nuclease of CRISPR/Cas system under any of the claims 17-19 in combination with the target-specific guide RNA or separately.
